# EUROPÄISCHE PATENTSCHRIFT

(11) **EP 2 046 410 B1**
(45) Veröffentlichungstag und Bekanntmachung des Hinweises auf die Patenterteilung: **10.04.2019**
(21) Anmeldenummer: 07764422.7
(22) Anmeldetag: 03.07.2007
(51) Int. Cl.: A61L 29/18, A61L 29/16, A61L 31/16, A61L 31/10

(54) **HERSTELLUNG, VERFAHREN UND VERWENDUNG VON WIRKSTOFFFREISETZENDEN MEDIZINPRODUKTEN ZUR PERMANENTEN OFFENHALTUNG VON BLUTGEFÄSSEN**
MANUFACTURE, METHOD, AND USE OF ACTIVE SUBSTANCE-RELEASING MEDICAL PRODUCTS FOR PERMANENTLY KEEPING BLOOD VESSELS OPEN
FABRICATION ET UTILISATION D'ARTICLES MÉDICAUX À LIBÉRATION DE PRINCIPES ACTIFS POUR MAINTENIR OUVERTS EN PERMANENCE DES VAISSEAUX SANGUINS ET PROCÉDÉS ASSOCIÉS

(30) Priorität: 03.07.2006 DE 102006030586
(43) Veröffentlichungstag der Anmeldung: 15.04.2009
(60) Teilanmeldung: 11075120.3
(73) Patentinhaber: Hemoteq AG, 52146 Würselen (DE)
(72) Erfinder: HOFFMANN, Erika, 52249 Eschweiler (DE); HOFFMANN, Michael, 52249 Eschweiler (DE); HORRES, Roland, 52223 Stolberg (DE); KÜSTERS, Sabine, 52382 Niederzier (DE)
(74) Vertreter: Arth, Hans-Lothar
(86) Internationale Anmeldenummer: PCT/DE2007/001173
(87) Internationale Veröffentlichungsnummer: WO 2008/003298

(56) Entgegenhaltungen:
- WO-A2-2005/086831
- DE-A1-102004 020 856
- US-A1- 2005 060 028
- US-A1- 2005 246 009

## Beschreibung

Die Erfindung betrifft Stents beschichtet mit mindestens einer polymeren Schicht aus Poly(D,L-lactid-co-Glycolid) und Rapamycin, wobei der Stent eine unterste Beschichtung aus einem hämokompatiblen Polymer aufweist, und wobei Rapamycin eine Konzentration von 0,001 - 10 mg per cm² der Stentoberfläche hat.

Im menschlichen Körper kommt das Blut nur im Falle einer Verletzung mit anderen Oberflächen in Kontakt als der Innenseite von natürlichen Blutgefäßen. Daher wird das Blutgerinnungssystem immer dann aktiviert, wenn Blut mit fremden Oberflächen in Kontakt kommt, um die Blutung zu stillen und einen lebensbedrohlichen Blutverlust zu verhindern. Da ein Implantat ebenfalls eine fremde Oberfläche darstellt, werden alle Patienten, die ein Implantat erhalten, das dauerhaft mit Blut in Kontakt steht, für die Dauer des Blutkontaktes mit Medikamenten, sogenannten Antikoagulantien, welche die Blutgerinnung unterdrücken, behandelt, wobei zum Teil erhebliche Nebenwirkungen in Kauf genommen werden müssen.

Das beschriebene Thromboserisiko tritt auch als einer der Risikofaktoren beim Einsatz von Gefäßstützen, sogenannten Stents,in blutführenden Gefäßen auf. Der Stent dient zur permanenten Aufdehnung der Gefäßwände beim Auftreten von Gefäßverengungen und -verschlüssen z.B durch artheriosklerotische Veränderungen speziell der Herzkranzgefäße. Das verwendete Material für den Stent ist in der Regel medizinischer Edelstahl, Ni-Ti-Legierungen oder Co-Cr-Legierungen, während polymere Stents noch in der Entwicklungsphase stecken.

Die Stentthrombose tritt in weniger als einem Prozent der Fälle noch im Herzkatheterlabor als frühe Thrombose oder in zwei bis fünf Prozent der Fälle während des Krankenhausaufenthaltes auf. Gefäßverletzungen durch den Eingriff werden in etwa fünf Prozent der Fälle aufgrund der arteriellen Schleusen verursacht und es besteht auch die Möglichkeit durch Ausbuchtung von Gefäßen Pseudo-Aneurysmen zu verursachen.

Ebenso wird bei einer PTCA durch Einführung eines Fremdkörpers die Blutgerinnung aktiviert. Da es sich in diesem Falle um ein Kurzzeitimplantat handelt, liegen die Probleme aber wesentlich mehr in der Stärke der Gefässdilatation, die notwendig ist, um einen Gefässengpass oder Verschluss zu weiten bzw. zu beseitigen. Eine weitere sehr häufig auftretende Komplikation ist die Restenose, der Wiederverschluss des Gefäßes. Obwohl Stents das Risiko eines erneuten Gefäßverschlusses verkleinern, sind sie bisher nicht in der Lage, Restenosen vollständig zu verhindern bzw. sind selber Ursache für Neointimahyperplasien. Die Wiederverengungsrate (Restenose) nach einer Stentimplantation ist bei besonders schweren Erkrankungen mit bis zu 30 % eine der Hauptursachen für den erneuten Aufenthalt von Patienten in der Klinik. Da die Wiederverschlussrate nach erfolgter PTCA im Vergleich zum Stent wesentlich höher liegt, so dass in der Regel bei Patienten mit massiver Stenose oder Restenose immer ein Stent gesetzt wird.

Eine genaue begriffliche Beschreibung der Restenose ist in der Fachliteratur nicht aufzufinden. Die am häufigsten verwendete morphologische Definition der Restenose ist diejenige, die nach erfolgreicher PTA (perkutane transluminale Angioplastie) die Restenose als eine Reduktion des Gefäßdurchmessers auf weniger als 50% des normalen festlegt. Hierbei handelt es sich um einen empirisch festgelegten Wert, dessen hämodynamische Bedeutung und Beziehung zur klinischen Symptomatik einer soliden wissenschaftlichen Basis entbehrt. In der Praxis wird häufig die klinische Verschlechterung eines Patienten als Zeichen einer Restenose des vormals behandelten Gefäßabschnitts angesehen.
Die während der Implantation des Stents bzw. bei der Überdehnung des Gefässes verursachten Gefäßverletzungen rufen Entzündungsreaktionen hervor, die für den Heilungsprozess in den ersten sieben Tagen eine entscheidende Rolle spielen. Die hierbei ablaufenden Prozesse sind unter anderem mit der Ausschüttung von Wachstumsfaktoren verbunden, womit eine verstärkte Proliferation der glatten Muskelzellen eingeleitet wird und damit schon kurzfristig zu einer Restenose, einem erneuten Verschluss des Gefäßes aufgrund unkontrollierten Wachstums, führen.

Auch noch nach einigen Wochen, wenn der Stent in das Gewebe des Blutgefäßes vollständig von glatten Muskelzellen umhüllt wird, können Vernarbungen zu stark ausgeprägt sein (Neointimahyperplasie) und dazu führen, dass nicht nur die Stentoberfläche bedeckt, sondern der ganze Innenraum des Stents verschlossen wird.

Man hat vergeblich versucht, das Problem der Restenose durch die Entwicklung von durch Microporen Heparinfreigebenden Ballonkathetern und später durch die Beschichtung von Stents mit Heparin zu lösen (J. Whörle et al, European Heart Journal (2001) 22 1808-1816). Heparin adressiert als Antikoagulanz jedoch nur die erstgenannte Ursache und kann darüber hinaus nur in Lösung seine volle Wirkung entfalten. Dieses erste Problem läßt sich mittlerweile medikamentös durch die Gabe von Antikoagulantien fast vollständig vermeiden. Das weitere Problem versucht man zur Zeit zu lösen, indem man das Wachstum der glatten Muskelzellen lokal zu hemmen versucht.

Das wird z.B. mit radioaktiven Stents oder mit Stents versucht, welche pharmazeutische Wirkstoffe, die bevorzugt antiproliferativ wirken, enthalten.Als erfolgreich hat sich der aus der Chemotherapie stammende Wirkstoff Paclitaxel herausgestellt, der eine sich im Mitoseprozess befindende Zelle durch irreversible Bindung an den sich bildenden Spindelapparat an der Teilung hindert. Die Zelle verbleibt in diesem Übergangszustand, den sie nicht aufrechterhalten kann und stirbt.
Allerdings zeigen sich aus der bestehenden Erfahrung mit dem Paxlitaxel-eluting stent, dass Paclitaxel im Gegensatz zum selben unbeschichteten Stent zu einer erhöhten Thromboserate als Spätfolge führt. Dies geht auf die Wirkweise des Paclitaxel zurück. Die irreversible Bindung und Stabilisierung von Tubulin während der Zellteilung führt dazu, dass die Zelle keine anderen zellerhaltenden Funktionen ausführen kann. Schlussendlich stirbt die Zelle.
Man will auf diese Weise den Wundheilungsprozess besser kontrollieren können, doch erreicht man durch die Erzeugung von nicht mehr lebensfähigem Zellmaterial unerwünscht eine erhöhte Entzündungsreaktion und folglich eine verstärkte Immunantwort.
Die Dosierung von Paclitaxel ist sehr schwierig einzuhalten. Es gilt zum einen neben den unvermeidbaren den Wundheilungsprozess induzierenden Reaktionen den durch Paclitaxel zusätzlich induzierten Entzündungsprozess zu bekämpfen, zum anderen darf die Dosierung nicht so gering sein, dass kaum ein Effekt erzielt wird. Diese Gratwanderung führt häufig dazu, dass sich noch nach einem halben Jahr nicht die erwünschte Endothelschicht über den Stent gebildet hat.
Entweder liegen noch Stentstreben frei und führt für den Patienten zu einem erhöhten Risiko noch nach Monaten an einer Thrombose (late acute thrombosis) zu versterben, oder das den Stent umgebende Zellgewebe besteht aus glatten Muskelzellen, Monocyten usw., was nach einiger Zeit wieder zu einem Verschluss führen kann.

Als viel versprechender Wirkstoff für den gleichen Zweck der Restenoseprophylaxe zeigt sich Rapamycin (syn. Sirolimus), ein hydrophiles Makrolid-Antibiotikum. Dieser Wirkstoff wird vor allem in der Transplantationsmedizin als Immunsuppresivum eingesetzt, wobei Rapamycin im Gegensatz zu anderen immunsuppresiven Wirkstoffen auch die Tumorbildung hemmt. Da nach einer Transplantation ein erhöhtes Tumorbildungsrisiko für den Patienten besteht, ist die Gabe von Rapamycin vorteilhaft, da andere Immunsupressiva wie beispielsweise Cyclosporin A bekanntermassen die Tumorbildung sogar fördern können.

Die Wirkung von Rapamycin ist bisher nicht im Einzelnen bekannt, doch wird sie vor allem auf die Komplexbildung mit dem Protein mTOR (mammalian Target of Rapamycin), einer 282kD großen Phospatidylinositol-3-Kinase zurückgeführt. Da mTOR für eine Reihe von Cytokin-vermittelten Signaltransduktionswegen, unter anderem auch für die Zellteilung notwendige Signalwege zuständig ist, besitzt es neben der immunsuppresiven Wirkung auch antiphlogistische, antiproliferative und sogar fungizide Eigenschaften

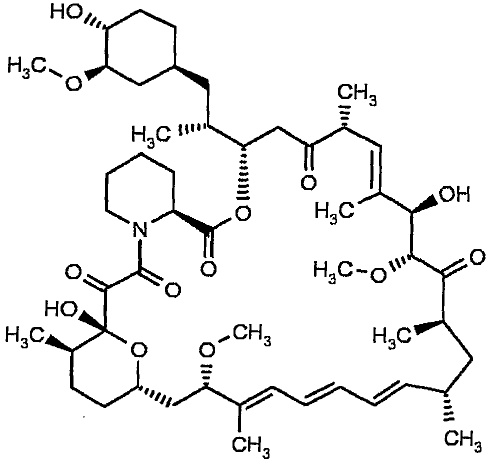

### IUPAC - Name:

[3S-[3R*[E(1S*,3S*,4S*)],4S*,5R*,8S*,9E,12R*,14R*,15S*,16R*,18S*,19S*,26aR*]]-5,6,8,11,12,13,14,15,16,17,18,19,24,25,26,26a-hexadecahydro-5,19-dihydroxy-3-[2-(4-hydroxy-3-methoxycyclohexyl)-1-methylethenyl]-14,16-dimethoxy-4,10,12,18-tetramethyl-8-(2-propenyl)-15,19-epoxy-3H-pyrido[2,1-c][1,4]-oxaazacyclotricosine-1,7,20, 21(4H,23H)-tetron Monohydrat.

Die Proliferation wird in der späten G1 Phase durch Unterbleiben der ribosomalen Proteinsynthese unterbrochen. Damit lässt sich die Wirkweise von Rapamycin ähnlich dem allerdings stark hydrophoben Paclitaxel im Vergleich zu anderen antiproliferativen Wirkstoffen als besonders hervorheben. Zudem sind die immunsuppresive und antiphlogistische Wirkung wie oben beschrieben mehr als vorteilhaft, da auch das Ausmass der Entzündungsreaktionen und der gesamten Immunantwort wie deren frühzeitige Kontrolle nach Stentimplantation entscheidend für den weiteren Erfolg ist.

Somit vereint Rapamycin alle notwendigen Bedingungen für den Einsatz gegen Stenosen und Restenosen.
Die begrenzte Haltbarkeit des Rapamycin auf bzw. in einem Implantat ist als weiterer Vorteil gegenüber Paclitaxel zu erwähnen, da der Wirkstoff in den ersten entscheidenden Wochen nach Stentimplantation notwendigerweise wirksam sein muss. In Folge dessen kann die für den Abschluss eines gesunden Heilungsprozess wichtige Endothelzellschicht den Stent lückenlos überwachsen und in die Gefässwand integrieren.

Die gleiche Wirkweise findet sich auch bei den bekannten Derivaten von Rapamycin (Biolimus, Everolimus), da die Veränderung an den funktionellen Gruppen des Moleküls stattfindet, die für die mTOR-Bindungsregion irrelevant sind.
In verschiedenen klinischen Studien (RAVEL, SIRIUS, SIROCCO) hat Rapamycin - im Gegensatz zu anderen Wirkstoffen wie beispielsweise Dexamethason, Tacrolimus, Batimastat - gezeigt, dass es im Vergleich mit dem stark hydrophoben Paclitaxel trotz unterschiedlicher physikalischer Eigenschaften zur Bekämpfung der Restenose mehr als geeignet ist

Der Wirkstoff allein ist kein Garant für eine optimale Restenoseprophylaxe. Der wirkstofffreisetzende Stent muss in seiner Gesamtheit den Erfordernissen gerecht werden. Neben der Dosierungsfindung muss die Wirkstofffreisetzung zeitlich verzögert und konzentrationsabhängig gesteuert werden. Die Wirkstofffreisetzung als auch die Wirkstofffreisetzungsrate sind nicht nur von den physikalischen und chemischen Eigenschaften des Wirkstoffs abhängig, sondern ebenso sehr von den Eigenschaften des eingesetzten Polymers und den Interaktionen von Polymer und Wirkstoff. Stentmaterial, Stenteigenschaften und Stentdesign sind weitere Faktoren, die es für ein optimal wirkendes Medizinprodukt zu berücksichtigen gilt.

Als Teilanmeldung von EP 0950386 B1, das einen Stent mit Kanälen in den Streben, in denen sich Rapamycin unter einer diffusionskontrollierenden Polymerschicht befindet, beschreibt, wird in EP1407726 A1 (Priorität 1998) ein aus polymerer Matrix Rapamycin freisetzender Stent beschrieben, der sich seit 2002 im Handel (Cypher™-Stent) befindet. Dabei wird auf ein mit Parylen C beschichteter Stent mit einer Mischung aus den beiden biostabilen Polymeren Polyethylen-Vinylacetetat (PEVA) und Poly-n-butylmethylmethacrylat (PBMA) und Rapamycin aufgebracht und mit einer diffusionskontrollierenden wirkstofffreien Deckschicht aus PBMA versehen. Die Erfahrungen mit diesem Stent haben gezeigt, dass allergische Reaktionen und Entzündungen als auch späte Thrombosen zu signifikanten Problemen führen (Prof.

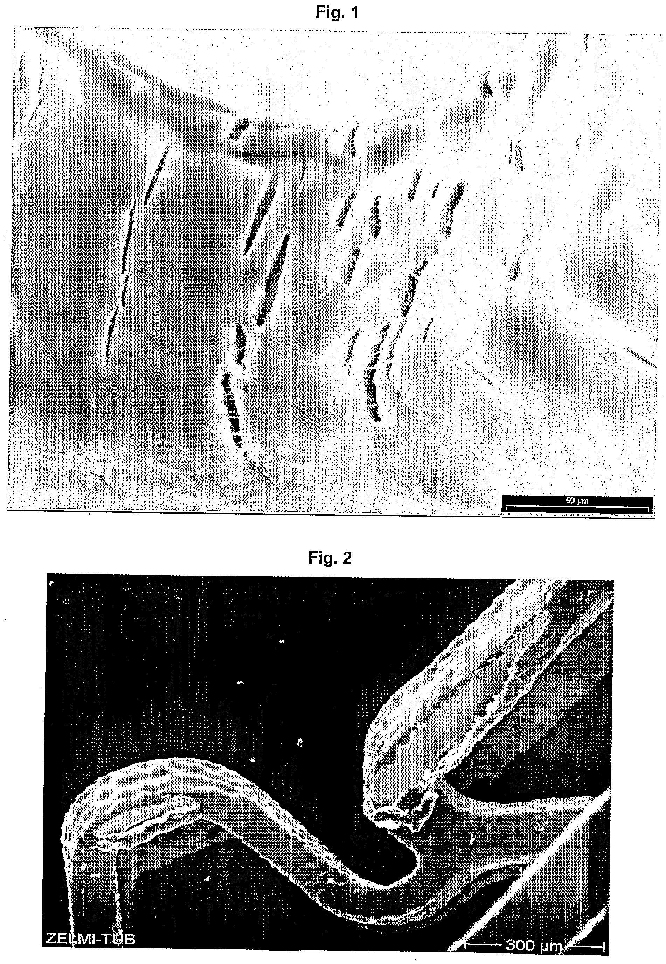

Renu Virmani, 2002-ff). Zudem ist PBMA als Deckschicht problematisch, da diese beim Expandieren bricht und es daraufhin zu einer unkontrollierten Freisetzung von Rapamycin kommt (siehe Abb. 1).
Dabei macht sich ein allgemeines Problem bei der Verwendung von Rapamycin bemerkbar. Die kontrollierte Bioverfügbarkeit von Rapamycin ist schwierig aufrecht zu erhalten :
Rapamycin als hydrophiles Molekül geht schnell in Lösung. Bricht nun die diffusionskontrollierte Deckschicht auf, ist die Freisetzung von Rapamycin schnell, unkontrolliert und ungerichtet.
Zusätzlich besteht aufgrund der mangelnden Elastizität von PMBA die Gefahr der Ablösung größerer Polymerstücke, die aufgrund ihrer Biostabilität in den Blutkreislauf langwierig für weitere Probleme sorgen können (siehe Abb. 2).

In EP 0568310 B1 wird die Wirkstoffkombination aus Heparin und Rapamycin für hyperproliferative vasculäre Erkrankungen beansprucht. Darin wird lediglich kurz in der Beschreibung erwähnt, dass die Verabreichung dieser Wirkstoffkombination mittels eines mit Rapamycin imprägnierten vaskulären Stents erfolgen kann. Ausführungsbeispiele existieren nicht, so dass es sich mehr um eine Bemerkung handelt und deshalb viele Fragen offen lässt. Da dieses Patent aus dem Jahre 1992 stammt, es aber bisher lediglich den auf EP1407726 A1 basierenden o.g. Cypher™ Stent von Cordis Corp. im Handel gibt, ist offensichtlich die wirtschaftliche Realisierung eines Rapamycin-Heparin imprägnierten Stents nicht die primäre Zielsetzung dieses Patentes gewesen.

EP 0 551 182 B1 beschreibt und beansprucht bereits unter Erwähnung eines Stentes ein mit Rapamycin imprägniertes Medikament, dass mechanisch induzierte hyperproliferative Erkrankungen vermindern bzw. vermeiden soll. Darin wird der mit Rapamycin imprägnierte Stent als Hilfmittel zur Einbringen von Rapamycin in das Gefäss erwähnt, doch wird nicht näher darauf eingegangen. Ein mit Rapamycin imprägnierter Stent bedeut eine reine Wirkstoffschicht auf dem Stentgerüst ohne die Anwenseheit eines Trägers. Diese Ausführungsform ist technisch nicht sinnvoll realisierbar, da Rapamycin an der Luft rasch hydrolysiert und sich durch Spaltung der Lactonbindung leicht zusetzt, vor allen bei der Anwesenheit von Wasser. Zudem wird eine reine Rapamycin-Wirkstoffschicht bei der Einführung eines mit Rapamycin beschichteten Katheterballons oder einer Ballons mit mit Rapamycin beschichtetem Stent zu leicht im Blutfluß abgelöst, so dass nicht sichergestellt werden kann, ob am Zielort noch eine genügende Menge an Rapamycin auf dem Medizinprodukt (Stent bzw. Katheterballon) zur Verfügung steht. Ferner weist eine reine Wirkstoffschicht den Nachteil auf, dass bei der Dilatation der Wirkstoff innerhalb kurzer Zeit vollständig freigesetzt wird, da eine wirkstofffreisetzende Beschichtung in Form eine Drug-Release-Systems fehlt und somit eine Spontanelution vorliegt und keine Elutionskinetik ausgenutzt werden kann.

WO2005/086831 A2 offenbart ein Stent beschichtet mit einer polymeren Schicht aus poly (DL- lactide-co-glycolide) und Rapamycin.

Die vorliegende Erfindung betrifft daher nicht die Bereitstellung von Rapamycinbeschichteten Stents was bereits zum Stand der Technik gehört, sondern ein optimiertes Trägersystem für den empfindlichen Wirkstoff Rapamycin.

Wie bereits oben erwähnt, ist allerdings nicht jeder Wirkstoff in beliebiger Weise als Restenoseprophylaxe einsetzbar. Für einen erfolgreichen Einsatz und langfristige Sicherheit des Patienten müssen unabhängig von der Qualität des unbeschichteten Implantates eine Vielzahl weiterer Bedingungen erfüllt sein. Die physikalischen und chemischen Eigenschaften eines geeigneten Wirkstoffes, des Lösungsmittel und der eventuell verwendeten Matrix müssen ebenso beachtet werden wie die Wechselwirkungen dieser Faktoren untereinander.

Erst durch die richtige Kombination dieser Parameter wird die zeitlich- und dosiskontrollierte Verfügbarkeit des therapeutischen Mittels optimal geregelt, womit letztendlich die Sicherheit und Gesundheit des Patienten gewährleistet wird.

Aufgabe der vorliegenden Erfindung ist es, Rapamycin-eluting Stents bereitzustellen, welche einen kontrollierten und gesunden Heilungsprozess gewährleisten und die Wiederherstellung einer lückenlosen endothelzellschichtbewachsenen Gefäßwand erlauben, ohne die oben erwähnten Nachteile aufzuweisen. Somit besteht die Aufgabe der vorliegenden Erfindung in der Bereitstellung optimierter Trägersysteme für Rapamycin, welche auf Stents eine ausreichende Haftstabilität und Zersetzungsstabilität des Wirkstoffs Rapamycin gewährleisten und eine Freisetzungskinetik aufweisen, welche zur Prophylaxe und Behandlung von Restenose bestens geeignet ist.

Die Unterdrückung der cellulären Reaktionen in den ersten Tagen und Wochen nach Implantation wird dabei vorrangig mit Hilfe des antiproliferativ, immunsupressiv und antiphlogistisch wirkenden Rapamycin, seiner gleichermassen wirkenden Derivate/Analoga und bzw. oder Metaboliten erreicht. Weitere die Wundheilung bzw. den Wundheilungsverlauf in sinnvoller Weise unterstützende Wirkstoffe und/oder Wirkstoffkombinationen können beigefügt werden.

Diese Aufgabe wird durch die technische Lehre der unabhängigen Ansprüche der vorliegenden Erfindung gelöst.Weitere vorteilhafte Ausgestaltungen der Erfindung ergeben sich aus den abhängigen Ansprüchen, der Beschreibung sowie den Beispielen.

Die erfindungsgemäßen Stents besitzen mindestens eine polymere Schicht aus Poly(D,L-lactid-co-Glycolid) und Rapamycin, wobei der Stent eine unterste Beschichtung aus einem hämokompatiblen Polymer aufweist, und wobei Rapamycin eine Konzentration von 0,001 - 10 mg per cm² der Stentoberfläche hat.

Rapamycin oder eine Wirkstoffkombination mit Rapamycin wird kovalent und/oder adhäsiv an die darunterliegende Schicht bzw. Stentoberfläche gebunden und/oder kovalent und/oder adhäsiv in die Schicht eingelagert, so dass die aktive Substanz kontinuierlich und in geringen Dosen freigesetzt wird und die Besiedelung der Stentoberfläche mit Zellen unterbunden wird, jedoch eine Überbesiedelung verhindert wird. Die Kombination beider Effekte verleiht dem erfindungsgemäßen Stent die Fähigkeit, schnell in die Gefäßwand einzuwachsen und vermindert sowohl das Risiko einer Restenose als auch das Risiko einer Thrombose. Die kontrollierte Freisetzung des Rapamycin erstreckt sich über einen Zeitraum von 1 bis 12 Monaten, bevorzugt über 1-2 Monate nach Implantation.

### Wirkstoffkombinationen

Bei den erfindungsgemäßen Ausführungsformen kann Rapamycin auch in Kombination mit anderen Wirkstoffen eingesetzt werden. Als weitere, die Wirkung von Rapamycin und/oder dessen chemische wie biologische Abkömmlinge unterstützende antiproliferative, antimigrative, antiangiogene, antiinflammatorische, antiphlogistische, zytostatische, zytotoxische und/oder antithrombotische Wirkstoffe lassen sich einsetzen: Somatostatin, Tacrolimus, Roxithromycin, Dunaimycin, Ascomycin, Bafilomycin, Erythromycin, Midecamycin, Josamycin, Concanamycin, Clarithromycin, Troleandomycin, Folimycin, Cerivastatin, Simvastatin, Lovastatin, Fluvastatin, Rosuvastatin, Atorvastatin, Pravastatin, Pitavastatin, Vinblastin, Vincristin, Vindesin, Vinorelbin, Etobosid, Teniposid, Nimustin, Carmustin, Lomustin, Cyclophosphamid, 4-Hydroxyoxycyclophosphamid, Estramustin, Melphalan, Ifosfamid, Tropfosfamid, Chlorambucil, Bendamustin, Dacarbazin, Busulfan, Procarbazin, Treosulfan, Tremozolomid, Thiotepa, Daunorubicin, Doxorubicin, Aclarubicin, Epirubicin, Mitoxantron, Idarubicin, Bleomycin, Mitomycin, Dactinomycin, Methotrexat, Fludarabin, Fludarabin-5'-dihydrogenphosphat, Cladribin, Mercaptopurin, Thioguanin, Cytarabin, Fluorouracil, Gemcitabin, Capecitabin, Docetaxel, Carboplatin, Cisplatin, Oxaliplatin, Amsacrin, Irinotecan, Topotecan, Hydroxycarbamid, Miltefosin, Pentostatin, Aldesleukin, Tretinoin, Asparaginase, Pegasparase, Anastrozol, Exemestan, Letrozol, Formestan, Aminoglutethemid, Adriamycin, Azithromycin, Spiramycin, Cepharantin, 8-α-Ergoline, Dimethylergoline, Agroclavin, 1-Allylisurid, 1-Allyltergurid, Bromergurid, Bromocriptin (Ergotaman-3',6',18-trione, 2-bromo-12'-hydroxy-2'-(1-methylethyl)-5'-(2-methylpropyl)-, (5'alpha)-), Elymoclavin, Ergocristin (Ergotaman-3',6',18-trione, 12'-hydroxy-2'-(1-methylethyl)-5'-(phenylmethyl)-, (5'-alpha)-), Ergocristinin, Ergocornin (Ergotaman-3',6',18-trione, 12'-hydroxy-2',5'-bis(1-methylethyl)-, (5'-alpha)-), Ergocorninin, Ergocryptin (Ergotaman-3',6', 18-trione, 12'-hydroxy-2'-(1-methylethyl)-5'-(2-methylpropyl)-, (5'alpha)- (9CI)), Ergocryptinin, Ergometrin, Ergonovin (Ergobasin, INN: Ergometrin, (8beta(S))-9,10-Didehydro-N-(2-hydroxy-1-methylethyl)-6-methyl-ergoline-8-carboxamid), Ergosin, Ergosinin, Ergotmetrinin, Ergotamin (Ergotaman-3',6',18-trione, 12,-hydroxy-2'-methyl-5,-(phenylmethyl)-, (5'-alpha)- (9Cl)), Ergotaminin, Ergovalin (Ergotaman-3',6',18-trione, 12'-hydroxy-2'-methyl-5'-(1-methylethyl)-, (5'alpha)-), Lergotril, Lisurid (CAS-Nr.: 18016-80-3, 3-(9,10-Didehydro-6-methylergolin-8alpha-yl)-1,1-diethylharnstoff), Lysergol, Lysergsäure (D-Lysergsäure), Lysergsäureamid (LSA, D-Lysergsäureamid), Lysergsäurediethylamid (LSD, D-Lysergsäurediethylamid, INN: Lysergamid, (8beta)-9,10-Didehydro-N,N-diethyl-6-methyl-ergoline-8-carboxamid), Isolysergsäure (D-Isolysergsäure), Isolysergsäureamid (D-Isolysergsäureamid), Isolysergsäurediethylamid (D-Isolysergsäurediethylamid), Mesulergin, Metergolin, Methergin (INN: Methylergometrin, (8beta(S))-9,10-Didehydro-N-(1-(hydroxymethyl)propyl)-6-methylergoline-8-carboxamid), Methylergometrin, Methysergid (INN: Methysergid, (8beta)-9,10-Didehydro-N-(1-(hydroxymethyl)propyl)-1,6-dimethyl-ergoline-8-carboxamid), Pergolid ((8beta)-8-((Methylthio)methyl)-6-propyl-ergolin), Protergurid und Tergurid, Celecoxip, Thalidomid, Fasudil®, Ciclosporin, SMC-Proliferation-Inhibitor-2w, Epothilone A und B, Mitoxanthrone, Azathioprin, Mycophenolatmofetil, c-myc-Antisense, b-myc-Antisense, Betulinsäure, Camptothecin, PI-88 (sulfatiertes Oligosaccharid), Melanocyte-stimulating hormon (α-MSH), aktiviertes Protein C, IL1-ß-Inhibitor, Thymosin α-1, Fumarsäure und deren Ester, Calcipotriol, Tacalcitol, Lapachol, ß-Lapachon,Podophyllotoxin, Betulin, Podophyllsäure-2-ethylhydrazid, Molgramostim (rhuGM-CSF), Peginterferon α-2b, Lanograstim (r-HuG-CSF), Filgrastim, Macrogol, Dacarbazin, Basiliximab, Daclizumab, Selectin (Cytokinantagonist), CETP-Inhibitor, Cadherine, Cytokininhibitoren, COX-2-Inhibitor, NFkB, Angiopeptin, Ciprofloxacin, Camptothecin, Fluroblastin, monoklonale Antikörper, die die Muskelzellproliferation hemmen, bFGF-Antagonisten, Probucol, Prostaglandine, 1,11-Dimethoxycanthin-6-on, 1-Hydroxy-11-Methoxycanthin-6-on, Scopolectin, Colchicin, NO-Donoren wie Pentaerythrityltetranitrat und Syndnoeimine, S-Nitrosoderivate, Tamoxifen, Staurosporin, ß-Estradiol, α-Estradiol, Estriol, Estron, Ethinylestradiol, Fosfestrol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Cyclosporin A, Paclitaxel und dessen Derivate wie 6-α-Hydroxy-Paclitaxel, Baccatin, Taxotere, synthetisch hergestellte als auch aus nativen Quellen gewonnene macrocyclische Oligomere des Kohlensuboxids (MCS) und seine Derivate, Mofebutazon, Acemetacin, Diclofenac, Lonazolac, Dapson, o-Carbamoylphenoxyessigsäure, Lidocain, Ketoprofen, Mefenaminsäure, Piroxicam, Meloxicam, Chloroquinphosphat, Penicillamin, Tumstatin, Avastin, D-24851, SC-58125, Hydroxychloroquin, Auranofin, Natriumaurothiomalat, Oxaceprol, Celecoxib, β-Sitosterin, Ademetionin, Myrtecain, Polidocanol, Nonivamid, Levomenthol, Benzocain, Aescin, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika wie Cefadroxil, Cefazolin, Cefaclor, Cefotixin, Tobramycin, Gentamycin, Penicilline wie Dicloxacillin, Oxacillin, Sulfonamide, Metronidazol, Antithrombotika wie Argatroban, Aspirin, Abciximab, synthetisches Antithrombin, Bivalirudin, Coumadin, Enoxoparin, desulfatiertens und N-reacetyliertes Heparin, Gewebe-Plasminogen-Aktivator, GpIIb/IIIa-Plättchenmembranrezeptor, Faktor Xₐ-Inhibitor Antikörper, Interleukininhibitoren, Heparin, Hirudin, r-Hirudin, PPACK, Protamin, Natriumsalz der 2-Methylthiazolidin-2,4-dicarbonsäure Prourokinase, Streptokinase, Warfarin, Urokinase, Vasodilatoren wie Dipyramidol, Trapidil, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren, Prostacyclin, Vapiprost, Interferon α, ß und γ, Histaminantagonisten, Serotoninblocker, Apoptoseinhibitoren, Apoptoseregulatoren wie p65, NF-kB oder Bcl-xL-Antisense-Oligonukleotiden, Halofuginon, Nifedipin, Tocopherol, Vitamin B1, B2, B6 und B12, Folsäure, Tranilast, Molsidomin, Teepolyphenole, Epicatechingallat, Epigallocatechingallat, Boswellinsäuren und ihre Derivate, Leflunomid, Anakinra, Etanercept, Sulfasalazin, Etoposid, Dicloxacyllin, Tetracyclin, Triamcinolon, Mutamycin, Procainimid, D24851, SC-58125, Retinolsäure, Quinidin, Disopyrimid, Flecainid, Propafenon, Sotolol, Amidoron, natürliche und synthetisch hergestellte Steroide wie Bryophyllin A, Inotodiol, Maquirosid A, Ghalakinosid, Mansonin, Streblosid, Hydrocortison, Betamethason, Dexamethason, nichtsteroidale Substanzen (NSAIDS) wie Fenoporfen, Ibuprofen, Indomethacin, Naproxen, Phenylbutazon und andere antivirale Agentien wie Acyclovir, Ganciclovir und Zidovudin, Antimykotika wie Clotrimazol, Flucytosin, Griseofulvin, Ketoconazol, Miconazol, Nystatin, Terbinafin, antiprozoale Agentien wie Chloroquin, Mefloquin, Quinin, des weiteren natürliche Terpenoide wie Hippocaesculin, Barringtogenol-C21-angelat, 14-Dehydroagrostistachin, Agroskerin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceanole A, B und C, Bruceantinoside C, Yadanzioside N und P, Isodeoxyelephantopin, Tomenphantopin A und B, Coronarin A, B, C und D, Ursolsäure, Hyptatsäure A, Zeorin, Iso-Iridogermanal, Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, des weiteren Cymarin, Apocymarin, Aristolochsäure, Anopterin, Hydroxyanopterin, Anemonin, Protoanemonin, Berberin, Cheliburinchlorid, Cictoxin, Sinococulin, Bombrestatin A und B, Cudraisoflavon A, Curcumin, Dihydronitidin, Nitidinchlorid, 12-beta-Hydroxypregnadien 3,20-dion, Bilobol, Ginkgol, Ginkgolsäure, Helenalin, Indicin, Indicin-N-oxid, Lasiocarpin, Inotodiol, Glykosid 1a, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Oxoushinsunin, Aristolactam-All, Bisparthenolidin, Periplocosid A, Ghalakinosid, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Akagerin, Dihydrousambaraensin, Hydroxyusambarin, Strychnopentamin, Strychnophyllin, Usambarin, Usambarensin, Berberin, Liriodenin, Oxoushinsunin, Daphnoretin, Lariciresinol, Methoxylariciresinol, Syringaresinol, Umbelliferon, Afromoson, Acetylvismion B, Desacetylvismion A, Vismion A und B und schwefelhaltige Aminosäuren wie Cystin sowie Salze, Hydrate, Solvate, Enantiomere, Racemate, Enantiomerengemische, Diastereomerengemische; Metaboliten und Mischungen der vorgenannten Wirkstoffe.

Die Wirkstoffe werden einzeln oder kombiniert in gleicher oder unterschiedlicher Konzentration eingesetzt. Besonders bevorzugt sind Wirkstoffe, welche neben ihrer antiproliferativen Wirkung weitere Eigenschaften aufweisen. Zudem bevorzugt ist eine Kombination mit den Wirkstoffen Tacrolimus, Paclitaxel und seine Derivate, Fasudil®, Vitronektinrezeptorantagonisten, Thalidomid, Cyclosporin A, Tergurid, Lisurid, Celecoxip, R-Lys-Verbindungen und deren Derivate/Analoga als auch wirksame Metaboliten. Insbesondere bevorzugt ist eine Kombination von Rapamycin mit Tergurid oder Rapamycin mit Lisurid oder Rapamycin mit Paclitaxel oder von Rapamycin mit einem Immunsuppressivum wie z.B. Cyclosporin A. Insbesondere bevorzugt ist eine Wirkstoffkombination von Rapamycinmit Paclitaxel, Derivaten von Paclitaxel, insbesondere die hydrophilen Derivate von Paclitaxel, Epothilon, Tergurid oder Lisurid.

Der Wirkstoff ist bevorzugt in einer pharmazeutisch aktiven Konzentration von 0,001-10 mg pro cm² Stentoberfläche enthalten. Weitere Wirkstoffe können in ähnlicher Konzentration in derselben oder in weiteren Schichten enthalten sein, wobei bevorzugt ist, wenn der oder die weiteren Wirkstoffe in einer anderen Schicht als Rapamycin enthalten sind.

### Polymere

Wird der Wirkstoff bzw. Wirkstoffkombination nicht direkt auf oder in den Stent aufgebracht, können neben der hämokompatiblen Konditionierung der Oberfläche mit geeigneten hämokompatiblen Substanzen synthetischer, semisynthetischer und/oder nativer Herkunft biostabile und/oder bioabbaubare Polymere bzw. Polysaccharide als Träger bzw. als Matrix verwendet werden.
Als in der Regel biologisch stabile und nur langsam biologisch abbaubare Polymere können genannt werden: Polyacrylsäure und Polyacrylate wie Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyvinylaromaten, Polyvinylester, Polyvinylpyrollidone, Polyoxymethylene, Polyethylen, Polypropylen, Polytetrafluorethylen, Polyurethane, Polyolefin-Elastomere, Polyisobutylene, EPDM-Gummis, Fluorosilicone, Carboxymethylchitosan, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetat-butyrate, Ethylvinylacetat-copolymere, Polysulfone, Polyethersulfone, Epoxyharze, ABS-Harze, EPDM-Gummis, Siliconpräpolymere, Silicone wie Polysiloxane, Polyvinylhalogene und Copolymere, Celluloseether, Cellulosetriacetate, Chitosan, Chitosanderivate, polymerisierbare Öle wie z.B. Leinöl und Copolymere und/oder Mischungen derselben.

Als in der Regel biologisch abbaubare oder resorbierbare Polymere können beispielsweise verwendet werden: Polyvalerolactone, Poly-ε-Decalactone, Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride wie Polymaleinsäureanhydride, Polyhydroxymethacrylate, Fibrin, Polycyanoacrylate, Polycaprolactondimethylacrylate, Poly-b-Maleinsäure Polycaprolactonbutyl-acrylate, Multiblockpolymere wie z.B. aus Oligocaprolactondiole und Oligodioxanondiole, Polyetherester-multiblockpolymere wie z.B. PEG und Poly(butylenterephtalat. Polypivotolactone, Polyglycolsäuretrimethyl-carbonate Polycaprolacton-glycolide, Poly(g-ethylglutamat), Poly(DTH-Iminocarbonat), Poly(DTE-co-DT-carbonat), Poly(Bisphenol A-iminocarbonat), Polyorthoester, Polyglycol-säuretrimethyl-carbonate, Polytrimethylcarbonate, Polyiminocarbonate, Poly(N-vinyl)-Pyrolidon, Polyvinylalkohole, Polyesteramide, glycolierte Polyester, Polyphosphoester, Polyphosphazene, Poly[p-carboxyphenoxy)propan], Polyhydroxypentansäure, Polyethylenoxid-propylenoxid, weiche Polyurethane, Polyurethane mit Aminosäurereste im Backbone, Polyetherester wie das Polyethylenoxid, Polyalkenoxalate, Polyorthoester sowie deren Copolymere, Carrageenane, Fibrinogen, Stärke, Kollagen, protein-basierte Polymere, Polyaminosäuren, synthetische Polyaminosäuren, Zein, modifiziertes Zein, Polyhydroxyalkanoate, Pectinsäure, Actinsäure, modifiziertes und unmodifiziertes Fibrin und Casein, Carboxymethylsulfat, Albumin, Hyaluronsäure, Heparansulfat, Heparin, Chondroitinsulfat, Dextran, b-Cyclodextrine, Copolymere mit PEG und Polypropylenglycol, Gummi arabicum, Guar, Gelatine, Collagen, Collagen-N-Hydroxysuccinimid, Lipide und Lipoide, polymersierbare Öle mit geringem Vernetzungsgrad, Modifikationen und Copolymere und/oder Mischungen der vorgenannten Substanzen.

Bevorzugte Polymere als Träger für Rapamycin bzw. Polymere für die Einlagerung von Rapamycin sind Polylactide, Polyglycolide, Copolymere der Polylactide und Polyglycolide, Polyhydroxybutyrate, Polyhydroxymethacrylate, Polyorthoester, glycolierte Polyester, Polyvinylalkohole, Polyvinylpyrrolidon, Acrylamid-Acrylsäurecopolymere, Hyaluronsäure, Heparansulfat, Heparin, Chondroitinsulfat, Dextran, ß-Cyclodextrine, hydrophil vernetzte Dextrine, Alginate, Phospholipide, Carbomere, vernetzte Peptide und Proteine, Silikone, Polyethylenglycol (PEG), Polypropylenglycol (PPG), Copolymere aus PEG und PPG, Collagen, polymersierbare Öle und Wachse , wie deren Mischungen und Copolymere.

Des weiteren sind Polyester, Polylactide sowie Copoymere aus Diolen und Estern bzw. Diolen und Lactiden bevorzugt. Als Diole werden beispielsweise Ethan-1,2-diol, Propan-1,3-diol oder Butan-1,4-diol eingesetzt.

Erfindungsgemäß finden insbesondere Polyester für die polymere Schicht Verwendung. Aus der Gruppe der Polyester sind wiederum solche Polymere bevorzugt, welche die folgende Wiederholungseinheit besitzen:

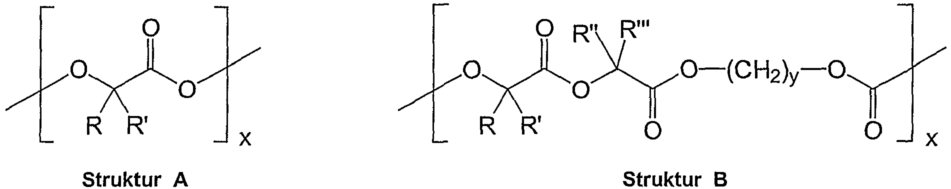

In den gezeigten Wiederholungseinheiten bedeutet R, R', R" und R'" einen Alkylrest mit 1 bis 5 Kohlenstoffatomen, insbesondere Methyl, Ethyl, Propyl, Isopropyl, n-Butyl, s-Butyl, t-Butyl, iso-Butyl, n-Pentyl oder Cyclopentyl und bevorzugt Methyl oder Ethyl. Y steht für eine ganze Zahl von 1 bis 9 und X steht für den Polymerisationsgrad. Insbesondere bevorzugt sind die folgenden Polymere mit den gezeigten Wiederholungseinheiten:

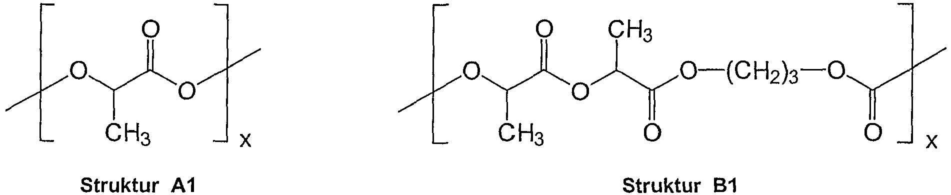

Als weitere Vertreter der resorbierbaren Polymere Resomer® seien genannt die Poly(L-lactid)e mit der allgemeinen Formel -(C6H8O4)n- wie L 210, L 210 S, L 207 S, L 209 S, die Poly(L-lactid-co-D,L-lactid)e mit der allgemeinen Formel -(C6H8O4)n-wie LR 706, LR 708, L 214 S, LR 704, die Poly(L-lactid-co-trimethylcarbonat)e mit der allgemeinen Formel -[(C6H8O4)x-(C4H6O3)y]n- wie LT 706, die Poly(L-lactid-co-glycolid)e mit der allgemeinen Formel -[(C6H8O4)x-(C4H4O4)y]n- wie LG 824, LG 857, die Poly(L-lactid-co-ε-caprolacton)e mit der allgemeinen Formel -[(C6H8O4)x-(C6H10O2)y]n- wie LC 703, die Poly(D,L-lactid-co-glycolid)e mit der allgemeinen Formel -[(C6H804)x-(C4H4O4)y]n- wie RG 509 S, RG 502 H, RG 503 H, RG 504 H, RG 502, RG 503, RG 504, die Poly(D,L-lactid)e mit der allgemeinen Formel -(C6H8O4)n- wie R 202 S, R 202 H, R 203 S und R 203 H. Resomer® 203 S stellt hierbei den Nachfolger des insbesondere bevorzugten Polymers Resomer® R 203 dar. Der Name Resomer® repräsentiert ein hochtechnologisches Produkt der Firma Boehringer Ingelheim.

Grundsätzlich ist die Verwendung von resorbierbaren Polymeren bei der vorliegenden Erfindung besonders bevorzugt. Ferner sind Homopolymere der Milchsäure (Polylactide) sowie Polymere bevorzugt, die Milch- und Glykolsäure hergestellt werden.

Es hat sich überraschenderweise gezeigt, dass bei Verwendung der Resomere, Polylactide, Polymere der Struktur A bzw. A1, Polymere der Struktur B bzw. B1 sowie den Copolymeren aus Milchsäure und Glykolsäure (PLGAs) eine für die Heilung vorteilhafte Freisetzung von Rapamycin erreicht wird. Wie man in der Elutionskurve erkennen kann, findet in den ersten Wochen eine kontinuierliche gleichmässig ansteigende Elution des Wirkstoffes statt, danach wird die Elutionskurve steiler und die Elution des Rapamycin findet schneller statt. Diese Tatsache ist von grossem Vorteil. In der ersten Phase nach einer Gefässweitung wird eine regelmässig steigende geringe Menge Rapamycin freigesetzt, die zur moderaten Unterdrückung einer überschiessenden Entzündungsreaktionen verhilft, diese notwendige Reaktion aber nicht unterdrückt. Nach den ersten entscheidenden Wochen wird ansschliessend durch die schnellere Freisetzung weiterer Mengen an Rapamycin jede erhöhte Proliferationsreaktion und immer noch vorhandene Entzündungsparameter eingedämmt.

### Rapamycin und PVA

Somit ist eine vorteilhafte Ausführungsform der vorliegenden Erfindung ein mit Rapamycin beschichteter Stent, der auf der Stentoberfläche eine reine Wirkstoffschicht aus Rapamycin besitzt, welche von einer Schutzschicht aus einem bioresorbierbaren Polymer und vorzugsweise von einer Schutzschicht aus einem Resomer, Polyvinylalkohol (PVA), Polylactide, Polymere der Struktur A1, Polymere der Struktur A2 sowie den Copolymeren aus Milchsäure und Glykolsäure (PLGA) oder Mischungen der vorgenannten Polymer bedeckt ist. Weitere Beispiele für bioresorbierbare Polymere sind unter erwähnt.
Die Eigenschaften des Topcoat bestimmen die Freisetzung des darunterliegenden Rapamycin und sind auch für die Stabilität und damit die Lagerstabilität des beschichteten Stents versentlich verantwortlich. Somit lässt sich der Beginn der Elution zeitlich verändern, während die Freisetzung selbst stark beschleunigt wird, so dass in kürzerer Zeit mehr Rapamycin eluiert.
So ist beispielsweise bei Verwendung von Polyvinylakohol als Schutzschicht für Rapamycin nach drei Tagen vollständig eluiert.
Durch die Zugabe von Rapamycin in das Topcoat lässt sich eine noch höhere Dosierung erreichen.

Die reine Rapamycinschicht ist vorzugsweise vollständig mit einer bioresorbierbaren, d.h. biologisch abbaubaren Polymerschicht überzogen.

In einer weiteren bevorzugten Ausführungsform kann sich direkt auf der Stentoberfläche und unter der reinen Wirkstoffschicht aus Rapamycin noch eine hämokompatible Beschichtung befinden. Als hämokompatible Substanzen können die hierin erwähnten verwendet werden, wobei die unten erwähnten Heparin-Derivate oder Chitosan-Derivate der allgemeinen Formeln Ia oder Ib sowie die unten beschriebenen Oligo- und Polysaccharide, welche zu über 95% die Zuckerbausteine N-Acylglucosamin und Uronsäure (bevorzugt Glucuronsäure und Iduronsäure) oder N-Acylgalactosamin und Uronsäure enthalten, bevorzugt sind. Somit ist ein Stent mit einer vorzugsweise kovalent gebundenen hämokompatiblen Beschichtung und einer darauf befindlichen reinen Rapamycinschicht mit einer äußeren bioabbaubaren Schutzschicht eine bevorzugte Ausführungsform.

Bei einer weiteren bevorzugten Ausführungsform wird der Stent mit einer reinen Rapamycinschicht versehen, worauf eine bioresorbierbare Schicht aufgebracht wird, wobei auf diese bioresorbierbare Schicht eine erneute Wirkstoffschicht aus Rapamycin aufgetragen wird, welche wiederum mit einer biologisch abbaubaren Schicht versehen wird. Somit sind Stents bevorzugt, welche eine alternierende Schichtfolge aus Rapamycin und bioresorbierbarer Schicht ausweisen, wobei zwischen 3 bis 10 Schichten möglich sind. In der Regel ist als äußere Schicht eine Schutzschicht bevorzugt, wobei die äußere Schicht jedoch auch eine Rapamycinschicht sein kann. Für die bioresorbierbaren Schichten können dieselben bioresorbierbaren Polymere eingesetzt werden oder zur Erzeugung unterschiedlich schnellen Abbaus der einzelnen Schichten auch unterschiedliche bioresorbierbare Polymere verwendet werden, wobei bevorzugt ist, wenn die Abbaugeschwindigkeit von der äußeren zur innersten Schicht oder von der innersten zur äußeren Schicht zunimmt. Auch bei den Vielschichtsystemen kann eine untere vorzugsweise kovalent auf die Stentoberfläche gebundene hämokompatible Schicht verwendet werden.

Des weiteren sind auch beschichtete Katheterballons bevorzugt, welche eine reine Wirkstoffschicht aus Rapamycin und eine darüberliegende Schutzschicht aus einem bioresorbierbaren Polymer ausweisen. Bei Katheterballon sind Zweischichtsysteme bevorzugt.

Bei einer weiteren Ausführungsform wird anstelle des bioresorbierbaren Polymeren ein Kontrastmittel oder Kontrastmittelanalogon (kontrastmittelähnlicher Stoff) verwendet. Als Kontrastmittel können die unter erwähnten Verbindungnen eingesetzt werden.

Bevorzugt sind somit Katheterballons oder Stents, welche eine reine Rapamycinschicht und eine darüberliegende Kontrastmittelschicht aufweisen.

Ferner können die Stents auch eine alternierende Abfolge an Rapamycinschichten und Kontrastmittelschichten aufweisen und optional kann der Stent eine vorzugsweise kovalent auf die Stentoberfläche gebundene hämokompatible Schicht vorzugsweise aus den hierin erwähnten hämokompatiblen Stoffen besitzen.

Die Rapamycinschicht und die Kontrastmittelschicht oder die Schicht aus bioresorbierbarem Polymer werden vorzugsweise im Sprühverfahren auf den Stent bzw. Katheterballon aufgebracht, wobei der Katheterballon im expandierten als auch im komprimierten Zustand beschichtet werden kann.

Derartige Zweischichtsysteme oder Mehrschichtsysteme auf einem Stent oder derartige Zweischichtsysteme auf einem Katheterballon werden hergestellt, indem man die vorzugsweise unbeschichtete oder mit einer hämokompatiblen Schicht beschichtete Oberfläche des Stents oder die vorzugsweise unbeschichtete Oberfläche des Katheterballons mit einer Lösung enthaltend Rapamycin besprüht und diese so erzeugte Wirkstoffschicht vorzugsweise nach dem Trocknen mit einer Lösung des Polymeren der Schutzschicht in einem polaren Lösungsmittel besprücht, welches einen Wasseranteil von weniger als 50 Vol.-%, bevorzugt weniger als 40 Vol.-% und insbesondere bevorzugt weniger als 30 Vol.-% aufweist.

Geeignete Lösungsmittel für das Polymer insbesondere das hydrophile Polymer der Schutzschicht sind hydrophile Lösungsmittel und vorzugsweise Aceton, Butanon, Pentanon, Tetrahydrofuran (THF), Essigsäureethylester (Ethylacetat), Methanol, Ethanol, Propanol, iso-Propanol sowie Gemische der vorgenannten Lösungsmittel, welche einen Gassergehalt von 1 Vol.-% bis 50 Vol.-%, bevorzugt 5 Vol.-% bis 40 Vol.-% und insbesondere bevorzugt 10 Vol.-% bis 30 Vol.-% aufweisen.

Derartig hergestellte Beschichtungssysteme sind den bekannten Beschichtungen hinsichtlich Stabilität des Rapamycins und der Elutionskinetik überlegen.

### Rapamycin und Polysulfon

Die Verwendung von Polysulfonen hat den entscheidenden Vorteil, dass das Polysulfon selbst sehr gute hämokompatible Eigenschaften besitzt und zudem biostabil ist, d.h. eine dauerhafte Beschichtung der Stentoberfläche vorliegt, welche hämokompatibel ist und nicht biologisch abgebaut wird und ferner als Wirkstoffträger für das Rapamycin fungiert.

Polysulfon hat den entscheidenen Vorteil, dass es keine Gefahr von Spätthrombosen hervorruft, welche andere Beschichtungssysteme haben können, wodurch polymerbeschichtete wirkstofffreisetzende Stents in der Vergangenheit negative Schlagzeilen gemacht haben.

Polysulfon als biologisch stabile Beschichtung, welche nach Implantation des Stent im Körper des Patienten nicht oder nur extrem langsam abgebaut wird, hat hingegen den Nachteil, Rapamycin nicht in ausreichendem Masse freizusetzen. Um nun eine ausreichende Freisetzung von Rapamycin zu gewährleisten, wird dem Polysulfon erfindungsgemäß ein bestimmter Anteil eines hydrophilen oder in Methanol quellbaren Polymeren zugesetzt.

Durch Beimischung von hydrophilen Polymeren lassen sich unterschiedliche Verfahren zur gezielten Applikation von Rapamycin bzw. Kombinationen mit anderen bevorzugten Wirkstoffen erreichen. Während bei einer Konzentration von 01-1% das hydrophile Polymer in Form von kleinen Poren in der Polysulfonmatrix verteilt ist, wird bei steigendem Anteil des hydrophilen Polymeren die Durchlässigkeit des Polysulfons höher, so dass sich ab einer kritischen Konzentration auch Kanäle bilden, die bis zur Oberfläche gelangen. Die kritische Konzentration für die Ausbildung von Kanälen liegt je nach hydrophilem Polymer bei 3 Gew.-% bis 8 Gew.-% bezogen auf die Masse der gesamten Beschichtung oder bezogen auf die Masse an Polysulfon und hydrophilem Polymer.

Befindet sich ein derart beschichteter Stent in einem Gefäss, kommt er mit dem wässrigen Medium wie den Körperflüssigkeiten in Kontakt und der hydrophile Wirkstoff nimmt Flüssigkeit auf. Dadurch baut sich innerhalb der Kanäle und Wirkstoffreservoirs ein Überdruck auf, so dass die Elution des ebenfalls hydrophilen Wirkstoffs in Form einer "Injektion" gezielt an und in die Gefässwand erfolgt. Zusätzlich kann die nicht quellende Matrix ebenfalls Rapamycin oder einen anderen bevorzugten Wirkstoff bzw. eine Kombination aus Rapamycin und einem anderen Wirkstoff enthalten und damit die langfristige Regulierung des Heilungsprozesses unterstützen.

Beispiele für hydrophile Polymere werden unter gegeben und sind dem Fachmann auch durchaus bekannt. Als hydrophile Polymere werden hierin solche Polymere bezeichnet, welche in Methanol löslich oder zumindest quellbar sind. Unter quellbar wird die Fähigkeit des Polymeren verstanden, Methanol in das Polymergerüst aufzunehmen, wodurch das Volumen des polymeren Materials sich vergrößert.

Um nun eine geeignete Elutionskinetik des Rapamycins aus dem Polysulfon zu erzeugen, werden dem Polysulfon 0,1 Gew.-% bis 50 Gew.-%, vorzugsweise 1,0 Gew.-% bis 30 Gew.-% und insbesondere bevorzugt 5 Gew.-% bis 20 Gew.-% eines in Methanol quellbaren Polymeren zugesetzt. Grundsätzlich gilt, dass mit steigenden Gehalt an hydrophilem bzw. in Methanol quellbarem Polymer die Neigung zur Kanalbildung in der Polysulfonbeschichtung zunimmt.

Geeignete in Methanol quellbare Polymere sind unter aufgelistet.
Geeignete Beispiele sind folgende Gemische:
- Polysulfon mit 2 Gew.-% Polyvinylpyrollidon (PVP)
- Polysulfon mit 11 Gew.-% Glycerin
- Polysulfon mit 8 Gew.-% Polyethylenglykol
- Polysulfon mit 6 Gew.-% Polyvinylalkohol
- Polysulfon mit 5 Gew.-% Polyhydroxyethyl-methacrylate
- Polysulfon mit 7 Gew.-% Polyacrylamid
- Polysulfon mit 4 Gew.-% Polylactid
- Polysulfon mit 9 Gew.-% Polyesteramide
- Polysulfon mit 1 Gew.-% Chondroitinsulfat
- Polysulfon mit 8 Gew.-% Polyhydroxybutyrate

Das in Methanol quellbare Polymer erzeugt nach Implantation des Stent Risse und Kanäle in der Polysulfonbeschichtung, welche zur Freisetzung des Rapamycin dienen und somit trotz biostabiler Polysulfonbeschichtung zu einer angemessenen Freisetzungsrate von Rapamycin nach Stentimplantation führen.Geeignete Polysulfone für die biostabile Beschichtung werden weiter unter ausführlich diskutiert.

Die erfindungsgemäßen Stents werden hergestellt indem man einen vorzugsweise unbeschichteten Stent bereitstellt und diesen mit einer Lösung von Polysulfon und Rapamycin und dem in Methanol quellbaren oder hydrophilen Polymer in einem geeigneten Lösungsmittel (Methylenchlorid (Dichlormethan), Methylacetat, Trichlorethylen: Methylenchlorid 1:1 (v/v), Chloroform, Dimethylformamid, Ethanol, Methanol, Aceton, THF, Ethylacetat, usw.) besprüht. Der Sprühvorgang kann kontinuierlich oder sequenziell mit Trocknungsschritten zwischen den Sprühschritten erfolgen oder die Beschichtung kann auch im Tauchverfahren, Streichverfahren oder Plasmaverfahren aufgebracht werden.

Bevorzugt werden bei dieser Ausführungsform Kombinationen von Polysulfon mit den hydrophilen Polymeren, die in den gleichen organischen Lösungsmitteln löslich sind wie Polysulfon. Somit kann der Fachmann leicht ein geeignetes Co-Polymer für das Polysulfon ermitteln, indem er das Lösungsverhalten des gewählten Polysulfons (geeignete und auch bevorzugte Polysulfone werden weiter unten eingehend beschrieben) ermittelt und danach überprüft, ob das gewählte Co-Polymer ähnliche Lösungseigenschaften aufweist. Die Lösungseigenschaften sind dann als ähnlich zu betrachten, wenn sich die gelöste Menge an Polysulfon K pro Volumeneinheit Lösungsmittel (z.B. pro 1 ml) zu der gelösten Menge J an Co-Polymer pro derselben Volumeneinheit Lösungsmittel (z.B. 1 ml) wie folgt verhält: 0,5K < J < 2K.

Beispiele für geeignete hydrophile bzw. in Methanol quellbare Polymere werden aus der Gruppe ausgewählt umfassend oder bestehend aus: Polyvinylpyrrolidon, Polylactide, Pectine, Glycerin, Polyethylenglykol, Polypropylenglycol, Polyvinylalkohol, Polyhydroxyethylmethacrylate, Polyacrylamid, Polyvalerolactone, Poly-ε-Decalactone, Polylactonsäure, Polyglycolsäure Polylactide, Polyglycolide, Copolymere der Polylactide und Polyglycolide, Poly-s-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride wie Polymaleinsäure-anhydride, Fibrin, Polycyanoacrylate, Polycaprolactondimethylacrylate, Poly-b-Maleinsäure Polycaprolactonbutyl-acrylate, Multiblockpolymere aus Oligocaprolactondiole und Oligodioxanondiole, Polyetherestermultiblockpolymere aus PEG und Poly(butylenterephtalat), Polypivotolactone, Polyglycolsäuretrimethyl-carbonate, Polycaprolacton-glycolide, Poly(g-ethylglutamat), Poly(DTH-Iminocarbonat), Poly(DTE-co-DT-carbonat), Poly(Bisphenol A-iminocarbonat), Polyorthoester, Polyglycol-säuretrimethyl-carbonate, Polytrimethylcarbonate, Polyiminocarbonate, Poly(N-vinyl)-Pyrrolidon, Polyvinylalkohole, Polyesteramide, glycolierte Polyester, Polyphosphoester, Polyphosphazene, Poly[p-carboxyphenoxy)propan], Polyhydroxypentansäure, Polyanhydride, Polyethylenoxid-propylenoxid, weiche Polyurethane, Polyurethane mit Aminosäurereste im Backbone, Polyetherester, Polyethylenoxid, Polyalkenoxalate, Polyorthoester sowie deren Copolymere, Lipide, Carrageenane, Fibrinogen, Stärke, Kollagen, protein-basierende Polymere, Polyaminosäuren, synthetische Polyaminosäuren, Zein, modifiziertes Zein, Polyhydroxyalkanoate, Pectinsäure, Actinsäure, modifiziertes und unmodifiziertes Fibrin und Casein, Carboxymethylsulfat, Albumin, Hyaluronsäure, Chitosan und seine Derivate, Chondroitinsulfat, Dextrane, b-Cyclodextrine, und Copolymere mit PEG und Polypropylenglycol, Gummi arabicum, Guar, Gelatine, Collagen Collagen-N-Hydroxysuccinimid, Lipide, Phospholipide, Modifikationen und Copolymere und/oder Mischungen der vorgenannten Substanzen.

Besonders bevorzugt werden Polyvinylpyrrolidon, Polyethylenglycol, Polylactide und -glycolide und deren Copolymere.
Bevorzugte Verwendung als Lösungsmittel finden Chloroform, Dichlormethan und Methylenchlorid, Aceton und Methylacetat, wobei insbesondere Chloroform bevorzugt ist. Der Anteil des Rapamycin in der Beschichtungslösung (bevorzugt Sprühlösung) liegt zwischen 60% Gew.-% und 10% Gew.-% bevorzugt zwischen 50% Gew.-% und 20% Gew.-%, besonders bevorzugt zwischen 40% Gew.-% und 30% Gew.-% bezogen auf die Masse der gesamten Beschichtung.

Ferner ist bevorzugt, wasserfreie, d.h. getrocknete Lösungsmittel oder Lösungsmittel mit einem Wassergehalt von weniger als 2 Vol.-%, vorzugsweise weniger als 1 Vol.-% und insbesondere bevorzugt weniger als 0,2 Vol.-% zu verwenden. Darüber hinaus hat es sich als vorteilhaft erwiesen, die Beschichtung unter Lichtausschluss durchzuführen, um eine Zersetzung des Rapamycins zu vermeiden und eine bessere Kontrolle über die Menge an aktivem Rapamycin in der Beschichtung zu haben. Ferner ist vorteilhaft, die Beschichtung in einer trockenen, d.h. wasserfreien Umgebung auszuführen und als Trägergas für die Beschichtung ein Inertgas wie z.B. Stickstoff oder Argon anstelle von Luft zu verwenden. Die vorliegende Erfindung betrifft somit auch beschichtete Stents, welche gemäß den vorgenannten Bedingungen beschichtet worden sind.

### Rapamycin und PLGA

Eine weitere bevorzugte Ausführungsform ist ein polymerer PLGA Träger für Rapamycin auf Stents.
PLGA bezeichnet ein Blockcopolymer aus Polylactid und Polyglycolsäure (Polyglycolid) der folgenden allgemeinen Fromel: 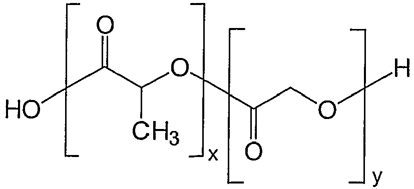 worin
x die Zahl der Milchsäurebausteine und y die Zahl der Glycolsäurebausteine angibt.

Zur Herstellung dieser Beschichtung wird Rapamycin und PLGA in einem geeigneten Lösungsmittel (Chloroform, Methanol, Aceton, THF, Ethylacetat, usw.) gelöst und auf die vorzugsweise unbeschichtete Stentoberfläche gesprüht.

Anstelle der Verwendung einer vorzugsweise unbeschichteten Stentoberfläche, kann die Stentoberfläche auch mit einer vorzugsweise kovalent verknüpften hämokompatiblen Schicht versehen sein, auf welche die Rapamycin-PLGA-Mischung aufgetragen wird.

Mit Hilfe dieser Ausführungsform lässt sich die Zuführung von Rapamycin in besonderer und überraschend einfacher Weise an den Zielort erreichen, wo es gezielt und dosiskontrolliert wirken kann.
Wie bereits zu Anfang ausgeführt, ist es wichtig, dass der eingesetzte Wirkstoff die für den Wundheilungsprozess wichtigen Entzündungsreaktionen nicht zu stark unterdrückt, weil damit die notwendige Bedingung für den einsetzenden Heilungsprozess unterdrückt wird. Also ist es vielmehr wichtig, die Entzündungsprozesse auf die Implantation möglichst zu reduzieren. Dieser Grundanspruch wird mit dieser Beschichtungsform excellent gelöst. Das Rapamycin als Entzündungshemmer und Immunsuppressivum greift in diese Prozesse ein, aber unterdrückt sie nicht.

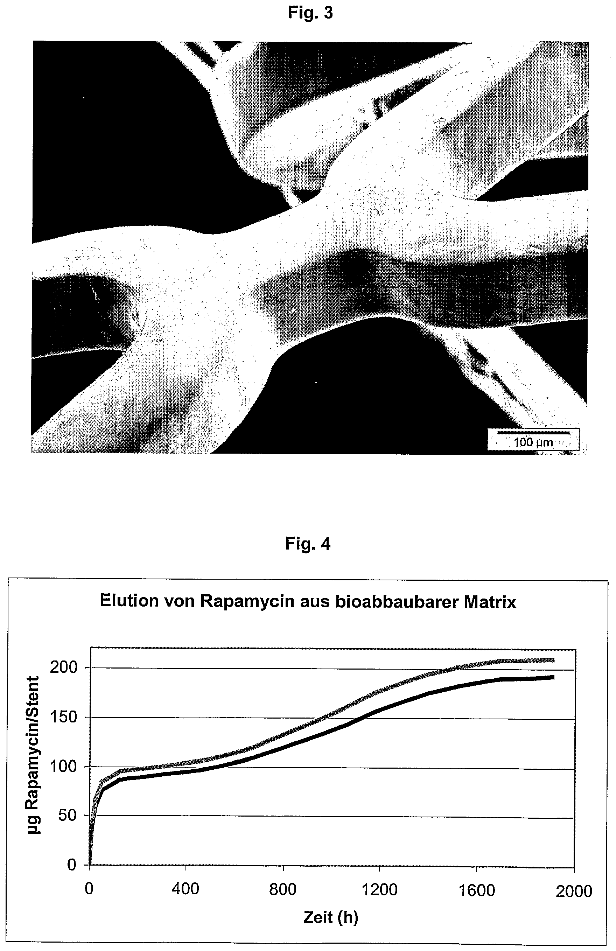

Nach der derartigen moderaten Regulation der Entzündungsvorgänge wird bis zum vollständigen Abbau des Polymeren die freigesetzte Rapamycindosis kontinuierlich erhöht. Die Elutionskurve in Fig. 4 verdeutlicht dies. Die Kurve lässt zwei Steigungen erkennen, wobei diese in der ersten Phase der Elution geringer ist als in der zweiten. Mit der in der zweiten erhöhten Freisetzung von Rapamycin wird der nächste wichtige Aspekt in der Restenoseprophylaxe berücksichtigt. Zum einen werden möglicherweise immer noch vorhandene Entzündungsbereiche im Gewebe zurückgedrängt, zum anderen wird nun die antiproliferative Wirkung des Rapamycin durch Regulation der Proliferation der Glatten Muskelzellen im Wundbereich wichtig. Im Idealfall sollte die lumenseitige Stentoberfläche von einer Endothelzellschicht überwachsen werden. Doch lässt die vermehrte Proliferationstätigkeit der Glatten Muskelzellen eine solche Schicht nicht zu und überwuchert den Stent unter Bildung von fibrotischem Gewebe. Dies führt letztendlich zu einer erneuten Erkrankung.
Mit der beschleunigten Freisetzung von Rapamycin wird die Proliferationstätigkeit der Glatten Muskelzellen reguliert und auf ein normales und notwendiges Mass der Wundschliessung reduziert.

Wird zusätzlich die Oberfläche des Stents wie bereits erwähnt mit einer kovalent gebundenen hämokompatiblen Schicht versehen, wird zusätzlich noch gewährleistet, dass während das PLGA in den folgenden Wochen nach der Implantation langsam abgebaut wird, freigelegte Bereiche vom Gerinnungssystem nicht als Fremdoberfläche erkannt werden. So wird eine athrombogene Oberfläche bereitgestellt, die für eine vollständige Maskierung der Stentoberfläche sorgt.

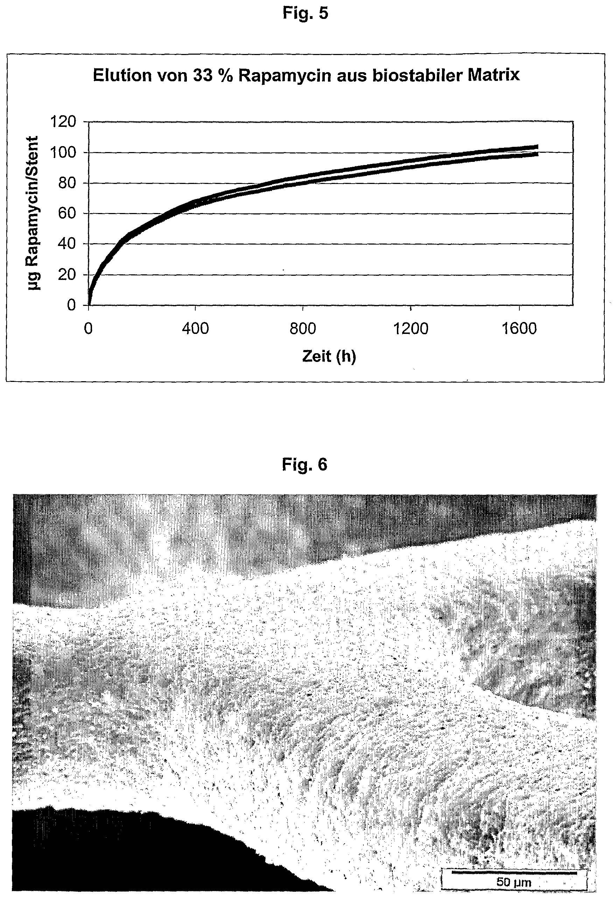

Diese ungewohnte in Fig. 4 gezeigte Elutionskinetik besonders vorteilhafte Elutionskinetik konnte bisher nur mit einem System aus PLGA als polymeren Träger für Rapamycin erhalten werden, während die normale Elutionskinetik in Fig. 5 gezeigt ist und bei den anderen Trägersystemen insbesondere den biostabilen Trägersystemen auftritt.

Die erfindungsgemäße PLGA-Rapamycin-Beschichtung wird erhalten, indem PLGA und vorzugsweise PLGA (50/50) zusammen mit dem Rapamycin in einem geeigneten plaren Lösungsmittel (wie z.B. Methylenchlorid (Dichlormethan), Methylacetat, Trichlorethylen: Methylenchlorid 1:1 (v/v), Chloroform, Dimethylformamid, Ethanol, Methanol, Aceton, THF, Ethylacetat, usw.) gelöst wird und die vorzugsweise unbeschichtete oder mit einer hämokompatiblen Schicht versehenen Stentoberfläche mit dieser Lösung besprüht wird. Der Sprühvorgang kann kontinuierlich oder sequenziell mit Trocknungsschritten zwischen den Sprühschritten erfolgen oder die Beschichtung kann auch im Tauchverfahren, Streichverfahren oder Plasmaverfahren aufgebracht werden.

Der Anteil des Rapamycin in der Beschichtungslösung (bevorzugt Sprühlösung) liegt zwischen 60% Gew.-% und 10% Gew.-% bevorzugt zwischen 50% Gew.-% und 20% Gew.-%, besonders bevorzugt zwischen 40% Gew.-% und 30% Gew.-% bezogen auf die Masse der gesamten Beschichtung.

Ferner ist bevorzugt, wasserfreie, d.h. getrocknete Lösungsmittel oder Lösungsmittel mit einem Wassergehalt von weniger als 2 Vol.-%, vorzugsweise weniger als 1 Vol.-% und insbesondere bevorzugt weniger als 0,2 Vol.-% zu verwenden. Darüber hinaus hat es sich als vorteilhaft erwiesen, die Beschichtung unter Lichtausschluss durchzuführen, um eine Zersetzung des Rapamycins zu vermeiden und eine bessere Kontrolle über die Menge an aktivem Rapamycin in der Beschichtung zu haben. Ferner ist vorteilhaft, die Beschichtung in einer trockenen, d.h. wasserfreien Umgebung auszuführen und als Trägergas für die Beschichtung ein Inertgas wie z.B. Stickstoff oder Argon anstelle von Luft zu verwenden. Die vorliegende Erfindung betrifft somit auch beschichtete Stents, welche gemäß den vorgenannten Bedingungen beschichtet worden sind.

### Ballonbeschichtung

Eine weitere bevorzugte Ausführungsform ist die Beschichtung von Ballonkathetern mit Rapamycin.

Bei der PTCA wird die verengte Stelle für eine kurze Zeit von 1-3 Minuten mit Hilfe des aufblasbaren Ballons am Ende des Katheters, wenn erforderlich mehr als zweimal wiederholt, aufgeweitet. Dabei müssen die Gefäßwände derart überdehnt werden, dass die Verengung behoben wird. Aus dieser Vorgehensweise resultieren Mikrorisse in den Gefäßwänden, die bis in die Adventitia reichen. Nach Entfernen des Katheters wird das verletzte Gefäß sich selbst überlassen, so dass dem Heilungsprozess in Abhängigkeit vom zugefügten Verletzungsgrad, der sich aus der Überdehnungsdauer, den Überdehnungswiederholungen und Überdehnungsgrad ergibt, mehr oder minder hochgradige Leistungen abverlangt werden. Dies zeigt sich in der hohen Wiederverschlussrate nach erfolgter PTCA.

Doch bestehen bei der Anwendung der PTCA Vorteile gegenüber dem Stent, nicht zuletzt deshalb, weil sich auf diese Weise zu keinem Zeitpunkt nach Durchführung der Behandlung ein Fremdkörper als zusätzliche Belastung bzw. Initiator für Folgeerscheinungen wie sie auch die Restenose ist, im Organismus befindet

Auch hier ist Rapamycin aufgrund seiner vielfältigen Wirkungsweise besonders gut geeignet. Es muss allerdings gewährleistet sein, dass der hydrophile Wirkstoff während der PTCA nicht verloren gehen kann oder beim Dilatation vorzeitig abplatzt.

Hierzu gibt es ein Verfahren, bei dem Rapamycin bzw. auch in Kombination mit anderen Wirkstoffen auf einen Ballon aufgebracht werden kann und eine gezielte Wirkstoffmenge während der Kontaktzeit von bis zu mehreren Minuten von der Gefässwand aufgenommen werden kann.

Dazu wird das Rapamycin in einem geeigneten organischen Lösungsmittel gelöst und mittels Sprüh- oder Pipettierverfahren auf den Ballon gebracht. Zusätzlich werden Hilfsstoffe in die Rapamycinlösung eingebracht, die entweder die Sichtbarmachung des Katheters gewährleisten oder als sogenannte Transportvermittler fungieren und die Aufnahme des Wirkstoffes in die Zelle beschleunigen. Dazu gehören Vasodilatoren, zu denen körpereigene Substanzen wie die Kinine, z.B. Bradykinin, Kallidin, Histamin oder die NOS-Synthase, die aus L-Arginin das vasodilatatorisch wirkende NO freisetzt. Substanzen pflanzlichen Ursprungs wie der Extrakt des Gingko biloba, DMSO, Xanthone, Flavonoide, Terpenoide, pflanzliche und tierische Farbstoffe, Lebensmittelfarben, NOfreisetzende Substanzen wie z.B. das Pentaerythrytiltetranitrat (PETN), Kontrastmittel und Kontrastmittelanaloga gehören ebenfalls zu diesen Hilfsmitteln bzw. sind selbst als Wirkstoff synergistisch einsetzbar.
Weitere zu nennende Substanzen sind 2-Pyrrolidon, Tributyl- und Triethylcitrat wie deren acteylierten Derivate, Bibutylphtalat, Benzoesäurebenzylester, Diethanolamin, Diethylphtalat, Isopropylmyristate und -palmitate, Triacetin usw.

Insbesondere bevorzugt werden DMSO, jodhaltige Kontrastmittel, PETN, Tributyl- und Triethylcitrat wie deren acteylierten Derivate, Isopropylmyristate und -palmitate, Triacetin und Benzoesäurebenzylester.

Je nach Einsatzort eines Katheters ist eine polymere Matrix notwendig. Damit wird das vorzeitige Abplatzen einer reinen Wirkstoffschicht vermieden. Verwenden lassen sich biostabile wie auch bioabbaubare Polymere, die sich aus der unten stehenden Auflistung ergeben. Als besonders bevorzugt sind Polysulfone, Polyurethane, Polylactide und Glycolide und deren Copolymere zu nennen.

### Hämokompatible Beschichtung

Zusätzlich kann die Stentoberfläche mit einer athrombogenen bzw. inerten bzw. biokompatiblen Oberfläche versehen werden, die gewährleistet, dass mit Abklingen des Wirkstoffeinflusses und Abbau der Matrix keine Reaktionen auf die bestehende Fremdoberfläche mehr einsetzen, die langzeitlich ebenfalls zu einem Wiederverschluß des Blutgefäßes führen können. Die den Stent unmittelbar bedeckende hämokompatible Schicht besteht bevorzugt aus Heparin nativen Ursprungs als auch synthetisch hergestellter Derivate unterschiedlicher Sulfatierungsgrade und Acteylierungsgrade im Molekulargewichtsbereich von den für die antithrombotische Wirkung verantwortlichen Pentasaccharides bis zum Standardmolekulargewicht des käuflichen Heparins, Heparansulfaten und dessen Derivate, Oligo- und Polysaccharide der Erythrocytenglycolcalix, die in perfekter Weise die athrombogene Oberfläche der Erythrocyten wiedergeben, da hier im Gegensatz zum Phosphorylcholin der tatsächliche Kontakt von Blut und Erythrocytenoberfläche stattfindet, Oligosacchariden, Polysacchariden, vollständig desulfatiertem und N-reacetyliertem Heparin, desulfatiertem und N-reacetyliertem Heparin, N-carboxymethyliertem und/oder partiell N-acetyliertem Chitosan, Polyacrylsäure, Polyvinylpyrrolidon, und Polyethylenglykol und/oder Mischungen dieser Substanzen. Diese Stents mit einer hämokompatiblen Beschichtung werden hergestellt, indem man herkömmliche in der Regel unbeschichtete Stents bereitstellt und bevorzugt kovalent eine hämokompatible Schicht aufbringt, welche nach Wirkstofffreigabe und damit nach Abklingen des Wirkstoffeinflusses und Abbau der Matrix die Oberfläche des Implantates permanent maskiert. Daher wird diese hämokompatible Beschichtung auch direkt auf die Stentoberfläche aufgetragen.

Somit betrifft eine bevorzugte Ausführungsform der vorliegenden Erfindung einen Stent beliebigen Materials, dessen Oberfläche durch die Auftragung der Glycocalixbestandteile von Blutzellen, Esothelzellen oder Mesothelzellen maskiert wird. Die Glycocalix ist die äußere Schicht von beispielsweise Blutzellen, Esothelzellen oder Mesothelzellen aufgrund derer diese Zellen blutverträglich (hämokompatibel) sind. Die Bestandteile dieser äußeren Schicht (Glycocalix) von Blutzellen, Esothelzellen und/oder Mesothelzellen wird von der Zelloberfläche vorzugsweise enzymatisch abgetrennt, von den Zellen separiert und als Beschichtungsmaterial für die Stents verwendet. Zu diesen Glycocalixbestandteilen zählen unter anderem Oligosaccharid-, Polysaccharid- und Lipid-Anteile der Glykoproteine, Glykolipide und Proteoglykane sowie Glykophorine, Glykosphingolipide, Hyaluronsäuren, Chondroitinsulfate, Dermatansulfate, Heparansulfate als auch Keratansulfate. Verfahren für die Isolierung und Verwendung dieser Stoffe als Beschichtungsmaterial sind ausgiebig in dem europäischen Patent EP 1 152 778 B1 der Firmengründer der Hemoteq GmbH, Herrn Dr. Michael Hoffmann und Herrn Dipl.-Chem. Roland Horres, beschrieben. Die kovalente Anbindung erfolgt wie beim Hemoparin (siehe bei den Beispielen Beispiel Nr 9 , 14)

Weitere bevorzugte Ausführungsformen weisen eine unterste direkt auf der Stentoberfläche aufgebrachte hämokompatible Beschichtung aus desulfatiertem und N-reacetyliertem Heparin und/oder N-carboxymethyliertem und/oder partiell N-acetyliertem Chitosan. Diese Verbindungen sowie die Glycocalixbestandteile haben sich in etlichen Studien bereits als sehr gute hämokompatible Beschichtung erwiesen und machen die Stentoberfläche blutverträglich nachdem die darüberliegenden Wirkstoff- und/oder Trägerschichten abgespalten oder biologisch abgebaut worden sind. Derartige besonders bevorzugte Materialien für die Beschichtung der Stentoberfläche sind in dem europäischen Patent Nr. EP 1 501 565 B1 der Firma Hemoteq AG offenbart. Auf diese untere hämokompatible Schicht werden eine oder mehrere Wirkstoffschichten und/oder wirkstofffreie oder wirkstoffenthaltende Träger- oder Polymerschichten aufgebracht.

Bei diesen Heparin-Derivaten oder Chitosan-Derivaten handelt es sich um Polysaccharide der allgemeinen Formel la 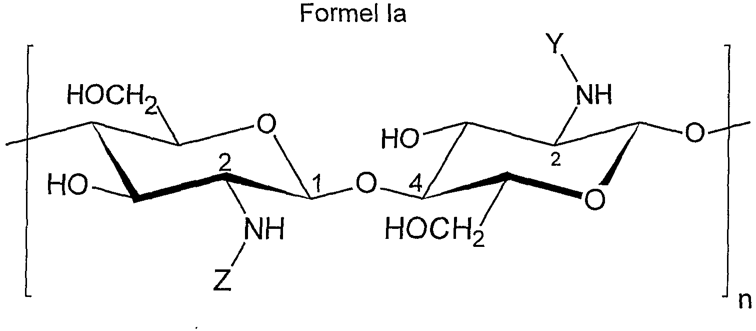 sowie strukturell sehr ähnliche Polysaccharide der allgemeinen Formel Ib

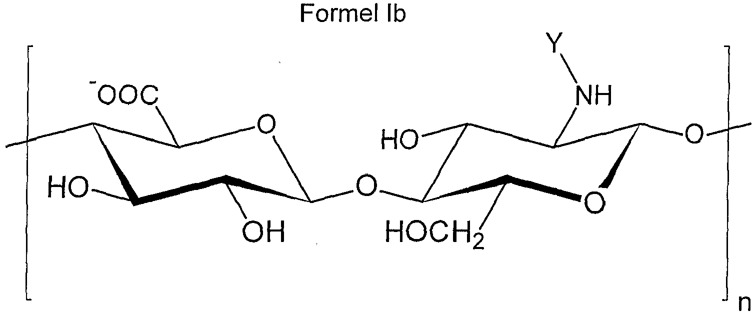

Die Polysaccharide gemäß Formel Ia weisen Molekulargewichte von 2kD bis 400kD auf, bevorzugt von 5kD bis 150kD, mehr bevorzugt von 10kD bis 100kD und insbesondere bevorzugt von 30kD bis 80kD. Die Polysaccharide gemäß Formel Ib weisen Molekulargewichte von 2kD bis 15kD auf, bevorzugt von 4kD bis 13kD, mehr bevorzugt von 6kD bis 12kD und insbesondere bevorzugt von 8kD bis 11kD. Die Variable n ist eine ganze Zahl im Bereich von 4 bis 1050. Bevorzugt ist n eine ganze Zahl von 9 bis 400, mehr bevorzugt von 14 bis 260 und insbesondere bevorzugt eine ganze Zahl zwischen 19 und 210.

Die allgemeine Formel Ia und Ib gibt ein Disaccharid wieder, welches als Grundbaustein des erfindungsgemäßen Polysaccharides anzusehen ist und durch n-fache Aneinanderreihung des Grundbausteins das Polysaccharid ergibt. Dieser aus zwei Zuckermolekülen aufgebaute Grundbaustein soll nicht dahingehend ausgelegt werden, daß unter die allgemeinen Formeln Ia und Ib nur Polysaccharide mit einer geraden Anzahl an Zuckermolekülen fallen. Natürlich umfaßt die allgemeine Formel la sowie die Formel Ib auch Polysaccharide mit einer ungeraden Anzahl an Zuckerbausteinen. Als Endgruppen der Oligo- bzw. Polysaccharide liegen Hydroxygruppen vor.

Die Reste Y und Z repräsentieren unabhängig voneinander die folgenden chemischen Acyl- oder Carboxyalkylgruppen:
-CHO, -COCH₃, -COC₂H₅, -COC₃H₇, -COC₄H₉, -COC₅H₁₁, -COCH(CH₃)₂, -COCH₂CH(CH₃)₂, -COCH(CH₃)C₂H₅, -COC(CH₃)₃, -CH₂COO⁻, -C₂H₄COO⁻, -C₃H₆COO⁻, -C₄H₈COO⁻.

Bevorzugt sind die Acylreste -COCH₃, -COC₂H₅, -COC₃H₇ sowie die Carboxyalkylreste -CH₂COO⁻, -C₂H₄COO⁻, -C₃H₆COO⁻. Mehr bevorzugt sind Acetyl- und Propanoylgruppe sowie der Carboxymethyl- und Carboxyethylrest. Insbesondere bevorzugt sind die Acetylgruppe und der Carboxymethylrest.
Zudem ist bevorzugt, wenn der Rest Y eine Acylgruppe und der Rest Z eine Carboxyalkylgruppe repräsentiert. Mehr bevorzugt ist, wenn Y ein Rest -COCH₃, -COC₂H₅ oder -COC₃H₇ und insbesondere -COCH₃ ist. Zudem ist ferner bevorzugt, wenn Z ein Carboxyethyl- oder Carboxymethylrest ist, wobei der Carboxymethylrest insbesondere bevorzugt ist.

Der gemäß Formel Ia gezeigte Disaccharidgrundbaustein enthält jeweils einen Substituenten Y und einen weiteren Rest Z. Dies soll verdeutlichen, daß das erfindungsgemäße Polysaccharid zwei verschiedene Reste, nämlich Y und Z enthält. Dabei soll die allgemeine Formel Ia gerade nicht nur Polysaccharide umfassen, welche die Reste Y und Z in streng alternierender Abfolge enthalten, wie sich aus der Aneinanderreihung der Disaccharidgrundbausteine ergeben würde, sondern auch Polysaccharide, welche die Reste Y und Z in vollständig statistischer Abfolge an den Aminogruppen tragen. Ferner soll die allgemeine Formel la auch solche Polysaccharide umfassen, die die Reste Y und Z in unterschiedlicher Anzahl enthalten. Verhältnisse zwischen der Anzahl an Resten Y zu der Anzahl an Resten X können zwischen 70% : 30%, bevorzugt zwischen 60% : 40% und besonders bevorzugt zwischen 45% : 55% liegen. Insbesondere bevorzugt sind derartige Polysaccharide der allgemeinen Formel la, welche im wesentlichen an der Hälfte der Aminogruppen den Rest Y und an der anderen Hälfte der Aminogruppen den Rest Z in einer rein statistischen Verteilung tragen. Der Begriff "im wesentlichen die Hälfte" bedeutet im Idealfall exakt 50%, soll jedoch den Bereich von 45% bis 55% und insbesondere 48% bis 52% mit umfassen.

Bevorzugt sind die Verbindungen der allgemeinen Formel la, worin die Reste Y und Z folgende Bedeutung haben:

| | | |
|---|---|---|
| Y = -CHO | und | Z = -C₂H₄COO⁻ |
| Y = -CHO | und | Z = -CH₂COO⁻ |
| Y = -COCH₃ | und | Z = -C₂H₄COO⁻ |
| Y = -COCH₃ | und | Z = -CH₂COO⁻ |
| Y = -COC₂H₅ | und | Z = -C₂H₄COO⁻ |
| Y = -COC₂H₅ | und | Z = -CH₂COO⁻ |

Insbesondere bevorzugt sind die Verbindungen der allgemeinen Formel la, worin die Reste Y und Z folgende Bedeutung haben:

| | | |
|---|---|---|
| Y = -CHO | und | Z = -C₂H₄COO⁻ |
| Y = -COCH₃ | und | Z = -CH₂COO⁻ |

Bevorzugt sind die Verbindungen der allgemeinen Formel Ib, worin Y eine der folgenden Gruppen ist: -CHO, -COCH₃, -COC₂H₅ oder -COC₃H₇. Weiter bevorzugt sind die Gruppen -CHO, -COCH₃, -COC₂H₅ und insbesondere bevorzugt ist die Gruppe -COCH₃.
Die Verbindungen der allgemeinen Formel Ib enthalten nur noch einen geringen Anteil an freien Aminogruppen. Da mit dem Ninhydrintest keine freien Aminogruppen mehr nachgewiesen werden konnten, kann aufgrund der Empfindlichkeit dieses Tests geschlossen werden, dass weniger als 2%, bevorzugt weniger als 1% und insbesondere bevorzugt weniger als 0,5% aller -NH-Y-Gruppen als freie Aminogruppen vorliegen, d.h. bei diesem geringen Prozentsatz der Gruppen -NH-Y bedeutet Y Wasserstoff.

Da die Polysaccharide der allgemeinen Formeln Ia und Ib Carboxylatgruppen und Aminogruppen enthalten, umfassen die allgemeinen Formlen Ia und Ib auch Alkalisowie Erdalkalimetallsalze der entsprechenden Polysaccharide. So können Alkalimetallsalze wie das Natriumsalz, das Kaliumsalz, das Lithiumsalz oder Erdalkalimetallsalze wie beispielsweise das Magnesiumsalz oder das Calciumsalz genannt werden. Ferner können mit Ammoniak, primären, sekundären, tertiären und quaternären Aminen, Pyridin und Pyridinderivaten Ammoniumsalze, bevorzugt Alkylammoniumsalze und Pyridiniumsalze gebildet werden. Zu den Basen, welche mit den Polysacchariden Salze bilden, zählen anorganische und organische Basen wie beispielsweise NaOH, KOH, LiOH, CaCO₃, Fe(OH)₃, NH₄OH, Tetraalkylammoniumhydroxide und ähnliche Verbindungen.

Die erfindungsgemäßen Verbindungen der allgemeinen Formel Ib können aus Heparin oder Heparansulfaten hergestellt werden, indem zuerst das Polysaccharid im wesentlichen vollständig desulfatiert und danach im wesentlichen vollständig N-acyliert wird. Der Begriff "im wesentlichen vollständig desulfatiert" steht für einen Desulfatierungsgrad von größer 90%, bevorzugt größer 95% und besonders bevorzugt größer 98%. Der Desulfatierungsgrad kann gemäß dem sogenannten Ninhydrintest bestimmt werden, der freie Aminogruppen nachweist. Die Desulfatierung erfolgt in dem Maße, daß mit DMMB (Dimethylmethylenblau) keine Farbreaktion mehr erhalten wird. Dieser Farbtest ist zum Nachweis sulfatierter Polysaccharide geeignet, seine Nachweisgrenze ist in der Fachliteratur jedoch nicht bekannt. Die Desulfatierung kann beispielsweise durch Erhitzen des Pyridiniumsalzes in einem Lösungsmittelgemisch durchgeführt werden. Insbesondere hat sich eine Mischung von DMSO, 1,4-Dioxan und Methanol bewährt.

Heparansulfate sowie Heparin wurden durch Totalhydrolyse desulfatiert und anschließend reacyliert. Danach wurde die Zahl der Sulfatgruppen pro Disaccharideinheit (S/D) mittels C¹³-NMR bestimmt. Die folgende Tabelle 1 gibt diese Resultate am Beispiel von Heparin und desulfatiertem, reacetyliertem Heparin (Ac-Heparin) wieder.

**Tab. 1 : Verteilung der funktionellen Gruppen pro Disaccharideinheit am Beispiel von Heparin und Ac-Heparin bestimmt mittels ¹³C-NMR-Messungen**

| | **2-S** | **6-S** | **3-S** | **NS** | **N-Ac** | **NH₂** | **S/D** |
|---|---|---|---|---|---|---|---|
| Heparin | 0,63 | 0,88 | 0,05 | 0,90 | 0,08 | 0,02 | 2,47 |
| Ac-Heparin | 0,03 | 0 | 0 | 0 | 1,00 | - | 0,03 |

| | | | | | | | |
|---|---|---|---|---|---|---|---|
| 2-S, 3-S, 6-S: Sulfatgruppen am Position 2, 3 bzw. 6 NS: Sulfatgruppen an den Aminogruppen N-Ac: Acetylgruppen an den Aminogruppen NH₂: freie Aminogruppen S/D: Sulfatgruppen pro Disaccharideinheit | | | | | | | |

Reproduzierbar ergab sich ein Sulfatgehalt von ca. 0,03 Sulfatgruppen/Disaccharideinheit (S/D) beim Ac-Heparin im Vergleich mit ca. 2,5 Sulfatgruppen/Disaccharideinheit beim Heparin
Die Verbindungen der allgemeinen Formeln Ia und Ib weisen einen Gehalt an Sulfatgruppen pro Disaccharideinheit von weniger als 0,2, bevorzugt weniger als 0,07, weiter bevorzugt weniger als 0,05 und insbesondere bevorzugt weniger als 0,03 Sulfatgruppen pro Disaccharideinheit auf.

Im wesentlichen vollständig N-acyliert bezieht sich auf einen N-Acylierungsgrad von größer 94%, bevorzugt größer 97% und besonders bevorzugt größer 98%. Die Acylierung verläuft derart vollständig, daß mit dem Ninhydrinnachweis auf freie Aminogruppen keine Farbreaktion mehr erhalten wird. Als Acylierungsmittel werden bevorzugt Carbonsäurechloride, -bromide oder -anhydride eingesetzt. Essigsäureanhydrid, Propionsäureanhydrid, Buttersäureanhydrid, Essigsäure-chlorid, Propionsäurechlorid oder Buttersäurechlorid eigenen sich beispielsweise zur Herstellung der erfindungsgemäßen Verbindungen. Insbesondere eignen sich Carbonsäureanhydride als Acylierungsmitttel.

Zudem offenbart die Erfindung Oligo- und/oder Polysacchariden für die hemokompatible Beschichtung von Oberflächen. Bevorzugt sind Polysaccharide innerhalb der oben genannten Molekulargewichtsgrenzen. Die verwendeten Oligo- und/oder Polysaccharide zeichnen sich dadurch aus, daß sie den Zuckerbaustein N-Acylglucosamin oder N-Acylgalactosamin in großer Menge enthalten. Dies bedeutet, dass 40 bis 60%, bevorzugt 45 - 55% und insbesondere bevorzugt 48 - 52% der Zuckerbausteine N-Acylglucosamin oder N-Acylgalactosamin sind und im wesentlichen die restlichen Zuckerbausteine jeweils einen Carboxylrest aufweisen. Die Oligo- und/oder Polysaccharide bestehen somit gewöhnlich zu über 95%, bevorzugt zu über 98%, aus nur zwei Zuckerbausteinen, wobei ein Zuckerbaustein einen Carboxylrest und der andere einen N-Acylrest trägt.

Ein Zuckerbaustein der Oligo- und/oder Polysaccharide ist N-Acylglucosamin bzw. N-Acylgalactosamin, bevorzugt N-Acetylglucosamin bzw. N-Acetylgalactosamin, und bei dem anderen handelt es sich um Uronsäuren, bevorzugt um Glucuronsäure und Iduronsäure.

Bevorzugt sind Oligo- und/oder Polysaccharide, welche im wesentlichen aus dem Zucker Glucosamin bzw. Galactosamin bestehen, wobei im wesentlichen die Hälfte der Zuckerbausteine eine N-Acylgruppe trägt, bevorzugt eine N-Acetylgruppe, und die andere Hälfte der Glucosaminbausteine eine über die Aminogruppe direkt oder über eine oder mehrere Methylenylgruppen gebundene Carboxylgruppe trägt. Bei diesen an die Aminogruppe gebundenen Carbonsäurereste handelt es sich bevorzugt um Carboxymethyl- oder Carboxyethylgruppen. Ferner sind Oligo- und/oder Polysaccharide bevorzugt, welche im wesentlichen zur Hälfte, d.h. zu 48 - 52%, bevorzugt zu 49 - 51% und insbesondere bevorzugt zu 49,5 - 50,5%, aus N-Acylglucosamin bzw. N-Acylgalactosamin, bevorzugt aus N-Acetylglucosamin oder N-Acetylgalactosamin und im wesentlichen zur anderen Hälfte aus einer Uronsäure, bevorzugt Glucuronsäure und Iduronsäure, bestehen. Besonders bevorzugt sind die Oligo- und/oder Polysaccharide, welche eine im wesentlichen alternierende Abfolge (d.h. abgesehen von der statistischen Fehlerrate bei der alternierenden Verknüpfung) der beiden Zuckerbausteine aufweisen. Die Rate der Fehlverknüpfungen sollte unter 1%, bevorzugt unter 0,1% liegen.

Überraschenderweise hat sich gezeigt, daß sich für die erfindungsgemäßen Verwendungen insbesondere im wesentlichen desulfatiertes und im wesentlichen N-acyliertes Heparin sowie partiell N-carboxyalkyliertes und N-acyliertes Chitosan als auch desulfatiertes und im wesentlichen N-acyliertes Dermatansulfat, Chondroitinsulfat und auch in der Kettenlänge reduzierte Hyaluronsäure besonders gut eignen. Insbesondere sind für die hemokompatible Beschichtung N-acetyliertes Heparin sowie partiell N-carboxymethyliertes und N-acetyliertes Chitosan geeignet.

Die durch "im wesentlichen" definierten Desulfatierungs- und Acylierungsgrade wurden bereits weiter oben definiert. Der Begriff "im wesentlichen" soll verdeutlichen, daß statistische Abweichungen zu berücksichtigen sind. Eine im wesentlichen alternierende Abfolge der Zuckerbausteine besagt, daß in der Regel keine zwei gleichen Zuckerbausteine aneinander gebunden sind, schließt aber eine derartige Fehlverknüpfung nicht vollkommen aus. Entsprechend bedeutet "im wesentlichen zur Hälfte" annähernd 50%, läßt aber geringe Schwankungen zu, da gerade bei biosynthetisch hergestellten Makromolekülen nie der Idealfall erreicht wird und immer gewisse Abweichungen berücksichtigt werden müssen, da Enzyme nicht perfekt arbeiten und bei der Katalyse mit einer gewissen Fehlerrate zu rechnen ist. Im Falle des natürlichen Heparins liegt hingegen eine streng alternierende Abfolge von N-Acetylglucosamin- und Uronsäureeinheiten vor.

Des weiteren wird ein Verfahren zur hemokompatiblen Beschichtung von Oberflächen offenbart, welche für den direkten Blutkontakt bestimmt sind. Bei diesem Verfahren wird eine natürliche und/oder künstliche Oberfläche bereitgestellt und die oben beschriebenen Oligo- und/oder Polysaccharide werden auf dieser Oberfläche immobilisiert.

Die Immobilisierung der Oligo- und/oder Polysaccharide auf diesen Oberflächen kann mittels hydrophober Wechselwirkungen, van der Waals Kräften, elektrostatischer Wechselwirkungen, Wasserstoffbrücken, ionischer Wechselwirkungen, Quervernetzung der Oligo- und/oder Polysaccharide und/oder durch kovalente Bindung an die Oberfläche bewirkt werden. Bevorzugt ist die kovalente Verknüpfung der Oligo- und/oder Polysaccharide mehr bevorzugt die kovalente Einzelpunktverknüpfung (side-on Bindung) und insbesondere bevorzugt die kovalente Endpunktverknüpfung (end-on Bindung).

Unter "im wesentlichen die restlichen Zuckerbausteine" ist zu verstehen, dass 93% der restlichen Zuckerbausteine, bevorzugt 96% und insbesondere bevorzugt 98% der restlichen 60% - 40% der Zuckerbausteine einen Carboxylrest tragen.

Somit sind Stents bevorzugt, welche als unterste Schicht eine hämokompatible Schicht aus den vorgenannten Heparin-Derivaten, Chitosan-Derivaten und/oder Oligo- bzw. Polypeptiden aufweisen. Auf dieser Schicht befindet sich Rapamycin als reine Wirkstoffschicht und/oder in eingebetteter Form in einer Matrix aus einer Trägersubstanz.

### Polysulfons als biostabile polymere Träger für Rapamycin

Es hat sich überraschend herausgestellt, dass die Beschichtung von Stents, die vorzugsweise in permanentem Blutkontakt stehen, mit Polysulfon, Polyethersulfon und/oder Polyphenylsulfon und deren Derivate ein äußerst gut geeigneter biokompatibler und hämikompatibler Träger für Rapamycin ist.

Ein bevorzugtes thermoplastisches Polysulfon wird aus Bisphenol A und 4,4'-Dichlorphenylsulfon über Polykondensationsreaktionen hergestellt (s. nachfolgende Formel (II)).

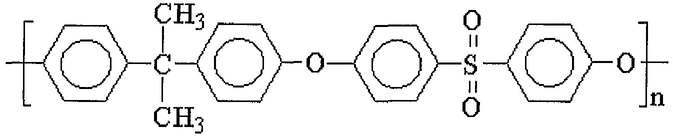

### Poly[oxy-1,4-phenylen-sulfonyl-1,4-phenylen-oxy-(4,4'-isopropylidendiphenylen)]

Die für die erfindungsgemäße Beschichtung einsetzbaren Polysulfone haben folgende allgemeine Struktur gemäß Formel (I): 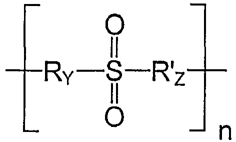 worin
n den Polymerisationsgrad bedeutet, der im Bereich von n = 10 bis n = 10.000, bevorzugt im Bereicht von n = 20 bis n = 3.000, weiter bevorzugt im Bereich von n = 40 bis n = 1.000, weiter bevorzugt im Bereich von n = 60 bis n = 500, weiter bevorzugt im Bereich von n = 80 bis n = 250 und insbesondere bevorzugt im Bereich von n = 100 bis n = 200 liegt.
Ferner ist bevorzugt, wenn n in einem solchen Bereich liegt, so dass sich ein Gewichtsmittel des Polymeren von 60.000 - 120.000 g/mol, bevorzugt von 70.000 bis 99.000 g/mol, weiter bevorzugt von 80.000 - 97.000, noch weiter bevorzugt von 84.000 - 95.000 und insbesondere bevorzugt von 86.000 - 93.000 g/mol ergibt. Zudem ist bevorzugt, wenn n in einem derartigen Bereich liegt, dass sich für das Zahlenmittel des Polymeren ein Bereicht von 20.000 - 70.000 g/mol, bevorzugt von 30.000 - 65.000 g/mol, weiter bevorzugt von 32.000 - 60.000, noch weiter bevorzugt von 35.000 - 59.000, besonders bevorzugt ein Bereich von 45.000 - 58.000 g/mol ergibt.

y und z sind ganze Zahlen im Bereich von 1 bis 10, und R und R' bedeuten unabhängig voneinander eine Alkylengruppe mit 1 bis 12 Kohlenstoffatomen, eine aromatische Gruppe mit 6 bis 20 Kohlenstoffatomen, eine heteroaromatische Gruppe mit 2 bis 10 Kohlenstoffatomen, eine Cycloalkylengruppe mit 3 bis 15 Kohlenstoffatomen, eine Alkylenarylengruppe mit 6 bis 20 Kohlenstoffatomen, eine Arylenalkylengruppe mit 6 bis 20 Kohlenstoffatomen, eine Alkylenoxygruppe mit 1 bis 12 Kohlenstoffatomen, eine Arylenoxygruppe mit 6 bis 20 Kohlenstoffatomen, eine Heteroarylenoxygruppe mit 6 bis 20 Kohlenstoffatomen, eine Cycloalkylenoxygruppe mit 3 bis 15 Kohlenstoffatomen, eine Alkylenarylenoxygruppe mit 6 bis 20 Kohlenstoffatomen oder eine Arylenalkylenoxygruppe mit 6 bis 20 Kohlenstoffatomen. Die vorgenannten Gruppen können weitere Substituenten tragen, insbesondere solche die weiter unten unter "substituierte" Polysulfone beschrieben sind.

Beispiele für die Gruppen R und R' sind -R¹-, -R²-, -R³-, -R⁴-, -R⁵-, -R⁶-, -R¹-R²-, -R³-R⁴-, -R⁵-R⁶-, -R¹-R²-R³-, -R⁴-R⁵-R⁶-, -R¹-R²-R³-R⁴-, -R¹-R²-R³-R⁴-R⁵- sowie -R¹-R²-R³-R⁴-R⁵-R⁶-;
wobei R¹, R², R³, R⁴, R⁵ und R⁶ unabhängig voneinander folgende Gruppen bedeuten:
-CH₂-, -C₂H₄-, -CH(OH)-, -CH(SH)-, -CH(NH₂)-, -CH(OCH₃)-, -C(OCH₃)₂-, -CH(SCH₃)-, -C(SCH₃)₂-, -CH(NH(CH₃))-, -C(N(CH₃)₂)-, -CH(OC₂H₅)-, -C(OC₂H₅)₂-, -CHF-, -CHCl-, -CHBr-, -CF₂-, -CCl₂-, -CBr₂-, -CH(COOH)-, -CH(COOCH₃)-, -CH(COOC₂H₅)-, -CH(COCH₃)-, -CH(COC₂H₅)-, -CH(CH₃)-, -C(CH₃)₂-, -CH(C₂H₅)-, -C(C₂H₅)₂-, -CH(CONH₂)-, -CH(CONH(CH₃))-, -CH(CON(CH₃)₂)-,
-C₃H₆-, -C₄H₈-, -C₅H₉-, -C₆H₁₀-, cyclo-C₃H₄-, cyclo-C₃H₄-, cyclo-C₄H₆-, cyclo-C₅H₈-, -OCH₂-, -OC₂H₄-, -OC₃H₆-, -OC₄H₈-, -OC₅H₉- -OC₆H₁₀-, -CH₂O-, -C₂H₄O-, -C₃H₆O-, -C₄H₈O-, -C₅H₉O-, -C₆H₁₀O-, -NHCH₂-, -NHC₂H₄-, -NHC₃H₆-, -NHC₄H₈-, -NHC₅H₉-, -NHC₆H₁₀-, -CH₂NH-, -C₂H₄NH-, -C₃H₆NH-, -C₄H₈NH-, -C₅H₉NH-, -C₆H₁₀NH-, -SCH₂-, -SC₂H₄-, -SC₃H₆-, -SC₄H₈-, -SC₅H₉-, -SC₆H₁₀-, -CH₂S-, -C₂H₄S-, -C₃H₆S-, -C₄H₈S-, -C₅H₉S-, -C₆H₁₀S-,
-C₆H₄-, -C₆H₃(CH₃)-, -C₆H₃(C₂H₅)-, -C₆H₃(OH)-, -C₆H₃(NH₂)-, -C₆H₃(Cl)-, -C₆H₃(F)-, -C₆H₃(Br)- , -C₆H₃(OCH₃)-, -C₆H₃(SCH₃)-, -C₆H₃(COCH₃)-, -C₆H₃(COC₂H₅)-, -C₆H₃(COOH)-, -C₆H₃(COOCH₃)-, -C₆H₃(COOC₂H₅)- -C₆H₃(NH(CH₃))-, -C₆H₃(N(CH₃)₂)-, -C₆H₃(CONH₂)-, -C₆H₃(CONH(CH₃))-, -C₆H₃(CON(CH₃)₂)-,
-OC₆H₄-, -OC₆H₃(CH₃)-, -OC₆H₃(C₂H₅)-, -OC₆H₃(OH)-, -OC₆H₃(NH₂)-, -OC₆H₃(Cl)-, -OC₆H₃(F)-, -OC₆H₃(Br)-, -OC₆H₃(OCH₃)-, -OC₆H₃(SCH₃)-, -OC₆H₃(COCH₃)-, -OC₆H₃(COC₂H₅)-, -OC₆H₃(COOH)-, -OC₆H₃(COOCH₃)-, -OC₆H₃(COOC₂H₅)-, -OC₆H₃(NH(CH₃))-, -OC₆H₃(N(CH₃)₂)-, -OC₆H₃(CONH₂)-, -OC₆H₃(CONH(CH₃))-, -OC₆H₃(CON(CH₃)₂)-,
-C₆H₄O-, -C₆H₃(CH₃)O-, -C₆H₃(C₂H₅)O-, -C₆H₃(OH)O-, -C₆H₃(NH₂)O-, -C₆H₃(Cl)O-, -C₆H₃(F)O-, -C₆H₃(Br)O-, -C₆H₃(OCH₃)O-, -C₆H₃(SCH₃)O-, -C₆H₃(COCH₃)O-, -C₆H₃(COC₂H₅)O-, -C₆H₃(COOH)O-, -C₆H₃(COOCH₃)O-, -C₆H₃(COOC₂H₅)O-, -C₆H₃(NH(CH₃))O-, -C₆H₃(N(CH₃)₂)O-, -C₆H₃(CONH₂)O-, -C₆H₃(CONH(CH₃))O-, -C₆H₃(CON(CH₃)₂)O-,
-SC₆H₄-, -SC₆H₃(CH₃)-, -SC₆H₃(C₂H₅)-, -SC₆H₃(OH)-, -SC₆H₃(NH₂)-, -SC₆H₃(Cl)-, -SC₆H₃(F)-, -SC₆H₃(Br)-, -SC₆H₃(OCH₃)-, -SC₆H₃(SCH₃)-, -SC₆H₃(COCH₃)-, -SC₆H₃(COC₂H₅)- -SC₆H₃(COOH)-, -SC₆H₃(COOCH₃)-, -SC₆H₃(COOC₂H₅)-, -SC₆H₃(NH(CH₃))-, -SC₆H₃(N(CH₃)₂)-, -SC₆H₃(CONH₂)-, -SC₆H₃(CONH(CH₃))- -SC₆H₃(CON(CH₃)₂)-,
-C₆H₄S-, -C₆H₃(CH₃)S-, -C₆H₃(C₂H₅)S-, -C₆H₃(OH)S-, -C₆H₃(NH₂)S-, -C₆H₃(Cl)S-, -C₆H₃(F)S-, -C₆H₃(Br)S-, -C₆H₃(OCH₃)S-, -C₆H₃(SCH₃)S-, -C₆H₃(COCH₃)S-, -C₆H₃(COC₂H₅)S-, -C₆H₃(COOH)S-, -C₆H₃(COOCH₃)S-, -C₆H₃(COOC₂H₅)S- -C₆H₃(NH(CH₃))S-, -C₆H₃(N(CH₃)₂)S-, -C₆H₃(CONH₂)S-, -C₆H₃(CONH(CH₃))S-, -C₆H₃(CON(CH₃)₂)S-,
-NH-C₆H₄-, -NH-C₆H₃(CH₃)-, -NH-C₆H₃(C₂H₅)-, -NH-C₆H₃(OH)-, -NH-C₆H₃(NH₂)-, -NH-C₆H₃(Cl)-, -NH-C₆H₃(F)-, -NH-C₆H₃(Br)-, -NH-C₆H₃(OCH₃)-, -NH-C₆H₃(SCH₃)-, -NH-C₆H₃(COCH₃)-, -NH-C₆H₃(COC₂H₅)-, -NH-C₆H₃(COOH)-, -NH-C₆H₃(COOCH₃)-, -NH-C₆H₃(COOC₂H₅)- -NH-C₆H₃(NH(CH₃))-, -NH-C₆H₃(N(CH₃)₂)-, -NH-C₆H₃(CONH₂)-, -NH-C₆H₃(CONH(CH₃))-, -NH-C₆H₃(CON(CH₃)₂)-,
-C₆H₄-NH-, -C₆H₃(CH₃)-NH-, -C₆H₃(C₂H₅)-NH-, -C₆H₃(OH)-NH-, -C₆H₃(NH₂)-NH-, -C₆H₃(Cl)-NH-, -C₆H₃(F)-NH-, -C₆H₃(Br)-NH-, -C₆H₃(OCH₃)-NH-, -C₆H₃(SCH₃)-NH-, -C₆H₃(COCH₃)-NH-, -C₆H₃(COC₂H₅)-NH-, -C₆H₃(COOH)-NH-, -C₆H₃(COOCH₃)-NH-, -C₆H₃(COOC₂H₅)-NH- -C₆H₃(NH(CH₃))-NH-, -C₆H₃(N(CH₃)₂)-NH-, -C₆H₃(CONH₂)-NH-, -C₆H₃(CONH(CH₃))-NH-, -C₆H₃(CON(CH₃)₂)-NH-.

Besonders bevorzugt werden Polysulfone sowie ihre Mischungen, worin die Gruppen -R¹-, -R²-, -R³-, -R¹-R²-, -R¹-R²-R³- unabhängig voneinander folgende Gruppen bedeuten: -C₆H₄O-, -C(CH₃)₂-, -C₆H₄-, -C₆H₄SO₂-, -SO₂C₆H₄-, -OC₆H₄-, -C₆H₄O-C(CH₃)₂-C₆H₄-.

R und R' können ferner unabhängig voneinander bevorzugt einen der an die Sulfongruppe in den Formel (II) bis (XV) gebundenen Rest darstellen.

Erfindungsgemäß wird das Polysulfon bzw. werden die Polysulfone für die biostabile Schicht oder die biostabilen Schichten aus der Gruppe ausgewählt umfassend: Polyethersulfon, substituiertes Polyethersulfon, Polyphenylsulfon, substituiertes Polyphenylsulfon, Polysulfonblockcopolymere, perfluorierte Polysulfonblockcopolymere, semifluorierte Polysulfonblockcopolymere, substituierte Polysulfonblockcopolymere und/oder Mischungen der vorgenannten Polymere.

Unter dem Begriff "substituierte" Polysulfone werden Polysulfone verstanden, welche funktionelle Gruppen aufweisen. Insbesondere können die Methyleneinheiten einen oder zwei Substituenten und die Phenyleneinheiten einen, zwei, drei oder vier Substituenten aufweisen. Beispiele für diese Substituenten (auch bezeichnet als X, X', X", X"') sind: -OH, -OCH₃, -OC₂H₅, -SH, -SCH₃, -SC₂H₅, -NO₂, -F, -Cl, -Br, -I, -N₃, -CN, -OCN, -NCO, -SCN, -NCS, -CHO, -COCH₃, -COC₂H₅, -COOH, -COCN, -COOCH₃, -COOC₂H₅, -CONH₂, -CONHCH₃, -CONHC₂H₅, -CON(CH₃)₂, -CON(C₂H₅)₂, -NH₂, -NHCH₃, -NHC₂H₅, -N(CH₃)₂, -N(C₂H₅)₂, -SOCH₃, -SOC₂H₅, -SO₂CH₃, -SO₂C₂H₅, -SO₃H, -SO₃CH₃, -SO₃C₂H₅, -OCF₃, -O-COOCH₃, -O-COOC₂H₅, -NH-CO-NH₂, -NH-CS-NH₂, -NH-C(=NH)-NH₂, -O-CO-NH₂, -NH-CO-OCH₃, -NH-CO-OC₂H₅, -CH₂F -CHF₂, -CF₃, -CH₂Cl -CHCl₂, -CCl₃, -CH₂Br -CHBr₂, -CBr₃, -CH₂I -CHI₂, -Cl₃, -CH₃, -C₂H₅, -C₃H₇, -CH(CH₃)₂, -C₄H₉, -CH₂-CH(CH₃)₂, -CH₂-COOH, -CH(CH₃)-C₂H₅, -C(CH₃)₃, -H. Weitere bevorzugte Substituenten oder funktionelle Gruppen sind -CH₂-X und -C₂H₄-X.

Die nachfolgenden allgemeinen Strukturformeln repräsentieren bevorzugte Wiederholungseinheiten für Polysulfone. Bevorzugt bestehen die Polymere nur aus diesen Wiederholungseinheiten. Es ist aber auch möglich, dass in einem Polymeren neben den gezeigten Wiederholungseinheiten noch andere Wiederholungseinheiten oder Blöcke vorhanden sind. Bevorzugt sind: 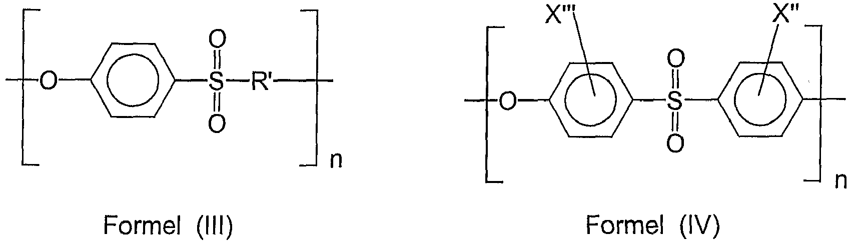 X, X', n und R' haben unabhängig voneinander die oben erwähnte Bedeutung. 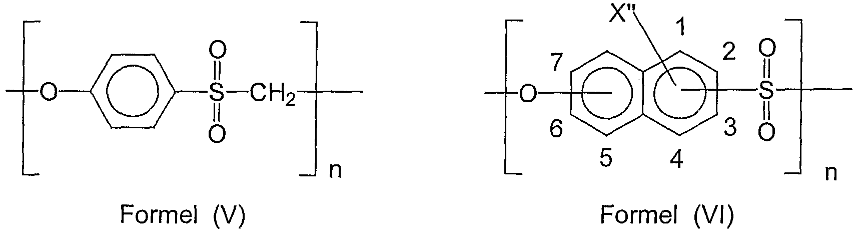 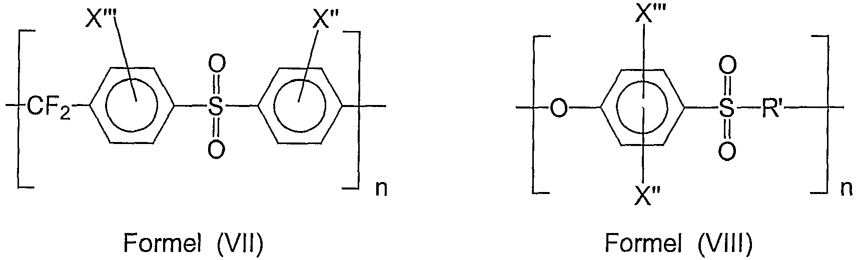 X, X', n und R' haben unabhängig voneinander die oben erwähnte Bedeutung.

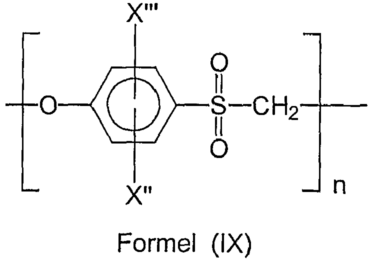

Ferner sind Polysulfone der folgenden allgemeinen Formel (X) bevorzugt 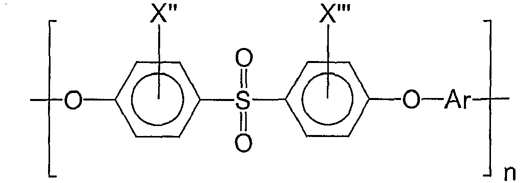 worin Ar bedeutet 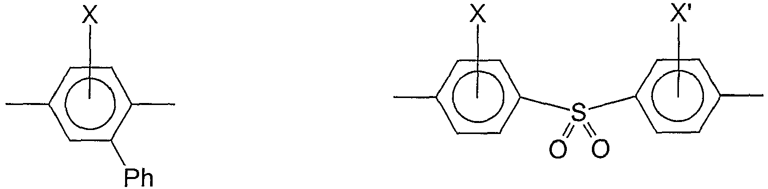 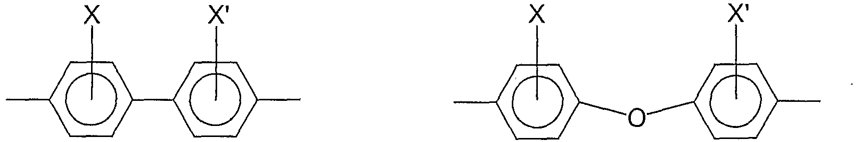 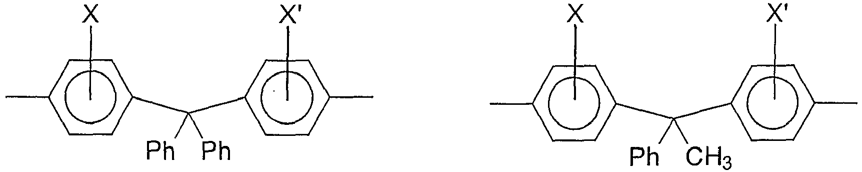 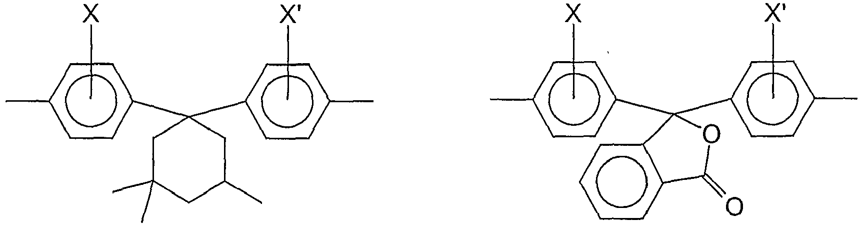 X, X' und n haben unabhängig voneinander die oben erwähnte Bedeutung.

Folgende Wiederholungseinheiten sind des weiteren bevorzugt 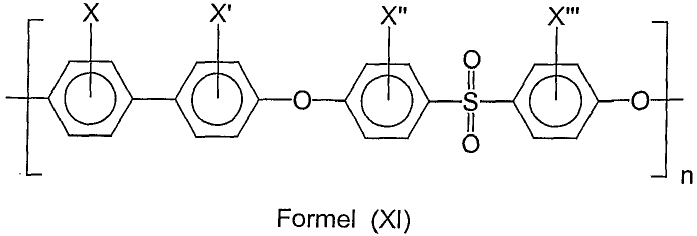 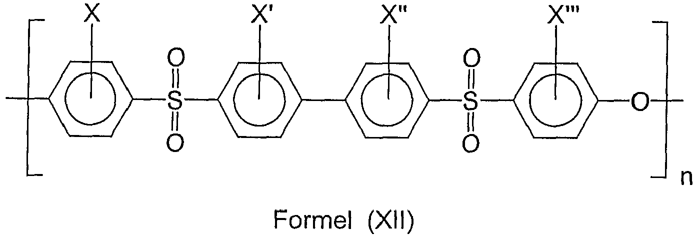 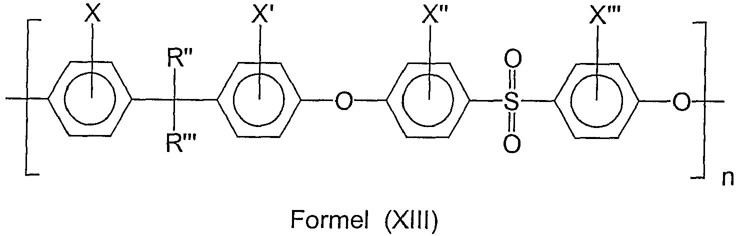 X, X', X", X'" und n haben unabhängig voneinander die oben erwähnte Bedeutung. R" und R'" können unabhängig voneinander für einen Substituenten stehen, wie er für X oder X' definiert ist oder können unabhängig voneinander einen Rest -R¹-H oder -R²-H bedeuten.

Eine weitere bevorzugte Wiederholungseinheit weist einen cyclischen Substituenten zwischen zwei aromatischen Ringen auf, wie beispielsweise Formel (XIV) oder (XV): 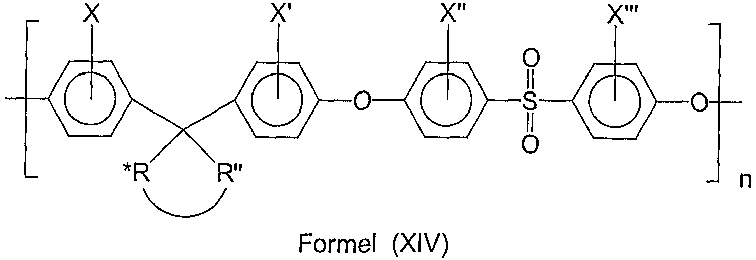 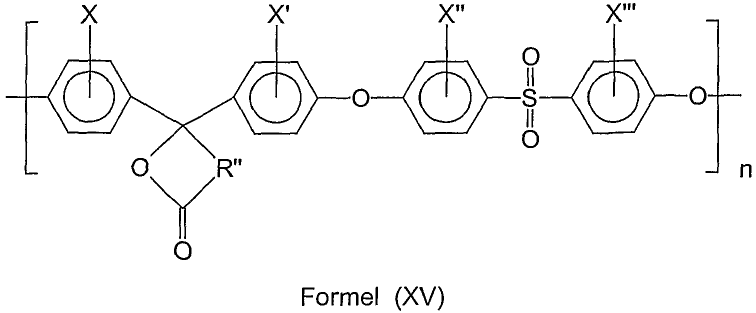 R" bedeutet bevorzugt -CH₂-, -OCH₂-, -CH₂O-, -O-, -C₂H₄-, -C₃H₆-, -CH(OH)-. Die Gruppe -*R-R"- bedeutet bevorzugt einen cyclischen Ester, Amid, Carbonat, Harnstoff oder Urethan wie beispielsweise: -O-CO-O-, -O-CO-O-CH₂-, -O-CO-O-C₂H₄-, -CH₂-O-CO-O-CH₂-, -C₂H₄-, -C₃H₆-, -C₄H₃-, -C₅H₁₀-, -C₆H₁₂- -O-CO-NH-, -NH-CO-NH-, -O-CO-NH-CH₂-, -O-CO-NH-C₂H₄-, -NH-CO-NH-CH₂-, -NH-CO-NH-C₂H₄-, -NH-CO-O-CH₂-, -NH-CO-O-C₂H₄-, -CH₂-O-CO-NH-CH₂-, -C₂H₄-SO₂-, -C₃H₆-SO₂-, -C₄H₈-SO₂-, -C₂H₄-SO₂-CH₂-, -C₂H₄-SO₂-C₂H₄-, -C₂H₄-O-, -C₃H₆-O-, -C₄H₈-O-, -C₂H₄-O-CH₂-, -C₂H₄-O-C₂H₄-, -C₂H₄-CO-, -C₃H₆-CO-, -C₄H₈-CO-, -C₂H₄-CO-CH₂-, -C₂H₄-CO-C₂H₄-, -O-CO-CH₂-, -O-CO-C₂H₄-, -O-CO-C₂H₂-, -CH₂-O-CO-CH₂-, oder cyclische Ester enthaltend einen aromatischen Ring.

Im folgenden werden polymeranaloge Reaktionen beschrieben, welche dem Fachmann bekannt sind und zur Modifizierung der Polysulfone dienen 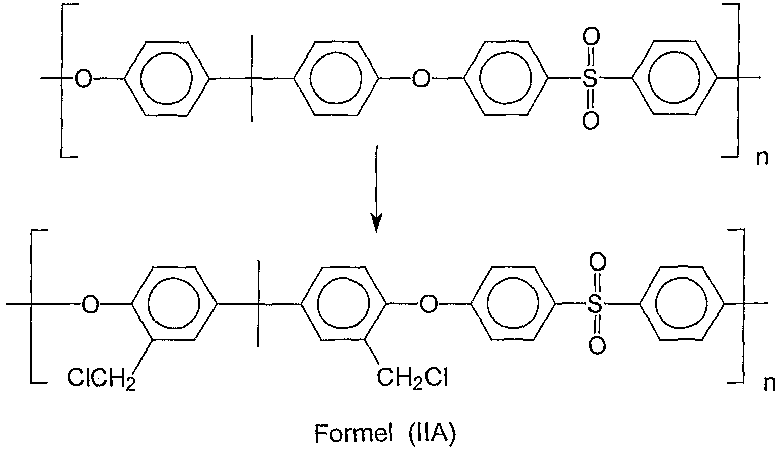 Chlormethylengruppen als Reste X und X' lassen sich unter Verwendung von Formaldehyd, CISMe₃ und einem Katalysator wie SnCl₄ einführen, welche danach weiter substituiert werden können. Durch diese Reaktion lassen sich beispielsweise Hydroxygruppen, Aminogruppen, Carboxylatgruppen, Ether oder Alkylreste durch eine nucleophile Substitution einführen, welche über eine Methylengruppe an den Aromaten gebunden sind. Eine Umsetzung mit Alkoholaten wie beispielsweise einem Phenolat, Benzylat, Methanolat, Ethanolat, Propanolat oder Isopropanolat führt zu einem Polymer, bei dem an mehr als 75% der Chlormethylengruppen eine Substitution stattgefunden hat. Man erhält folgendes Polysulfon mit lipophilen Seitengruppen: 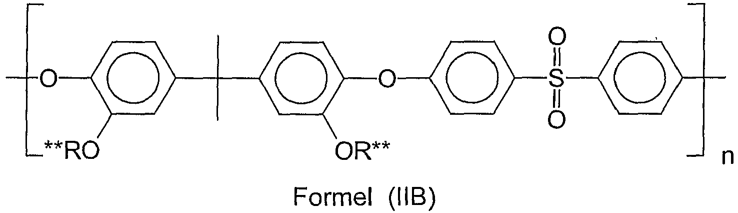 worin
R** beispielsweise einen Alkylrest oder Arylrest bedeutet.

Die Reste X" und X'" lassen sich, soweit in den Monomeren noch nicht vorhanden, am Polymeren durch folgende Reaktion einführen:

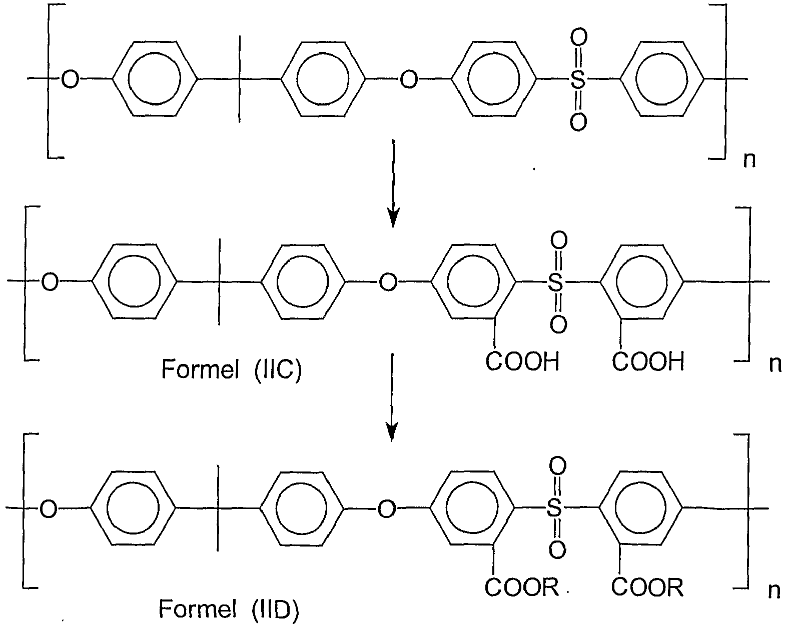

Neben einer Estergruppe können diverse andere Substituenten eingeführt werden, indem zuerst mit einer starken Base, z.B. n-BuLi oder tert-BuLi eine einfache oder zweifache Deprotonierung durchgeführt wird und danach ein Elektrophil zugesetzt wird. Im obigen Beispielsfall wurde zur Einführung der Estergruppe Kohlendioxid zugesetzt und die erhaltene Carbonsäuregruppe in einem weiteren Schritt verestert.

Eine erfindungsgemäße Kombination aus einem Polysulfon mit lipophilen Resten und einem Polysulfon mit lipophoben Resten wird beispielsweise durch die Verwendung von Polysulfon gemäß Formel (IIB) zusammen mit Polysulfon gemäß Formel (IIC) erreicht. Die Mengenverhältnisse von beiden Polysulfonen zueinander können von 98% : 2% bis 2% zu 98% liegen. Bevorzugte Bereiche sind 10% zu 90%, 15% zu 85%, 22% zu 78% und 27% zu 73%, 36% zu 64%, 43% zu 57% und 50% zu 50%. Diese Prozentangaben gelten für beliebige Kombinationen von hydrophilen und hydrophoben Polysulfonen und sind nicht auf die vorgenannte Mischung beschränkt.

Ein Beispiel für ein Polysulfon mit hydrophilen und hydrophoben Resten in einem Molekül erhält man beispielsweise, indem das Polysulfon gemäß Formel (IIC) nur unvollständig verestert wird und somit hydrophile Carboxylatgruppen und hydrophobe Estergruppen in einem Molekül vorliegen. Das Molverhältnis (Anzahl) an Carboxylatgruppen zu Estergruppen kann 5% zu 95% bis 95% zu 5% betragen. Diese Prozentangaben gelten für beliebige Kombinationen von hydrophilen und hydrophoben Gruppen und sind nicht auf die vorgenannten beschränkt.

Es wird vermutet, dass durch diese erfindungsgemäße Kombination von hydrophilen Gruppen bzw. Polymeren mit hydrophoben Gruppen bzw. Polymeren sich amorphe Polymerschichten auf dem Medizinprodukt ausbilden. Es ist sehr wichtig, dass die Polymerschichten aus Polysulfon nicht kristallin oder überwiegend kristallin sein dürfen, da Kristallinität zu starren Schichten führt, welche einreißen und sich ablösen. Flexible als Barriereschicht dienende Polysulfonbeschichtungen lassen sich nur mit amorphen oder überwiegend amorphen Polysulfonschichten erreichen.

Natürlich ist es auch möglich, bereits entsprechend substituierte Monomere einzusetzen, um nach erfolgter Polymerisation das gewünschte Substitutionsmuster zu erhalten. Die entsprechenden Polymere ergeben sich dann in bekannter Weise gemäß folgenden Reaktionsschema: 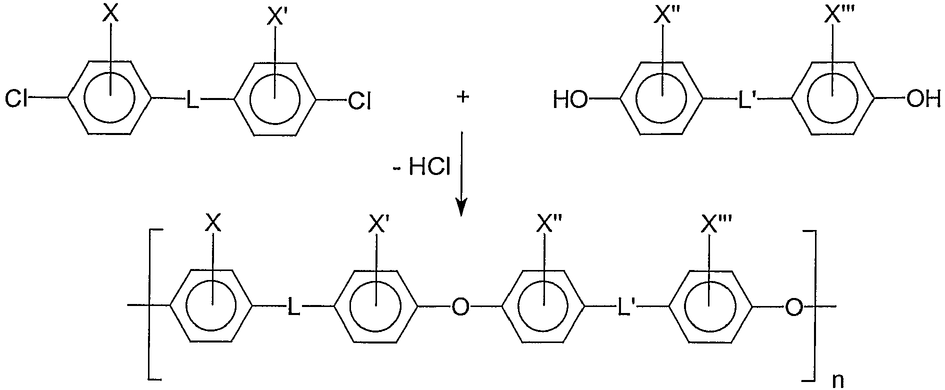 worin
L und L' unabhängig voneinander beispielsweise für folgende Gruppen stehen: -SO₂-, -C(CH₃)₂-, -C(Ph)₂- oder -O-. L und L' können somit die Bedeutungen der entsprechenden Gruppen in den Formeln (I) bis (XV) haben. Dem Fachmann sind derartige nucleophile Substitutionsreaktionen bekannt, welche durch das obige Schema beispielhaft verdeutlicht werden.

Wie bereits erwähnt, ist insbesondere bevorzugt, wenn die Polymere hydrophile und hydrophobe Eigenschaften aufweisen, zum einen innerhalb eines Polymers und zum anderen durch Verwendung mindestens eines hydrophilen Polymers in Kombination mit mindestens einem hydrophoben Polymeren. Somit ist bevorzugt, wenn es sich beispielsweise bei X und X' um hydrophile Substituenten und bei X" und X'" um hydrophobe Substituenten handelt oder umgekehrt.

Als hydrophile Substituenten kommen in Frage: -OH, -CHO, -COOH, -COO⁻, -CONH₂, -NH₂, -N⁺(CH₃)₄, -NHCH₃, -SO₃H, -SO₃⁻, -NH-CO-NH₂, -NH-CS-NH₂, -NH-C(=NH)-NH₂, -O-CO-NH₂ und insbesondere protonierte Aminogruppen.

Als hydrophobe Substituenten kommen in Frage: -H, -OCH₃, -OC₂H₅, -SCH₃, -SC₂H₅, -NO₂, -F, -Cl, -Br, -I, -N₃, -CN, -OCN, -NCO, -SCN, -NCS, -COCH₃, -COC₂H₅, -COCN, -COOCH₃, -COOC₂H₅, -CONHC₂H₅, -CON(CH₃)₂, -CON(C₂H₅)₂, -NHC₂H₅, -N(CH₃)₂, -N(C₂H₅)₂, -SOCH₃, -SOC₂H₅, -SO₂CH₃, -SO₂C₂H₅, -SO₃CH₃, -SO₃C₂H₅, -OCF₃, -O-COOCH₃, -O-COOC₂H₅, -NH-CO-OCH₃, -NH-CO-OC₂H₅, -CH₂F -CHF₂, -CF₃, -CH₂Cl -CHCl₂, -CCl₃, -CH₂Br -CHBr₂, -CBr₃, -CH₂I -CHI₂, -Cl₃, -CH₃, -C₂H₅, -C₃H₇, -CH(CH₃)₂, -C₄H₉, -CH₂-CH(CH₃)₂, -CH₂-COOH, -CH(CH₃)-C₂H₅, -C(CH₃)₃.

Des weiteren sind cyclische Polysulfone bevorzugt, welche beispielsweise eine Struktur wie in Formel (XVI) gezeigt aufweisen:

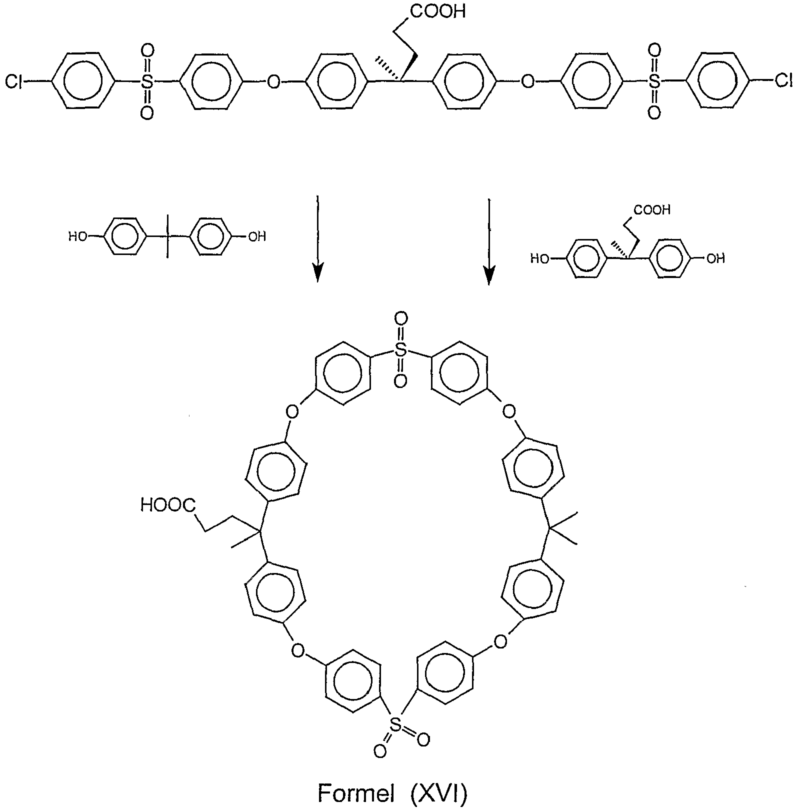

Die Carboxyethylengruppe ist für die obige Beispielsreaktion nicht essentiell. Anstelle der Carboxyethylen- und der Methylsubstituenten können beliebige andere Substituten oder auch Wasserstoff vorhanden sein.

### Öle und Fette als Trägersubstanzen

Neben den oben erwähnten biostabilen und bioabbaubaren Polymeren als Trägermatrix für Rapamycin und andere Wirkstoffe können auch physiologisch verträgliche Öle, Fette, Lipide, Lipoide und Wachse eingesetzt werden.

Solche Öle, Fette und Wachse, die als Trägersubstanzen für Rapamycin oder andere Wirkstoffe oder als wirkstofffreie Schichten, insbesondere Deckschichten eingesetzt werden können, eignen sich Stoffe, die sich durch folgende allgemeine Formeln darstellen lassen: 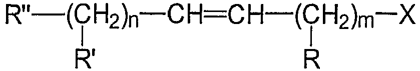 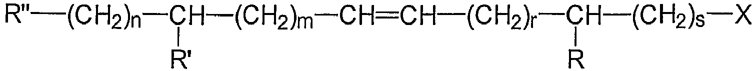 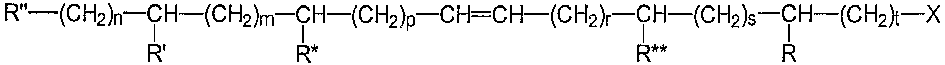 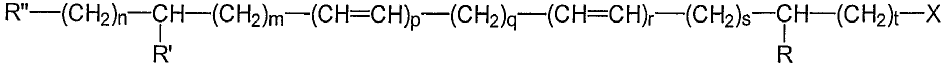 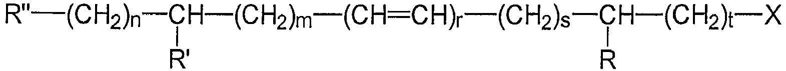 worin
R, R', R", R* und R** unabhängig voneinander für Alkyl-, Alenyl-, Alkinyl-, Heteroalkyl-, Cycloalkyl-, Heterocyclylreste mit 1 bis 20 Kohlenstoffatomen, Aryl-, Arylalkyl-, Alyklaryl-, Heteroarylreste mit 3 bis 20 Kohlenstoffatomen oder für funktionelle Gruppen stehen und bevorzugt folgende Reste bedeuten: -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -SCH₃, -SC₂H₅, -NO₂, -F, -Cl, -Br, -I, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CON(CH₃)₂, -CON(C₂H₅)₂, -CON(C₃H₇)₂, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO₂CH₃, -SO₂C₂H₅, -SO₂C₃H₇, -SO₃H, -SO₃CH₃, -SO₃C₂H₅, -SO₃C₃H₇, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-N(CH₃)₂, -NH-CO-N(C₂H₅)₂, -O-CO-NH₂, -O-CO-NHCH₃, -O-CO-NHC₂H₅, -O-CO-NHC₃H₇, -O-CO-N(CH₃)₂, -O-CO-N(C₂H₅)₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂l, -CH₃, -C₂H₅, -C₃H₇, -cyclo-C₃H₅, -CH(CH₃)₂, -C(CH₃)₃, -C₄H₉, -CH₂-CH(CH₃)₂, -CH(CH₃)-C₂H₅, -Ph, -CH₂-Ph, -CPh₃, -CH=CH₂, -CH₂-CH=CH₂, -C(CH₃)=CH₂, -CH=CH-CH₃, -C₂H₄-CH=CH₂, -CH=C(CH₃)₂, -C≡CH, -C≡C-CH₃, -CH₂-C≡CH;

X für eine Estergruppe oder Amidgruppe und insbesondere für -O-alkyl, -O-CO-alykl, -O-CO-O-alkyl, -O-CO-NH-alkyl, -O-CO-N-dialkyl, -CO-NH-alkyl, -CO-N-dialkyl, -CO-O-alkyl, -CO-OH, -OH;

m, n, p, q, r, s und t unabhängig voneinander ganze Zahlen von 0 bis 20, bevorzugt von 0 bis 10 bedeuten.

Die Bezeichnung "alkyl" beispielsweise bei -CO-O-alkyl bedeutet vorzugsweise eine der für die vorgenannten Reste R, R' usw. genannten Alkylreste, z.B. -CH₂-Ph. Die Verbindungen der vorgenannten allgemeinen Formeln können auch in Form ihrer Salze, als Racemate oder Diastereomerengemische, als reine Enantiomeren oder Diastereomeren sowie als Gemische oder Oligomere oder Copolymere oder Blockcopolymere vorliegen. Ferner können die vorgenannten Verbindungen im Gemisch mit anderen Substanzen wie den biostabilen und biodegradierbaren Polymeren und insbesondere im Gemisch mit den hierin genannten Ölen und/oder Fettsäuren eingesetzt werden. Bevorzugt sind derartige Gemische und Einzelsubstanzen, welche zur Polymerisation, insbesondere zur Autopolymerisation geeignet sind.

Die zur Polymerisation insbesondere Autopolymerisation geeigneten Substanzen umfassen unter anderem Öle, Fette, Lipide, Fettsäuren sowie Fettsäureester, welche im folgenden näher beschrieben werden.
Bei den Lipiden handelt es sich vorzugsweise um einfach oder mehrfach ungesättigte Fettsäuren und/oder Mischungen aus diesen ungesättigten Fettsäuren in Form ihrer Triglyceride und/oder in nicht glyceringebundener, freier Form.

Vorzugsweise werden die ungesättigten Fettsäuren aus der Gruppe ausgewählt, welche Ölsäure, Eicosapentaensäure, Timnodonsäure, Docosahexaensäure, Arachidonsäure, Linolsäure, α-Linolensäure, γ-Linolensäure sowie Mischungen der vorgenannten Fettsäuren umfasst. Diese Mischungen umfassen insbesondere Mischungen der reinen ungesättigten Verbindungen.

Als Öle werden bevorzugt Leinöl, Hanföl, Maiskeimöl, Walnussöl, Rapsöl, Sojaöl, Sonnenblumenöl, Mohnöl, Safloröl (Färberdistelöl), Weizenkeimöl, Distelöl, Traubenkernöl, Nachtkerzenöl, Borretschöl, Schwarzkümmelöl, Algenöl, Fischöl, Lebertran und/oder Mischungen der vorgenannten Öle eingesetzt. Insbesondere geeignet sind Mischungen der reinen ungesättigten Verbindungen.

Fischöl und Lebertran enthalten hauptsächlich Eicosapentaensäure (EPA C20:5) und Docosahexaensäure (DHA C22:6) neben wenig alpha-Linolensäure (ALA C18:3). Bei allen drei Fettsäuren handelt es sich um Omega-3-Fettsäuren, die im Organismus als bedeutende biochemische Bausubstanz für zahlreiche Zellstrukturen benötigt werden (DHA und EPA), zum Beispiel sind sie wie bereits erwähnt, wesentlich für den Aufbau und Bestand der Zellmembran (Sphingolipide, Ceramide, Ganglioside) verantwortlich.
Omega-3-Fettsäuren finden sich nicht nur in Fischöl, sondern auch in Pflanzenölen. Weitere ungesättigte Fettsäuren, wie die Omega-6-Fettsäuren, sind in Ölen pflanzlicher Herkunft vorhanden, die hier teils einen höheren Anteil als in tierischen Fetten ausmachen. Daher werden verschiedene Pflanzenöle wie Leinöl, Walnussöl, Flachsöl, Nachtkerzenöl mit entsprechend hohem Gehalt an essentiellen Fettsäuren als besonders hochwertige und wertvolle Speiseöle empfohlen. Vor allem Leinöl stellt ein wertvoller Lieferant von Omega-3- und Omega-6-Fettsäuren dar und ist als hochwertiges Speiseöl seit Jahrzehnten bekannt.

Als an der Polymerisationsreaktion teilnehmende Substanzen sind die Omega-3 als auch die Omega-6-Fettsäuren bevorzugt sowie sämtliche Substanzen, welche mindestens einen Omega-3 und/oder Omega-6-Fettsäurerest tragen. Derartige Substanzen zeigen auch eine gute Befähigung zur Autopolymerisation.
Die Fähigkeit auszuhärten, d.h. die Fähigkeit zur Autopolymerisation, ist in der Zusammensetzung der auch als trocknende Öle bezeichneten Öle begründet und geht auf den hohen Gehalt an essentiellen Fettsäuren, genauer auf die Doppelbindungen der ungesättigten Fettsäuren zurück. An der Luft werden mit Hilfe des Sauerstoffs an den Doppelbindungsstellen der Fettsäuremoleküle Radikale gebildet, die die radikalische Polymerisation initiieren und propagieren, so dass die Fettsäuren unter Verlust der Doppelbindungen untereinander vernetzen. Durch die Aufhebung der Doppelbindung im Fettmolekül steigt der Schmelzpunkt und die Vernetzung der Fettsäuremoleküle bewirkt eine zusätzliche Härtung. Es entsteht ein hochmolekulares Harz, dass als flexibler Polymerfilm die medizinische Oberfläche gleichmäßig bedeckt.

Die Autopolymerisation wird auch als Selbstpolymerisation bezeichnet und kann beispielsweise durch Sauerstoff, insbesondere Luftsauerstoff initiiert werden. Diese Autopolymerisation kann auch unter Lichtausschluss durchgeführt werden. Eine weitere Möglichkeit besteht in der Initiierung der Autopolymerisation durch elektromagnetische Strahlung, insbesondere Licht. Eine weitere aber weniger bevorzugt Variante stellt die Autopolymerisation ausgelöst durch chemische Zerfallsreaktionen dar, insbesondere Zerfallsreaktionen der zu polymerisierenden Stoffe.

Je mehr Mehrfachbindungen der Fettsäurerest aufweist, desto ist der Vernetzungsgrad. Somit ist die Menge an Substanzen, welche aktiv an der Polymerisationsreaktion teilnehmen um so geringer, je höher die Dichte an Mehrfachbindungen in einem Alkylrest (Fettsäurerest) als auch in einem Molekül ist.

Der Gehalt an aktiv an der Polymerisationsreaktion teilnehmenden Substanzen in Bezug auf die Gesamtmenge aller auf die Oberfläche des Medizinproduktes aufgetragenen Substanzen beträgt mindestens 25 Gew.-%, bevorzugt 35 Gew.-%, weiter bevorzugt 45 Gew.-% und insbesondere bevorzugt 55 Gew.-%.

Die folgende Tabelle 1 zeigt eine Aufstellung der Fettsäurebestandteile in verschiedenen Ölen, welche bei der vorliegenden Erfindung bevorzugt eingesetzt werden.

**Tabelle 1**

| **Ölsorte** | **Ölsäure** | **Linolsäure** | **Linolensäure** | Eicosapentaensäure | Docosaexaensäure |
|---|---|---|---|---|---|
| | (C 18:1) | (C 18:2) | (C 18:3) | (C 20:5) | (C 22:6) |
| | Omega-9 | Omega-6 | Omega-3 | Omega-3 | Omega-3 |
| Olivenöl | 70 | 10 | 0 | 0 | 0 |
| Maisöl | 30 | 60 | 1 | 0 | 0 |
| Leinöl | 20 | 20 | 60 | 0 | 0 |
| Lebertran | 25 | 2 | 1 | 12 | 8 |
| Fischöl | 15 | 2 | 1 | 18 | 12 |

Die bei der erfindungsgemäßen Beschichtung eingesetzten Öle bzw. die Mischungen der Öle enthalten einen Anteil von mindestens 40 Gew.-% an ungesättigten Fettsäuren, bevorzugt einen Anteil von 50 Gew.-%, weiter bevorzugt einen Anteil von 60 Gew.-%, noch weiter bevorzugt einen Anteil von 70 Gew.-% und insbesondere bevorzugt einen Anteil von 75 Gew.-% an ungesättigten Fettsäuren. Sollten kommerziell erhältliche Öle, Fette oder Wachse verwendet werden, welche einen geringeren Anteil als 40 Gew.-% an Verbindungen mit mindestens einer Mehrfachbindung enthalten, so können ungesättigte Verbindungen in dem Maße zugesetzt werden, dass der Anteil an ungesättigten Verbindungen auf über 40 Gew.-% ansteigt. Bei einem Anteil von weniger als 40 Gew.-% sinkt die Polymerisationsgeschwindigkeit zu stark ab, so dass gleichmäßige Beschichtungen nicht mehr gewährleistet werden können.

Die Eigenschaft zur Polymerisation macht vor allem die Lipide mit hohen Anteilen mehrfach ungesättigter Fettsäuren zu hervorragenden Substanzen für die vorliegende Erfindung.

So besitzt die Linolsäure (Octadecadiensäure) zwei Doppelbindungen und die Linolensäure (Octadecatriensäure) drei Doppelbindungen. Eicosapentaensäure (EPA C20:5) hat fünf Doppelbindungen und Docosahexaensäure (DHA C22:6) besitzt sechs Doppelbindungen in einem Molekül. Mit der Anzahl der Doppelbindungen steigt auch die Bereitschaft zur Polymerisation.
Diese Eigenschaften der ungesättigten Fettsäuren und deren Mischungen sowie deren Neigung zur Autopolymerisation lässt sich zur biokompatiblen und flexiblen Beschichtung von medizinischen Oberflächen, speziell von Stents mit z.B. Fischöl, Lebertran oder Leinöl nutzen (siehe Beispiele 13-18).

Linolsäure wird auch als cis-9, cis-12-Octadecadiensäure (chemische Nomenklatur) oder als Δ9,12-Octadecadiensäure oder als Octadecadiensäure (18:2) bzw. Octadecadiensäure 18:2 (n-6) bezeichnet (biochemische bzw. physiologische Nomenklatur). Bei Octadecadiensäure 18:2 (n-6) ist n die Anzahl an Kohlenstoffatomen und die Zahl "6" gibt die Position der letzten Doppelbindung an. 18:2 (n-6) ist somit eine Fettsäure mit 18 Kohlenstoffatomen, zwei Doppelbindungen und einem Abstand von 6 Kohlenstoffatomen von der letzten Doppelbindung bis zur äußeren Methylgruppe.

Bevorzugt werden für die vorliegende Erfindung die folgenden ungesättigten Fettsäuren als Substanzen eingesetzt, welche an der Polymerisationsreaktion teilnehmen bzw. Substanzen, welche diese Fettsäuren enthalten oder Substanzen, welche den Alkylrest dieser Fettsäuren, also ohne die Carboxylatgruppe (-COOH), enthalten.

**Tabelle 1: Monoolefinische Fettsäuren**

| **Systematischer Name** | **Trivialname** | **Kurzform** |
|---|---|---|
| cis-9-Tetradecensäure | Myristoleinsäure | 14:1(n-5) |
| cis-9-Hexadecensäure | Palmitoleinsäure | 16:1(n-7) |
| cis-6-Octadecensäure | Petroselinsäure | 18:1(n-12) |
| cis-9-Octadecensäure | Ölsäure | 18:1(n-9) |
| cis-11-Octadecensäure | Vaccensäure | 18:1(n-7) |
| cis-9-Eicosensäure | Gadoleinsäure | 20:1(n-11) |
| cis-11-Eicosensäure | Gondoinsäure | 20:1(n-9) |
| cis-13-Docosensäure | Erucinsäure | 22:1(n-9) |
| cis-15-Tetracosensäure | Nervonsäure | 24:1(n-9) |
| t9-Octadecensäure | Elaidinsäure | |
| t11-Octadecensäure | t-Vaccensäure | |
| t3-Hexadecensäure | | trans-16:1 n-13 |

**Tabelle 2: Polyungesättigte Fettsäuren**

| **Systematischer Name** | **Trivialname** | **Kurzform** |
|---|---|---|
| 9,12-Octadecadiensäure | Linolsäure | 18:2(n-6) |
| 6,9,12-Octadecatriensäure | γ-Linolsäure | 18:3(n-6) |
| 8,11,14-Eicosatriensäure | Dihomo-γ-linolensäure | 20:3(n-6) |
| 5,8,11,14-Eicosatetraensäure | Arachidonsäure | 20:4(n-6) |
| 7,10,13,16-Docosatetraensäure | - | 22:4(n-6) |
| 4,7,10,13,16-Docosapentaensäure | - | 22:5(n-6) |
| 9,12,15-Octadecatriensäure | α-Linolensäure | 18:3(n-3) |
| 6,9,12,15-Octadecatetraensäure | Stearidonsäure | 18:4(n-3) |
| 8,11,14,17-Eicosatetraensäure | - | 20:4(n-3) |
| 5,8,11,14,17-Eicosapentaensäure | EPA | 20:5(n-3) |
| 7,10,13,16,19-Docosapentaensäure | DPA | 22:5(n-3) |
| 4,7,10,13,16,19-Docosahexaensäure | DHA | 22:6(n-3) |
| 5,8,11-Eicosatriensäure | Meadsäure | 20:3(n-9) |
| 9c 11t 13t Eleostearinsäure | | |
| 8t 10t 12c Calendinsäure | | |
| 9c 11t 13c Catalpicsäure | | |
| 4, 7, 9, 11, 13, 16, 19 Docosaheptadecansäure | Stellaheptaensäure | |
| | Taxolsäure | all-cis-5,9-18:2 |
| | Pinolensäure | all-cis-5,9,12-18:3 |
| | Sciadonsäure | all-cis-5,11,14-20:3 |

**Tabelle 3: Acetylenische Fettsäuren**

| **Systematischer Name** | **Trivialname** |
|---|---|
| 6-Octadecinsäure | Taririnsäure |
| t11-Octadecen-9-insäure | Santalbin- oder Ximeninsäure |
| 9-Octadecinsäure | Stearolinsäure |
| 6-Octadecen-9-insäure | 6,9-Octadeceninsäure∼ |
| t10-Heptadecen-8-insäure | Pyrulinsäure |
| 9-Octadecen-12-insäure | Crepenynsäure |
| t7,t11-Octadecadiene-9-insäure | Heisterinsäure |
| t8,t10-Octadecadiene-12-insäure | - |
| 5,8,11,14-Eicosatetrainsäure | ETYA |

Nach der Durchführung der beschriebenen Polymerisation der einen linearen oder verzweigten und einen substituierten oder unsubstituierten Alkylrest mit mindestens einer Mehrfachbindung enthalten Substanzen, wird eine Oberfläche eines Medizinproduktes erhalten, welche zumindest teilweise mit einer polymeren Schicht versehen ist. Im Idealfall entsteht eine homogene gleichmäßig dicke polymere Schicht auf der gesamten äußeren Oberfläche des Stents oder eines Katheterballons mit oder ohne aufgesetztem Stent. Diese polymere Schicht auf der Oberfläche des Stents oder des Katheterballons mit oder ohne Stent besteht aus den polymerisierbaren Substanzen und schließt die nicht aktiv an der Polymerisationsreaktion teilnehmenden Substanzen und/oder Wirkstoffe und/oder Rapamycin in der Polymermatrix ein. Vorzugsweise ist der Einschluss so ausgestaltet, dass die nicht an der Polymerisation teilnehmenden Substanzen, vor allem Rapamycin und zusätzliche Wirkstoffe aus der Polymermatrix hinausdiffundieren können.

Der biokompatible Überzug aus den polymerisierten Substanzen sorgt für die notwendige Blutverträglichkeit des Stents oder des Katheterballons mit oder ohne Stent und stellt gleichzeitig einen geeigneten Träger für Rapamycin und andere Wirkstoffe dar. Ein hinzugefügter Wirkstoff (oder Wirkstoffkombination), der über die Gesamtoberfläche des Stents und/oder Katheterballons gleichmäßig verteilt ist, bewirkt, dass der Bewuchs der Oberfläche mit Zellen, insbesondere glatten Muskel- und Endothelzellen, in einer kontrollierten Weise abläuft. Es findet somit keine rasche Besiedlung und Überwucherung einer Stentoberfläche mit Zellen statt, was zu einer Restenose führen könnte, wohingegen der Bewuchs der Stentoberfläche mit Zellen aber auch nicht durch eine hohe Medikamentenkonzentration vollkommen unterbunden wird, was die Gefahr einer Thrombose mit sich bringt. Diese Kombination beider Effekte verleiht der erfindungsgemäßen Oberfläche eines Medizinprodukts, insbesondere der Oberfläche eines Stent die Fähigkeit, schnell in die Gefäßwand einzuwachsen und vermindert sowohl das Risiko einer Restenose als auch das Risiko einer Thrombose. Die Freisetzung des oder der Wirkstoffe erstreckt sich über einen Zeitraum von 1 bis 12 Monaten, bevorzugt über 1 - 2 Monate nach der Implantation.

Weitere bevorzugte Stents mit Rapamycin als Wirkstoff zur Freisetzung bieten eine deutlich vergrößerte Oberfläche für die Beladung mit Rapamycin, da bei diesen Stents nicht nur die Stentstreben sondern auch die Zwischenräume zwischen den Stentstreben mit einem Polymer oder einer Trägermatrix überzogen werden, worin sich Rapamycin befindet. Derartige vollflächig, also Stentstreben und Strebenzwischenräume, beschichtete Stents werden nach einem besonderen Verfahren hergestellt, welches ausführlich in der internationalen Patentanmeldung PCT / DE 2006 / 000766 mit dem Titel "Vollflächige Beschichtung von Gefäßstützen" als auch der deutschen Patentanmeldung DE 10 2005 021 622.6 der Hemoteq GmbH beschrieben ist.

Dies wurde dadurch erreicht, dass das gesamte gitterförmige bzw. netzartige Gerüst der Gefäßstütze vollflächig überzogen wird. Unter vollflächiger Beschichtung wird eine die Zwischenräume vollflächig überdeckende Beschichtung verstanden. Diese Beschichtung wird auch als durchgängig bezeichnet, d.h. es wird ein Film über einen Zwischenraum ausgebildet, der nur auf den diesen Zwischenraum umschließenden Streben aufliegt. Diese Beschichtung überspannt den Zwischenraum wie eine Hängebrücke, welche nur an den Rändern befestigt ist und im Zwischenraum auf keinem massiven Untergrund aufliegt. Damit diese vollflächige Beschichtung genügend an den Streben bzw. auf der Gefäßstütze haftet, werden in einem ersten Beschichtungsschritt mit einem Polymer A die Streben zumindest teilweise beschichtet, jedoch ohne die Zwischenräume zu überdecken und nach Benetzung bzw. Anlösung dieser ersten polymeren Beschichtung erfolgt dann die flächendeckende Beschichtung mit einem Polymer B in einem zweiten Beschichtungsschritt, wobei die erste polymere Beschichtung für eine bessere Anhaftung der zweiten vollflächigen bzw. durchgängigen polymeren Schicht sorgt.

Dabei können auch Polymer A und Polymer B identisch sein und sich vorzugsweise nur in ihrer Konzentration in der Beschichtungslösung unterscheiden.

Die Streben bzw. Knotenpunkte werden mit der ersten Beschichtung wie ein Schlauch oder eine Isolierung um einen Draht umgeben, wobei diese Beschichtung jedoch nur die einzelnen Streben umgibt und nicht schon zwei benachbarte Streben miteinander verbindet. Die erste Beschichtung dient als Untergrund für eine bessere Anhaftung der nachfolgenden die Zwischenräume zwischen den Streben und Knotenpunkten überspannende Beschichtung.

Die einzelnen Streben oder Knotenpunkte der Gefäßstütze können zudem Aussparungen oder Kavitäten aufweisen, welche sich beispielsweise mit einem pharmakologischen Wirkstoff füllen und mit der ersten polymeren Beschichtung als auch mit der zweiten Beschichtung überdecken ließen. Eine derartige Überdeckung solcher Aussparungen und Kavitäten ist Stand der Technik und nur eine bevorzugte Ausführungsform aber nicht der wesentliche Aspekt der vorliegenden Erfindung.

Die unbeschichtete Gefäßstütze bzw. der Bare-Stent kann aus gängigen Materialien wie beispielsweise medizinischem Edelstahl, Titan, Chrom, Vanadium, Wolfram, Molybdän, Gold, Nitinol, Magnesium, Zink, Legierungen der vorgenannten Metalle oder Keramiken oder Polymeren bestehen. Diese Materialien sind entweder selbstexandierbar oder ballonexpandierbar und biostabil oder biologisch abbaubar.

Vorzugsweise wird der Beschichtungsschritt b) mittels Sprühverfahren oder electro spinning ausgeführt, während die Schritte c) und d) bevorzugt mittels Tauchverfahren, Mikropipettiertechnik, Electrospinning, oder/und "Seifenblasenverfahren" durchgeführt werden.

Die polymere Oberfläche kann in einem weiteren Schritt mit einem Polymer C vollständig oder teilweise von innen und/oder außen beschichtet werden.
So ist beispielsweise für die luminale Seite eines Trachea Bronchial-Stents von Wichtigkeit, dass diese gleitfähig genug ist, damit der Abtransport von Sekret, Schleim etc. nicht behindert wird. Die Hydrophilie kann durch Beschichtung mit einem geeigneten Polymer wie beispielsweise Polyvinylpyrrolidon (PVP) gesteigert werden.

Mit diesem Beschichtungsverfahren lassen sich zum jetzigen Stand der Technik auftretenden und erwähnten Nachteile einer flächendeckenden Beschichtung verhindern und die damit für den Patienten entstehenden Risiken vermeiden. Derartige erfindungsgemäß einsetzbare Medizinprodukte können einerseits beschichtet werden, indem auf dem massiven Material, beispielsweise den einzelnen Streben eines Stents eine Beschichtung aufgetragen wird und die die durch die Streben begrenzte freie Ebene mit einer polymeren Schicht B gefüllt wird. Diese polymere Schicht vermag aufgrund der Polymereigenschaften die Zwischenräume der mit Polymer A beschichteten Stentstreben zu überdecken. Die Stabilität der Einhüllung wird durch den Verbund der beiden Schichten des Polymeren B mit dem Polymeren A rund um die Elemente des Medizinproduktes bestimmt.
Somit können alle Medizinprodukte erfindungsgemäß beschichtet werden, welche eben solche freien Zwischenräume in der Struktur aufweisen, wie es beispielsweise Stents zwischen den einzelnen Stentstreben tun.

Dabei ist die Verwendung eines bioabbaubaren oder/und biostabilen Polymers A für die erste Beschichtung und eines biologisch abbaubaren oder resorbierbaren Polymer B oder/und biostabiles Polymer für die überdeckende zweite Beschichtung in Abhängigkeit von der Art der Anwendung möglich.

Ferner lässt sich in einem dem Beschichtungsschritt mit Polymer A vorgelagerten Schritt eine hämokompatible Schicht vorzugsweise kovalent auf die unbeschichtete Oberfläche des Medizinproduktes gebundene oder mittels Quervernetzung z.B. mit Glutardialdehyd auf der Oberfläche des Medizinproduktes immobilisieren. Eine solcherart die Blutgerinnung nicht aktivierende Schicht ist dann sinnvoll, wenn unbeschichtetes Stentmaterial in Kontakt mir Blut kommmen kann. So ist zu bevorzugen, einen partiell beschichteten Stent, wie er z.B. in US595159 zur Behandlung von Aneurysmen beschrieben wird, zunächst mit dieser hämokompatible Schicht auszustatten.

Ferner ist bevorzugt, dass bei dem zweiten vollflächigen Beschichtungsschritt keine glatte oder plane äußere Oberfläche resultiert, sondern beispielsweise bei einem Stent die Stentstruktur, d.h. die Struktur der Streben erkenntlich bleibt. Dies hat den Vorteil, dass die äußere zur Gefäßwand gerichtete beschichtete Oberfläche der Gefäßstütze eine gewellte und unebene Struktur aufweist, wodurch ein besserer Halt im Gefäß garantiert wird.

Das Polymer A, welches die Stentstreben umgibt, kann einen zusätzlichen antiproliferativen, antimigrativen, antiangiogenen, antiinflammatorischen, antiphlogistischen, zytostatischen, zytotoxischen und/oder antithrombotischen Wirkstoff enthalten, wobei das Polymer B, welches den Stent flächendeckend überzieht, den Wirkstoff Rapamycin enthält. Die Rapamycin-freisetzende Oberfläche ist daher deutlich vergrößert gegenüber einer herkömmlichen Beschichtung, welche nur die einzelnen Stentstreben umgibt (siehe Beispiel Nr 18). Die Konzentration an Rapamycin und falls vorhanden pro weiterem Wirkstoff liegt vorzugsweise im Bereich von 0,001-500 mg pro cm² vollflächig beschichteter Oberfläche der Gefäßstütze, d.h. die Oberfläche wird unter Berücksichtigung der Gesamtoberfläche der beschichteten Streben und der Oberfläche der überdeckten Zwischenräume zwischen den Streben berechnet.

Die erfindungsgemäßen Verfahren sind zur Beschichtung von beispielsweise Gefäßstützen und insbesondere Stents wie beispielsweise Koronarstents, Vaskularstents, Tracheastents (Luftröhrenstent), Bronchialstents, Harnröhrenstents, Speiseröhrenstents (Ösophagusstent), Gallenstents, Nierenstents, Dünndarmstents, Dickdarmstents geeignet. Zudem können Führungsdrähte, Spiralen, Katheter, Kanülen, Schläuche sowie allgemein röhrenförmige Implantate oder Teile der vorgenannten Medizinprodukte erfindungsgemäß beschichtet werden, wenn ein mit einem Stent vergleichbares Strukturelement in einem solchen Medizinprodukt enthalten ist. Sofern expandierbare Medizinprodukte bzw. Gefäßstützen eingesetzt werden, erfolgt die Beschichtung vorzugsweise im expandierten Zustand.

Die beschichteten Medizinprodukte werden insbesondere zur Offenhaltung aller gangartigen Strukturen eingesetzt, beispielsweise von Harnwegen, Speiseröhren, Luftröhren, Gallenwegen, Nierenwegen, Blutgefäßen im gesamten Körper einschließlich Gehirn, Duodenum, Pilorus, Dünn- und Dickdarm aber auch zum Offenhalten künstlicher Ausgänge wie sie für den Darm oder für die Luftröhre verwendet werden.

Somit sind die beschichteten Medizinprodukte zur Verhinderung, Verminderung oder Behandlung von Stenosen, Restenosen, Arteriosklerose, Atherosklerose, und allen anderen Formen des Gefäßverschlüsses oder Gefäßverengungen von Durchgängen oder Ausgängen geeignet.

Ferner ist bevorzugt, wenn die vollflächige Beschichtung mit Polymer B über die Länge der Gefäßstütze hinausgeht und nicht mit den Enden der Gefäßstütze abschließt. Die überstehende Hülle wird in einem weiteren Schritt um die Ränder der Gefäßstütze zur Außenseite gelegt und die entstehenden Kanten unter Druck und erhöhter Temperatur in die darunterliegende Polymerschicht B integriert. Damit ist eine durchgängige Beschichtung auch der Ränder der Gefäßstütze gewährleistet und die Gefahr der Ablösung an diesen Schwachstellen ist nicht gegeben. Ausserdem lässt sich auf diese Weise unter den Rand eine Zugvorrichtung einbringen, mit der der Stent jederzeit sicher wieder entfernt werden kann. So lässt sich in der Faltung ein polymerer Faden umlegen, der an einer oder zwei gegenüberliegenden Seiten durch die Polymerschicht als Schlinge aus dem Rand nach außen ragt.
Eine weitere Möglichkeit bietet sich in der Nutzung dieses Randbereiches als Reservoir für Wirkstoffe bzw. zum Einbringen von Wirkstoffen speziell in diesen Randbereich, die sich durchaus von in/auf dem vollflächig beschichteten Hohlkörper eventuell vorhandenen Wirkstoffen unterscheiden.

Dabei erhält die den Stent umgebende Hülle die Flexibilität des Stents, trägt aber gleichzeitig zur mechanischen Festigkeit des Medizinproduktes bei. Es besteht mit diesem zusätzlich die Möglichkeit gezielt Wirkstoffe seitenspezifisch einzubringen, so z.B. ein Cytostatikum, dass von der äußeren Oberfläche in die Gefäßwand diffundieren kann und beispielsweise ein Antibiotikum in der Innenfläche des Medizinproduktes Infektionen verhindert. Zudem lassen sich zusätzlich durch die Möglichkeit der unterschiedlichen Beschichtung der Innen- und Außenseite weitere Optimierungen in der Anpassungen an die vor Ort herrschenden physiologischen Bedingungen realisieren.

Weitere Additive sind möglich, z.B. Substanzen, wie Bariumsulfat oder Edelmetalle, die ein implantiertes derart beschichtetes Medizinprodukt röntgenographisch sichtbar machen können. Des weiteren lassen sich die Außenfläche und die Innenfläche mit unterschiedlichem Material ummanteln wie oben ausgeführt. So lässt sich beispielsweise ein Medizinprodukt herstellen, dass auf der Außenseite eine hydrophobe Polymerhülle besitzt, während die Innenfläche aus hydrophilem Polymer besteht.

Dieses Verfahren bietet daher eine Vielfalt von Möglichkeiten, Medizinprodukte durch Variation und Kombination jedwede biostabile und biodegradierbare BeschichtungsMaterialien mit und ohne Additive auf Medizinprodukte im Bedarfsfall in Form einer Hülle aufzubringen.

Gleichzeitig kann der Überzug zur mechanischen Festigkeit eines Implantates beitragen ohne seine Flexibilität zu beeinträchtigen.

So ist beispielsweise bis heute der Einsatz von Stents zum Zurückdrängen von Gallengangkarzinomen nicht üblich. Doch kann nur in ca. 10% der Fälle die chirurgische Entfernung durchgeführt werden. Die Lebenserwartung dieser Patienten liegt bei durchschnittlich 1 Jahr. Der Einsatz eines mit Hilfe dieses Verfahrens vollständig beschichteten und für den Gallengang geeigneten Implantates, welches z.B. ein Chemotherapeutikum enthält, könnte zum einen die Verengung des Körperdurchganges durch Gegendruck der Gefäßstütze verhindern und gleichzeitig die Tumorbildung verlangsamen oder sogar stoppen und würde somit zumindest eine lebensverlängernde Maßnahme bei hoher oder guter Lebensqualität darstellen (Beispiel 18).

Ferner lässt sich die erfindungsgemäße Beschichtung auch im vaskulären System nutzen. Bei der Aneurysmenbildung beispielsweise so, dass die Beschichtung verhindert, dass sich das Aneurysma durch weitere Blutzufuhr vergrößern kann (Beispiel Nr. 19)

Weitere erfindungsgemäße Ausführungsformen zur Vergrößerung der Oberfläche betreffen Kathetersysteme insbesondere Dilatationskathetersystems umfassend einen Katheterballon mit einem aufgesetzten (gecrimpten) Stent. Bei diesen Systemen wird ein unbeschichteter oder beschichteter Stent auf den Katheterballon gecrimpt und danach zusammen mit dem Katheterballon beschichtet.
Die Beschichtung kann derart erfolgen, dass die feinen Zwischenräume zwischen den einzelnen Stentstreben des gecrimpten Stents als Reservoire für einen Wirkstoff oder für Rapamycin verwendet werden. Beispielsweise kann Rapamycin oder einer der hierin genannten Wirkstoffe in einem geeigneten Lösungsmittel gelöst und auf Stent und Ballon aufgetragen werden. Wirkstoff samt Lösungsmittel läuft in die Zwischenräume zwischen den einzelnen Stentstreben und in die Zwischenräume zwischen Katheterballon und Innenseite des Stents, wobei das Lösungsmittel verdunstet und der reine Wirkstoff zurückbleibt. Danach können eine oder mehrere Trägerschichten auf den Katheterballon mit Stent aufgetragen werden.

Eine bevorzugte Variante dieser Ausführungsform weist reines Paclitaxel zwischen den Stentstreben und zwischen Ballon und Stent auf, welches durch Sprüh- oder Tauchverfahren aufgebracht wurde und nach Verdunstung des Lösungsmitteln dort zurückbleibt. Diese erste Paclitaxel-Beschichtung wird danach mit einem vorzugsweise biologisch abbaubaren Polymer und/oder vorzugsweise polaren, hydrophilen Polymer überdeckt, welches den Wirkstoff Rapamycin enthält.

Eine andere bevorzugte Ausführungsform weist keinen Träger oder keine polymere Schicht auf, sondern nur reines Rapamycin, welches zusammen mit einem Lösungsmittel auf Stent und Katheterballon aufgetragen worden ist und am Stent und Ballon nach Verdunstung des Lösungsmittels verbleibt.

Eine dritte bevorzugte Ausführungsform umfasst einen Stent, der mit vorzugsweise einem biostabilen Polymer enthaltend Rapamycin beschichtet ist und auf den Katheterballon gecrimpt wurde. Unbeschichteten Katheterballon mit Rapamycinenthaltendem beschichtetem Stent wird dann mit Paclitaxel aufgenommen in einem geeigneten Lösungsmittel besprücht, so dass sich nach Verdunstung des Lösungsmittels eine unregelmäßige Schicht aus reinem Paclitaxel auf dem Stent und Ballon befindet.

### Kontrastmittel

Von besonderem Interesse sind die erfindungsgemäßen Ausführungsformen, welche als Matrix oder Träger für Rapamycin keine Polymere sondern niedermolekulare chemische Verbindungen und insbesondere Kontrastmittel und Kontrastmittelanaloga einsetzen.

Derartige Kontrastmittel und/oder Kontrastmittelanaloga enthalten zumeist Barium, Iod, Mangan, Eisen, Lanthan, Cer, Praseodym, Neodym, Samarium, Europium, Gadolinium, Terbium, Dysprosium, Holmium, Erbium, Thulium, Ytterbium und/oder Lutetium bevorzugt als Ionen in gebundener und/oder komplexierter Form.

Prinzipiell zu unterscheiden sind Kontrastmittel für verschiedene bildgebende Verfahren. Zum einen gibt es Kontrastmittel, die bei Röntgenuntersuchungen zum Einsatz kommen (Röntgenkontrastmittel) oder Kontrastmittel für magnetresonanztomographische Untersuchungen (MR-Kontrastmittel).

Im Falle von Röntgenkontrastmitteln handelt es sich um Substanzen, die entweder zu einer vermehrten Absorption einfallender Röntgenstrahlen gegenüber der umgebenden Struktur führen (sog. positive Kontrastmittel) oder einfallende Röntgenstrahlen vermehrt ungehindert durchlassen (sog. negative Kontrastmittel).

Bevorzugte Röntgenkontrastmittel sind diejenigen, welche zur Gelenkdarstellung (Arthrographie) und bei der CT (Computertomographie) eingesetzt werden. Der Computertomograph ist ein Gerät zur Aufnahme von Schnittbildern des menschlichen Körpers mit Hilfe von Röntgenstrahlen.

Obwohl erfindungsgemäß auch Röntgenstrahlung für die Detektion bei den bildgebenden Verfahren eingesetzt werden kann, ist diese Strahlung aufgrund ihrer Schädlichkeit nicht bevorzugt. Bevorzugt ist, wenn es sich bei der einfallenden Strahlung um nichtionisierende Strahlung handelt.

Als bildgebende Verfahren werden Röntgenaufnahmen, Computertomographie (CT), Kernspintomographie, Magnetresonanztomographie (MRT) und Ultraschall eingesetzt, wobei Kernspintomographie und Magnetresonanztomographie (MRT) bevorzugt sind.

Somit sind als Substanzen, welche aufgrund ihrer Fähigkeit durch einfallende Strahlung angeregt zu werden, und somit eine Detektierbarkeit des Medizinprodukts bei in-vivo-Ereignissen durch bildgebende Verfahren ermöglichen, insbesondere solche Kontrastmittel bevorzugt, welche bei der Computertomographie (CT), Kernspintomographie, Magnetresonanztomographie (MRT) oder Ultraschall eingesetzt werden. Die bei der MRT eingesetzten Kontrastmittel beruhen auf dem Wirkungsprinzip, dass sie eine Änderung des magnetischen Verhaltens der zu differenzierenden Strukturen bewirken.

Des weiteren sind iodhaltige Kontrastmittel bevorzugt, welche bei der Gefäßdarstellung (Angiographie und Phlebographie) und bei der CT (Computertomographie) verwendet werden.

Als iodhaltige Kontrastmittel können folgende Beispiele genannt werden:

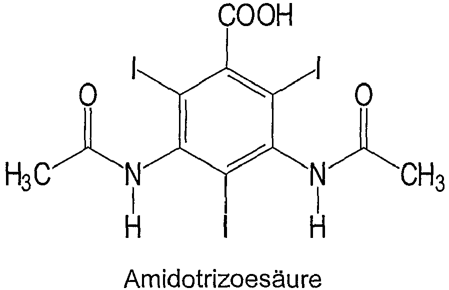

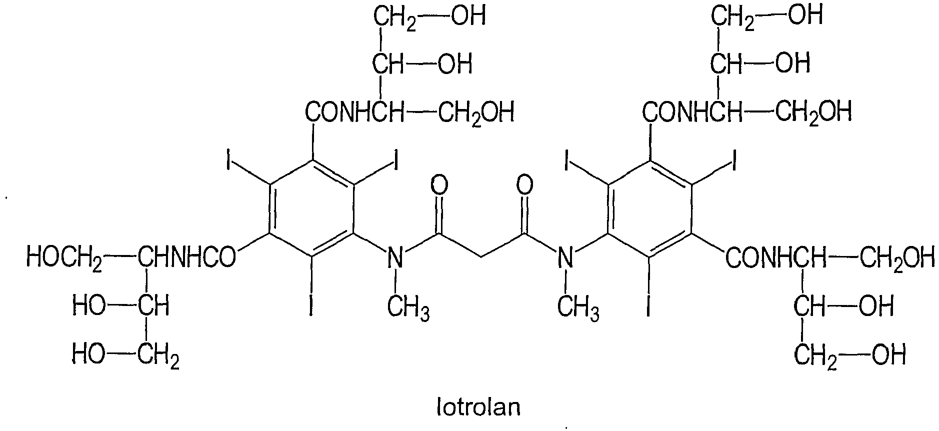

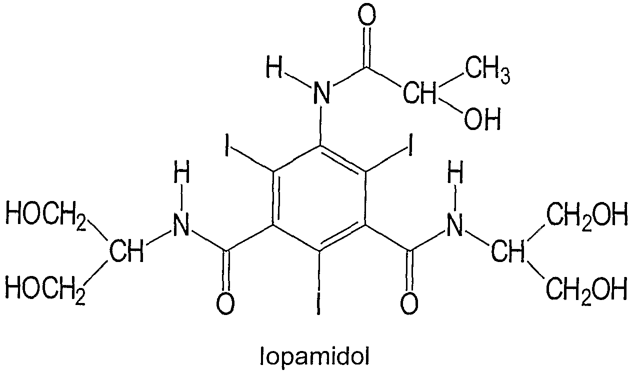

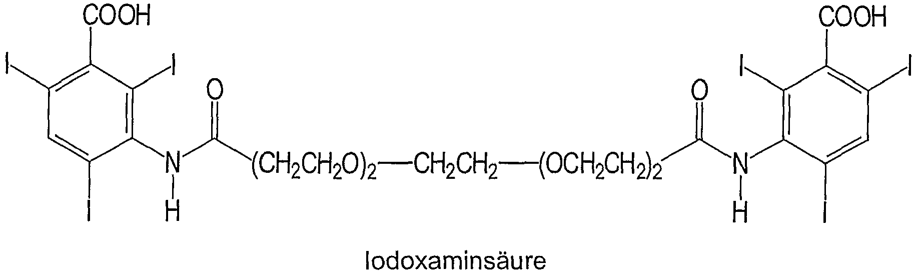

Ein weiteres Beispiel ist Jod-Lipiodol®, ein iodiertes Oleum papaveris, ein Mohnöl. Unter den Handelsnamen Gastrografin® und Gastrolux® ist die Muttersubstanz der iodierten Kontrastmittel, das Amidotrizoat in Form von Natrium- und Megluminsalzen kommerziell erhältlich.

Auch Gadolinium-enthaltende oder superparamagnetische Eisenoxidpartikel sowie ferrimagnetische oder ferromagnetische Eisenpartikel wie beispielsweise Nanopartikel sind bevorzugt.

Eine weitere Klasse von bevorzugten Kontrastmitteln stellen die paramagnetischen Kontrastmittel dar, welche zumeist ein Lanthanoid enthalten.

Zu den paramagnetischen Substanzen, die über ungepaarte Elektronen verfügen, zählt z.B. das Gadolinium (Gd³⁺), das insgesamt sieben ungepaarte Elektronen besitzt. Des weiteren gehören zu dieser Gruppe das Europium (Eu²⁺, Eu³⁺), Dysprosium (Dy³⁺) und Holmium (Ho³⁺). Diese Lanthanoide können auch in chelatisierter Form unter Verwendung von beispielsweise Hämoglobin, Chlorophyll, Polyazasäuren, Polycarbonsäuren und insbesondere EDTA, DTPA sowie DOTA als Chelatbildner eingesetzt werden.

Beispiele für Gadolinium-haltige Kontrastmittel sind Gadolinium-Diethylentriaminpentaessigsäure oder

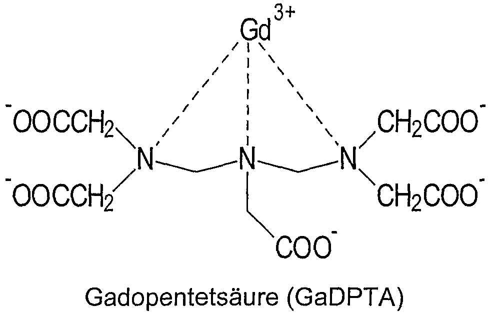

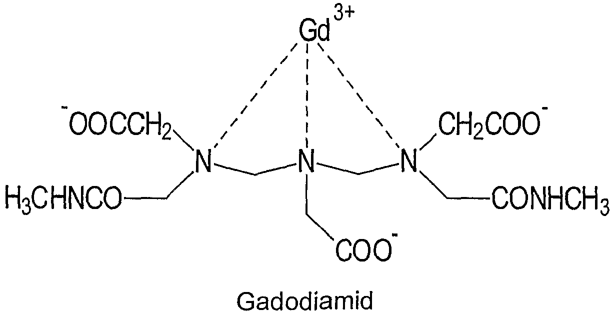

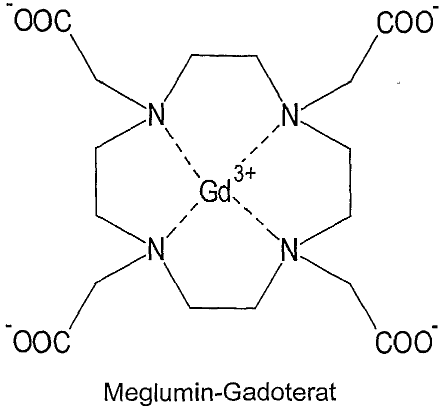

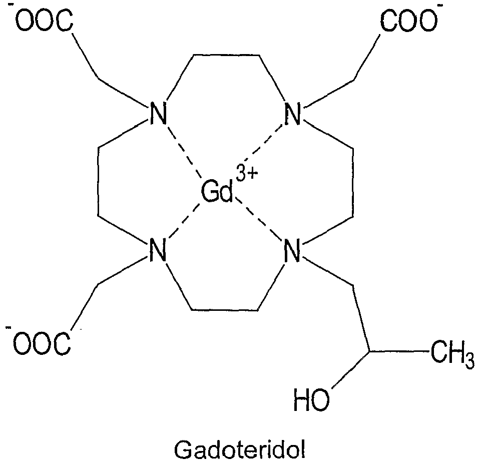

Weitere erfindungsgemäß einsetzbare paramagnetische Substanzen sind Ionen sogenannter Übergangsmetalle wie Kupfer (Cu²⁺), Nickel (Ni²⁺), Chrom (Cr²⁺, Cr³⁺), Mangan (Mn²⁺, Mn³⁺) und Eisen (Fe²⁺, Fe³⁺). Auch diese Ionen können in chelatisierter Form eingesetzt werden.

Die mindestens eine Substanz, welche aufgrund ihrer Fähigkeit durch einfallende Strahlung angeregt zu werden, eine Darstellbarkeit des Grundkörpers bei in-vivo-Ereignissen durch bildgebende Verfahren ermöglicht, befindet sich entweder auf der Oberfläche des Grundkörpers oder im Inneren des Grundkörpers.

Bei der einen bevorzugten Ausführungsform wird der Ballon des Katheters in seiner komprimierten Form im Inneren mit einem Kontrastmittel und/oder Kontrastmittelanalogon gefüllt. Das Kontrastmittel liegt bevorzugt als Lösung vor. Neben den Eigenschaften des Kontrastmittels oder Kontrastmittelanalogons als Träger oder Matrix für das Rapamycin haben derartige Beschichtungen zudem noch den Vorteil, den Katheterballon bei den bildgebenden Verfahren besser sichtbar, d.h. detektierbar zu machen. Die Expansion des Ballons erfolgt, indem der Ballon durch weiteres Einfüllen einer Kontrastmittellösung ausgedehnt wird.

Ein Vorteil dieser Ausführungsform ist, dass das Kontrastmittel bzw. Kontrastmittelanalogon beliebig oft wiederverwendet werden kann und nicht in den Körper gelangt und somit zu keinen schädlichen Nebenwirkungen führt.

Als Kontrastmittelanaloga werden kontrastmittelähnliche Stoffe bezeichnet, welche die Eigenschaften von Kontrastmitteln aufweisen, d.h. durch bei einer Operation einsetzbare bildgebende Verfahren sichtbar gemacht werden können.

Somit umfassen weitere bevorzugte Ausführungsformen der vorliegenden Erfindung einen mit Rapamycin und einem Kontrastmittel oder einem Kontrastmittelanalogon beschichtete Katheterballons. Befindet sich ein beschichteter oder unbeschichteter Stent auf dem Katheterballon, so können natürlich auch Ballon mit Stent gemeinsam beschichtet werden. Zu diesem Zweck wird Rapamycin eventuell zusammen mit einem oder mehreren anderen Wirkstoffen in dem Kontrastmittel gelöst oder suspendiert und auf den Katheterballon mit oder ohne Stent aufgetragen. Ferner besteht die Möglichkeit, dem Gemisch aus Kontrastmittel und Rapamycin noch ein Lösungsmittel zuzusetzen, welches nach der Beschichtung verdunstet oder im Vakuum entfernt werden kann. Es besteht zudem die Möglichkeit, dass unter und/oder auf die Kontrastmittel-enthaltende Schicht noch eine oder mehrere Schichten aus einem reinen Wirkstoff oder einem Polymer oder einem Wirkstoffenthaltenden Polymer aufgetragen wird/werden.

Eine insbesondere bevorzugte Ausführungsform verwendet einen Katheterballon mit gecrimptem Stent. Bei dem Stent kann es sich um einen unbeschichteten (bare) Stent oder vorzugsweise einen nur mit einer hämokompatiblen Schicht überzogenen Stent handeln. Als hämokompatible Beschichtung sind insbesondere die hierin offenbarten Heparin-Derivate oder Chitosan-Derivate bevorzugt und vorrangig defulfatiertes und reacetyliertes oder repropionyliertes Heparin. Das System aus Katherballon und Stent wird mit einer Lösung bzw. Suspension bzw. Dispersion von Rapamycin zusammen mit z.B. Paclitaxel oder Thalidomid in einem Kontrastmittel besprüht oder darin getaucht (siehe Beispiel 20).
Es ist auch möglich, besonders ausgestaltete Katheterballons wie beispielsweise Faltenballons zu verwenden. Derartige Faltenballons bilden im komprimierten Zustand des Ballons Falten, welche sich mit einem Wirkstoff wie beispielsweise mit reinem Rapamycin, oder mit einem Gemisch aus Rapamycin und einem Lösungsmittel oder einem Kontrastmittel oder einem Gemisch aus Rapamycin und einem Öl oder einem Polymer in einem geeigneten Lösungsmittel befüllen lassen. Ein eventuell verwendetes Lösungsmittel kann unter vermindertem Druck entfernt und die sich in den Falten befindende Mischung dadurch getrocknet werden. Bei der Dilatation eines solchen Faltenballons, der in der Regel ohne Stent verwendet wird, kehren oder wölben sich die Falten nach außen und geben dadurch ihren Inhalt an die Gefäßwand ab.
Eine weitere bevorzugte Ausführungsform des Stents bzw. Katheters besteht im Einsatz von Transportvermittlern, die den Zugang des bzw. der Wirkstoffe in die Zelle beschleunigen oder unterstützen. Nicht selten haben diese Stoffe gleichsam eine unterstützende oder auch synergistische Wirksamkeit.Hierzu zählen beispielsweise die Vasodilatoren, zu denen körpereigene Substanzen wie die Kinine, z.B. Bradykinin, Kallidin, Histamin oder die NOS-Synthase, die aus L-Arginin das vasodilatatorisch wirkende NO freisetzt. Substanzen pflanzlichen Ursprungs wie der Extrakt des Gingko biloba, DMSO, Xanthone, Flavonoide, Terpenoide, pflanzliche und tierische Farbstoffe, Lebensmittelfarben, NO-freisetzende Substanzen wie z.B. das Pentaerythrytiltetranitrat (PETN), Kontrastmittel und Kontrastmittelanaloga gehören ebenfalls zu diesen Hilfsmitteln bzw. sind selbst als Wirkstoff synergistisch einsetzbar.
Weitere zu nennende Substanzen sind 2-Pyrrolidon, Tributyl- und Triethylcitrat wie deren acteylierten Derivate, Bibutylphtalat, Benzoesäurebenzylester, Diethanolamin, Diethylphtalat, Isopropylmyristate und -palmitate, Triacetin usw.

### Stentmaterialien

Die herkömmlichen Stents, welche gemäß der erfindungsgemäßen Verfahren beschichtet werden können, können aus gängigen Materialien wie beispielsweise medizinischem Edelstahl, Titan, Chrom, Vanadium, Wolfram, Molybdän, Gold, Nitinol, Magnesium, Zink, Legierungen der vorgenannten Metalle oder Keramiken oder biostabilen oder/und bioabbaubaren Polymeren bestehen. Diese Materialien sind entweder selbstexpandierbar oder ballonexpandierbar, biostabil und/oder biologisch abbaubar.

### Ballonmaterialien

Der Katheterballon kann aus den gängigen Materialien, insbesondere Polymeren bestehen, wie sie weiter unter beschrieben werden und insbesondere aus Polyamid, wie z.B. PA 12, Polyester, Polyurethan, Polyacrylaten, Polyethern usw.

Wie eingangs erwähnt, sind neben der Wahl des multipotenten Wirkstoffes Rapamycin selbst, weitere Faktoren von Bedeutung, um ein auch optimal langfristig antirestenotisch wirksames Medizinprodukt zu erhalten. So stellen die physikalischen und chemischen Eigenschaften von Rapamycin und des eventuell zugefügten weiteren Wirkstoffes wie deren mögliche Wechselwirkungen, Wirkstoffkonzentration, Wirkstofffreisetzung, Wirkstoffkombination, ausgewählte Polymere und Beschichtungsverfahren wichtige und einander direkt beeinflussende und deshalb für jede Ausführungsform genau zu bestimmende Parameter dar. Durch Steuerung dieser Parameter kann der Wirkstoff bzw. die Wirkstoffkombination von den angrenzenden Zellen der Gefäßwand in genügender bzw. optimal wirksamer Menge über den gesamten restenosegefährdeten kritischen Zeitraum hinweg aufgenommen werden.

Die erfindungsgemäßen Stents sind bevorzugt mit mindestens einer vollständig oder unvollständig den Stent überdeckenden den Wirkstoff Rapamycin bzw. eine bevorzugte Wirkstoffkombination mit Rapamycin enthaltenden Schicht versehen und/oder der erfindungsgemäße Stent enthält den Wirkstoff Rapamycin und/oder eine Wirkstoffkombination mit Rapamycin im Stentmaterial selbst.
Zusätzlich lässt sich mit Hilfe der hämokompatiblen Schicht auf der Oberfläche während und auch nach Diffusion des Wirkstoffes in die Umgebung gewährleisten, dass keine Abwehrreaktionen auf den Fremdkörper aufkommen können.

Die Schichten können zum einen aus reinen Wirkstoffschichten, wobei mindestens eine der Schichten Rapamycin enthält und zum anderen aus wirkstofffreien bzw. wirkstoffenthaltenden Polymerschichten, oder aus deren Kombinationen bestehen.

Als Verfahren zur Herstellung eines solchen Medizinproduktes lassen sich das Sprühverfahren, Tauchverfahren, das Pipettierverfahren, Elektrospinning und/oder die Lasertechnik anwenden. Dabei wird je nach gewählter Ausführungsform das am besten geeignete Verfahren zur Herstellung des Medizinproduktes ausgewählt, wobei auch die Kombination zweier oder mehrerer Verfahren anwendbar ist.

Weiterhin bevorzugt ist die Zugabe mindestens eines weiteren Wirkstoffes, der entweder zusammen mit Rapamycin in einer Schicht vorliegt oder in einer separaten Schicht aufgebracht wird. Als weitere Kombination ist die Anwendung von beispielsweise Acetylsalicylsäure (Aspirin) vorteilhaft, da neben der unterstützenden antiphlogistischen Wirkung Aspirin antithrombotische Fähigkeit besitzt.

In der Kombination mit dem hydrophoben Paclitaxel lässt sich der antiproliferative Effekt je nach Ausführungsform verstärken bzw. verlängern, da sich Paclitaxel und Rapamycin durch ihre unterschiedliche Bioverfügbarkeit ergänzen. Hier kann beispielsweise die hydrophile Rapamycinschicht auf einer Paclitaxelschicht aufgebracht werden, wobei Rapamycin sich mehr gegen die früher auftretenden Entzündungsreaktionen richtet und Paclitaxel langfristig die Proliferation der SMC's hemmt.

Eine weitere bevorzugte Ausführungsform besteht in der Verwendung geeigneter biokompatibler Materialien als Reservoir für Rapamycin bzw. einer Wirkstoffkombination mit Rapamycin auf dem Stent.
Dazu ist die Beschichtung des Stentkörpers mit mindestens einer biostabilen und/oder bioresorbierbaren polymeren Schicht, die Rapamycin und/oder eine Wirkstoffkombination mit Rapamycin enthält.
Der Rapamycingehalt der polymeren Schicht beträgt zwischen 1Gew. % und 60Gew.%, bevorzugt zwischen 5 und 50Gew.%, besonders bevorzugt sind zwischen 10 und 40Gew.%.

Es hat sich überraschend herausgestellt, dass die Verwendung von bioabbaubaren Polymeren vorteilhaft ist, da der Abbau des Polymeren als sogenannte Bulk erosion stattfindet. Hierbei verläuft der Kettenabbau bis zu einem gewissen Grad unter weitestgehender Erhaltung der Polymereigenschaften. Erst nach Unterschreitung einer bestimmten Kettenlänge verliert das Material seine Eigenschaften und wird brüchig. Der Abbau erfolgt in Form von kleinen sich ablösenden Bruchstücken, die in kürzester Zeit vom Organismus vollständig metabolisiert werden. Es hat sich nun gezeigt, dass dieser Abbauvorgang zu einer gezielt gesteuerten Steigerung der Rapamycinfreisetzung genutzt werden kann und damit eine wesentliche Verbesserung der Restenoseprophylaxe bietet
Während die Elution eines Wirkstoffes normalerweise in den ersten Tagen nach Implantation besonders hoch ist, um wie bereits erörtet, die Summe der akuten Abwehrreaktionen des Organismus (auf die Wunde selbst und den Fremdkörper) besser kontrollieren zu können, flacht im weiteren Verlauf diese Kurve ziemlich schnell ab, so dass die freigesetzte Wirkstoffmenge immer geringer wird bis letztendlich keine Elution mehr stattfindet und der noch verbleibende Wirkstoff aus dem Polymer nicht mehr messbar eluiert
Doch werden je nach Verletzungsgrad oder Patientenhabitus noch nach 2-4 Wochen Reaktionen beobachtet, die eine erhöhte Wirkstoffdosierung erfordern, um einer Restenose Einhalt zu gebieten.

Mit Hilfe des zeitlich kontrollierten einsetzenden Verlustes der Polymereigenschaften und Zersetzung eines bioabbaubaren Polymeren lässt sich mit dem gleichen Drug eluting stent zu einem einstellbaren späteren Zeitpunkt erneut eine für die Restenoseprophylaxe wichtige Steigerung der Wirkststoffelution erreichen (siehe Abb. 4).
So ist beispielsweise der hydrolytische Abbau von PLGA je nach Mischungsverhältnis von PLA zu PGA bzw. in der Kombination mit anderen geeigneten Polymeren so einstellbar, dass die Elutionskurve nach mehr als 2 Wochen eine weitere erhöhte Freisetzung von Rapamycin aufweist. Je nach Kombination der beiden Komponenten zueinander oder zu anderen geeigneten Polymeren lässt sich die Dosierung, der Zeitpunkt und die Dauer der späten über einen weiteren Zeitraum nochmals erhöhten Wirkstoffverfügbarkeit ("late burst") genau einstellen (siehe Abb. 4).
Zusätzlich gelingt es bei Verwendung von mindestens einem Zweischichtsystem die Dosierung und kontrollierte Wirkstofffreisetzung noch weiter gezielt zu erhöhen und/oder zu erweitern. Dies kann beispielsweise erreicht werden, indem eine erste auf den Stent (bzw. auf den hämokompatibel beschichteten Stent) aufgebrachte Schicht eine höhere Konzentration an Rapamycin als die zweite auf diese erste Schicht gebrachte Polymerschicht oder aber auch eine reine Rapamycin-Schicht darstellt. Der Einsatz von Rapamycin unterstützenden Wirkstoffen in der Rapamycin-enthaltenden Schicht oder auch in einer von dieser separat vorhandenen Schicht ist denkbar.

Eine andere bevorzugte Variante, die Beladung eines Drug eluting stent mit Rapamycin zu erhöhen, liegt mit dem Einschluss von Rapamycin in stark quellbaren Substanzen wie beispielsweise Alginat-, Pektin-, Hyaluronan, Agar-Agar, Gummi arabicum, liposomale Hydrogele, Peptidhydrogele, Gelatinekapseln und/oder stark quellbare Polymer wie PVP vor, die in die mindestens eine bioabbaubare und/oder biostabile Polymerschicht eingebracht werden Als weiteren Vorteil lässt sich hier die weitestgehende Abschirmung des Wirkstoffes von den Einflüssen der Umgebung nennen. Gleichzeitig besteht die Möglichkeit der Zugabe von Rapamycin und/oder weiteren Wirkstoffen in die Wirkstoffkapseln umgebenden Polymerschicht.
Bei Zugabe von hydrophilen Porenbildnern wie PVP erreicht man neben der Freisetzungsbeschleunigung in der Frühphase der Stentimplantation auch einen schnelleren Abbau des bioresorbierbaren Polymeren aufgrund des erleichterten Eindringens von Wasser bzw. Plasma bzw. Zellflüssigkeit in die Polymerschicht. Auf diese Weise wird Rapamycin schneller und in höherer Dosierung freigesetzt. Dies ist von grossem Vorteil, weil mit der Erhöhung der Dosierung die Wirksamkeit positiv beeinflusst wird, allerdings im Gegensatz zu Paclitaxel ohne nekrotischen Veränderungen hervorzurufen.

Eine besondere Ausführungsform stellt die Verwendung von biostabilem Polymer als Matrix und hydrophilen wirkstoffbeladenen Materialien (hydrophile Polymere wie z.B. PVP und/oder Mikrokapseln und Mikrokügelchen aus z.B. Gelatine, Alginat, vernetzten Dextrinen, Gummi arabicum, Agar-Agar etc.), als Poren und/oder Kanalbildnern dar. Bei Zugabe von wässrigen Medien bzw. beim Implantieren und Expandieren eines derart beschichteten Stents quillt das hydrophile Material an. Da allerdings die Quellbarkeit des biostabilen Polymeren im Vergleich zum hydrophilen Anteil gering ist, entsteht aufgrund des Eindringens von Flüssigkeit und der nachfolgenden Quellung Druck in den Poren, so dass das hydrophile Rapamycin aus den Poren und Kanälen gleich einer Injektion in die vasculäre Umgebung gedrückt wird (siehe Abb. 5).

Um die Aufnahme von Rapamycin in das Zellinnere zu erhöhen, lassen sich Substanzen z.B. DMSO, Lecithin und andere dergenannten Transfektionsreagentien, die die Permeabilität des Zellmembran erhöhen, zufügen. Dieses System lässt sich ebenso mit bioabbaubaren Polymeren als Matrix realisieren.
Ausschlaggebend für diese Ausführungsform ist die unterschiedlich hohe Quellfähigkeit der eingesetzten Substanzen. In dem Masse wie das quellbare Material Flüssigkeit aufnimmt, wird Rapamycin eluiert. Somit lässt sich über die Geschwindigkeit der Flüssigkeitsaufnahme der Release des Wirkstoffes steuern. Dieses System lässt sich ebenso mit bioabbaubaren Polymeren als Matrix realisieren. Wichtig ist vor allem die unterschiedlich hohe Quellfähigkeit der eingesetzten Substanzen.

Eine weitere Ausführungsform unter Einbeziehung biostabiler Polymere, insbesondere Polysulfone oder polymerisierbare Öle ergibt sich dadurch, dass man die polymere Oberfläche eines biostabil polymerbeschichteten Stents mit Hilfe von Lasertechnik in definierter Folge Löcher erzeugt, in denen über Tauch oder Pipettiertechnik Rapamycinlösung mit oder ohne beigefügtes bioabbaubares Polymer eingebracht wird. Ein abbaubares Polymer kann in diesem Falle als Diffusionsbarriere entweder nur über die einzelnen Löcher oder auch auf der gesamten Stentoberfläche aufgebracht werden. In Falle dieser Ausführungsform lässt sich gezielt die vasculäre Seite des Stents therapieren. Die Zugabe von beispielsweise antithrombotisch wirksamen Agentien im auch die Innenseite des Stents bedeckenden biostabilen Polymeren helfen die an der luminalen Seite bestehende Thrombosegefahr zu verringern.

Gemäß dieser Zweischichtausführung besteht die erste biostabile Schicht aus einer Schicht, welche im wesentlichen vollständig durch eine weitere bioabbaubare Schicht überzogen ist, so dass die oben genannten Vorteile der Wirkstofffreisetzung erhalten bleiben. Desweiteren bevorzugt ist die Aufbringung von zwei polymeren Schichten, die entweder aus demselben oder aus unterschiedlichen Materialien bestehen, wobei Rapamycin in einer oder in beiden Schichten in gleicher oder unterschiedlicher Konzentration mit und/oder ohne weitere Wirkstoffe vorliegt.
Ebenso lässt sich die Elution von Rapamycin und/oder einer Wirkstoffkombination durch die Zugabe von Porenbildner derart steuert, dass sich in den beiden Schichten unterschiedliche Mengen an Porenbildner befinden, wie es auch die Möglichkeit gibt gezielt unterschiedliche Wirkstoffe mit einzubringen, die abhängig vom Porenbildner und dessen Anteile in der Beschichtung, unterschiedlich eluieren.

Es besteht ab dieser Zweischichtausführung die Möglichkeit verschiedene Wirkstoffe voneinander getrennt in jeweils der für den jeweiligen Wirkstoff geeigneten Schicht unterzubringen, so das sich beispielsweise ein hydrophober Wirkstoff in der einen hydrophileren Schicht aufhält und eine andere Elutionskinetik aufweist als der ein anderer hydrophober Wirkstoff, der in der hydrophoberen Polymerschicht vorliegt oder umgekehrt. Dies bietet ein weites Feld der Möglichkeiten die Verfügbarkeit der Wirkstoffe in eine bestimmte sinnvolle Abfolge zu bringen als auch die Elutionszeit und Konzentration zu steuern.
Als weitere bevorzugte geeignete Polymere lassen sich z.B. Polycaprolacton, Polycaprolactam, Polyaminosäuren, Trimethylencarbonat und niedrig vernetze poymerisierbare Öle nennen.

### Figurenbeschreibung

**Fig. 1** **:** Cypher™- Drug eluting stent bei 500facher Vergrösserung (Rasterelektronenmikroskopaufnahme). Die vielfachen und tiefe Risse in der Beschichtung sind deutlich zu erkennen. Dies führt zur unkontrollierten Freisetzung von Wirkstoff.
**Fig. 2** : Cypher™-Drug eluting stent (Cypher-Stent) (Rasterelektronenmikroskopaufnahme) ; man sieht deutlich die sich ablösenden Bruchstücke der biostabilen Polymerbeschichtung. Damit sind folgenden Probleme verbunden :
   a) vom Organismus nicht abbaubare Polymerstücke werden in den Blutkreislauf gebracht
   b) der Wirkstoff wird nicht gezielt, kontrolliert und richtig dosiert freigesetzt
   c) die Stentoberfläche wird als Fremdoberfläche freigesetzt, so dass das Thromboserisiko steigt.
**Fig. 3** **:** Rasterelektronenmikroskop-Aufnahme eines polymerbeschichteten Rapamycin-eluting Stents gemäss dieser Erfindungsmeldung. Der Unterschied zum Cypher-Stent ist deulich zu erkennen : keine Risse und keine Abspaltungen von Polymerbruchstücken. Im gezeigten Fall wurde ein biodegradierbares Polymer verwendet.
**Fig. 4** : Elutionsprofil von Rapamycin in dem bioabbaubarem Polymer PLGA. Es ist gut zu erkennen, dass nach ca. 400-500 Stunden ein dem "first release" (direkt nach Implantation) erneuter Anstieg in der Freisetzungsrate von Rapamycin erfolgt, der von uns als "late burst" bezeichnet wird.
**Fig. 5** : Elutionsverhalten von Rapamycin aus einer biostabilen Matrix.
**Fig. 6** : Schema der Wirkweise eines porenbildenden Systems und Rapamycin-Release durch Freisetzung über Kanäle und Quellung
   Der hydrophile Wirkstoff gelangt durch die von den Porenbildnern gebildeten Kanäle direkt an die Gefässwand.
   Werden stark quellende Substanzen mit Rapamycin in eine nicht oder merklich geringer quellende Matrix gemischt, wird der Wirkstoff durch den beim Quellvorgang entstehenden Druck an die Oberfläche gedrückt ("Injektionsmodell")

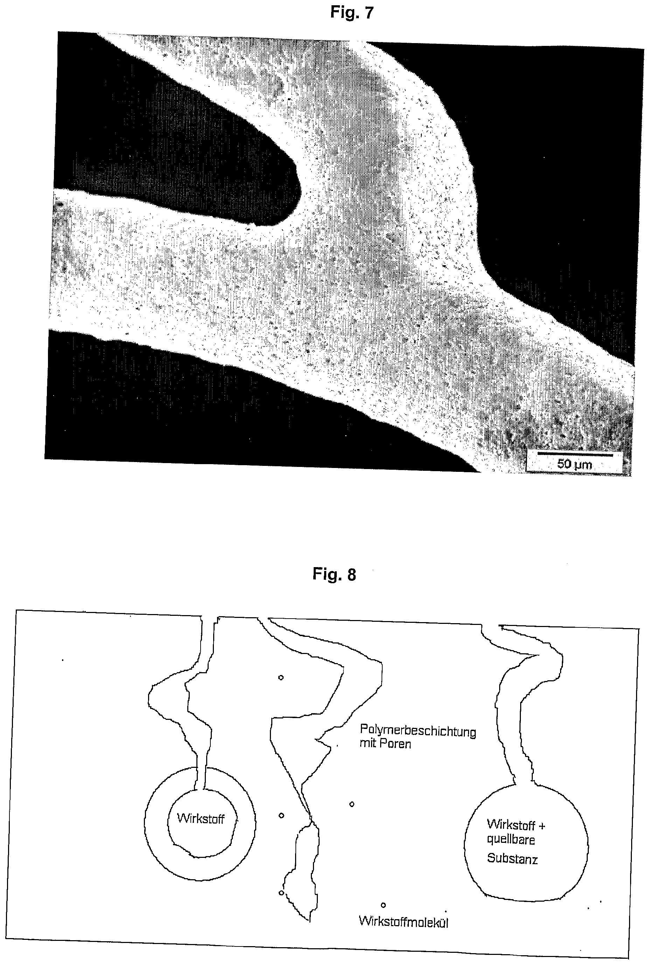

**Fig. 7** **:** Die Matrix besteht aus einer biostabilen Matrix, die einen hohen Anteil Porenbildner bzw. Mikrokanäle enthält, über die das Rapamycin schnell, kontorlliert und in hoher Dosierung zum Bestimmungsort gelangt. Auch in diesem Fall wird keine Ablösung von Polymerbruchstücken bemerkt oder sonstige Mängel erkannt.
**Fig. 8** **:** Schema zur Beschichtung von Rapamycin eluierenden Stents mit Matrices, die Mikrokanäle bilden, durch die Rapamycin an die Oberfläche gelangt . Der hydrophile Wirkstoff gelangt durch die von den Porenbildnern gebildeten Kanäle direkt an die Gefässwand. Werden stark quellende Substanzen mit Rapamycin in eine nicht oder merklich geringer quellende Matrix gemischt, wird der Wirkstoff durch den beim Quellvorgang entstehenden Druck an die Oberfläche gedrückt ("Injektionsmodell").

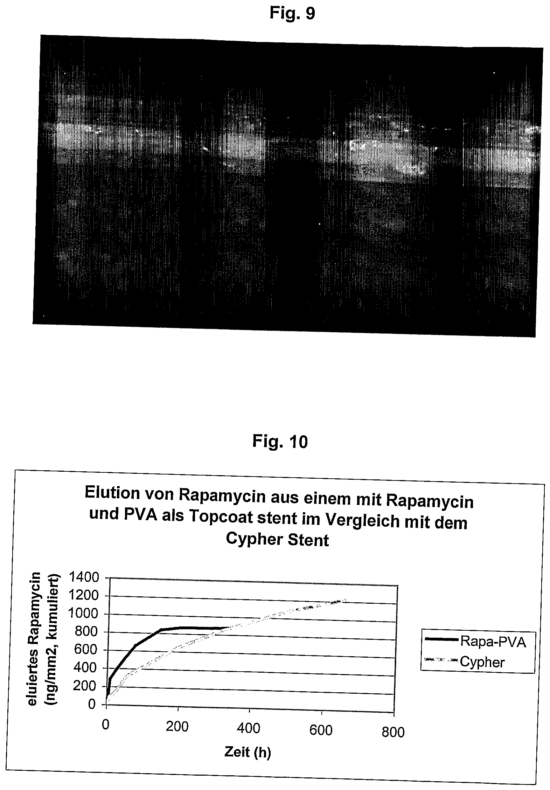

**Fig. 9** : Ein gemäss dieser Erfindungsanmeldung in einem kombinierten Beschichtungsverfahren mit Rapamycin und Isopropylmyristat als Hilfsstoff vollflächig versehener und expandierter Ballonkatheter. Man kann erkennen, dass die Beschichtung auch nach Expansion nicht abbröselt oder rissig geworden ist.
**Fig. 10** : Elutionsverhalten von Rapamycin aus dem Cypher-Stent (gelb) im Vergleich zu einem Stent mit einer reinen Rapamycinschicht und einem Topcoat aus PVA (blau). Das wesentlich beschleunigte Elutionsverhalten des Rapamycin/PVA-Systems ist deutlich vom Cypher abzugrenzen.

### Beispiele

Beispiele 1-4, 8, 13-24 sind Vergleichsbeispiele.

### Beispiel 1 : Sprühbeschichtung von Stents mit Rapamycin

Die gereinigten nicht expandierten Stents werden horizontal auf eine dünne Metallstange (d= 0,2mm) gehängt, die auf die Rotationsachse der Rotations- und Vorschubanlage gesteckt ist und mit 28 U/min rotiert. Die Stents werden so aufgebracht, das die Innenseite des Stents die Stange nicht berührt und mit einer 2%igen Sprühlösung Rapamycin in Chloroform oder Ethylacetat besprüht. Anschliessend wird im Abzug über Nacht getrocknet.
Falls erforderlich kann der Beschichtungsvorgang wiederholt werden, bis die gewünschte Wirkstoffbeladung auf dem Stent vorliegt.

### Beispiel 2 : Ermittlung des Elutionsverhaltens in PBS-Puffer

Je ein Stent wird in einem genügend kleinen Gefäß mit 2 ml PBS-Puffer versetzt, verschlossen und im Trockenschrank bei 37°C inkubiert. Nach Ablauf der gewählten Zeitintervalle wird jeweils der Überstand abpipettiert und dessen UV-Absorption gemessen.

### Beispiel 3 : Stent mit Rapamycin als Basecoat und PVA als Topcoat

Der mit Rapamycin sprühbeschichtete und getrocknete Stent wird in einem zweiten Schritt mit einer methanolisch-wässrigen 1,5%igen PVA Lösung sprühbeschichtet. Anschliessend wird getrocknet.

### Beispiel 3 : Sprühbeschichtung von Stents mit Rapamvcin und Cyclosporin A

Die gereinigten nicht expandierten Stents werden horizontal auf eine dünne Metallstange gehängt, die auf die Rotationsachse der Rotations- und Vorschubanlage gesteckt ist und mit 28 U/min rotiert. Die Stents werden so aufgebracht, das die Innenseite des Stents die Stange nicht berührt und mit einer 2%igen Sprühlösung Rapamycin und Cyclosporin A im Verhältnis 2:0,5 in Chloroform besprüht. Anschliessend wird über Nacht getrocknet.

### Beispiel 4 : : Sprühbeschichtung von Stents mit Rapamycin und Paclitaxel in zwei Schichten

Die gereinigten nicht expandierten Stents werden mit einer 0,8%igen Sprühlösung Paclitaxel in Chloroform besprüht. Anschliessend wird der Stent bei Raumtemperatur getrocknet. In einem zweiten Sprühvorgang wird wie in Beispiel 1 verfahren.

### Beispiel 5 : Beschichtung von Stents mit einem bioabbaubaren oder biostabilen Polymer und Rapamycin bzw. einer Wirkstoffkombination mit Rapamycin

Sprühlösung : 145,2 mg PLGA bzw. Polysulfon und 48,4 mg Rapamycin oder einer 33%igen Sprühlösung aus einer entsprechenden Wirkstoffkombination von Rapamycin (Anteil 20%-90%) mit einem oder mehreren weiteren Wirkstoffen wie Paclitaxel, Cyclosporin A, Thalidomid, Fusadil etc. werden mit Chloroform auf 22 g aufgefüllt.
Diese Sprühlösung wird wie bereits beschrieben auf den Stent aufgebracht.

Der eingesetzte Stent kann ein bare Stent sein, ein hämokompatibel beschichteter Stent und/oder ein mit einer Wirkstoffschicht über Sprüh oder Tauchverfahren beschichteter Stent sein.
Die reine Wirkstoffschicht oder Wirkstoffkombination nach Beispiel 1 und 3 kann wahlweise auch auf die polymere Schicht aufgebracht werden.

### Beispiel 6 : Zweischichtsystem mit einem bioabbaubaren Polymer und Rapamycin bzw. einer Wirkstoffkombination mit Rapamycin mit unterschiedlicher Konzentration des Wirkstoffes in den Schichten

Lösung 1 : 25%ige Lösung aus Rapamycin oder in Kombination mit einem oder mehreren Wirkstoffen und PLGA in Chloroform oder wahlweise Ethylacetat (0,8%ige Lösung)
Lösung 2 : 35%ige Lösung aus Rapamycin oder in Kombination mit einem oder mehreren Wirkstoffen und PLGA in Chloroform oder wahlweise Ethylacetat (0,8%ige Lösung)

Der Stent ist wie entweder ein bare Stent oder hämokompatibel beschichteter Stent und kann bereits eine reine Wirkstoffschicht aus Rapamycin, einer Kombination mit anderen Wirkstoffen oder einer Rapamycinfreien Wirkstoffschicht durch Tauchen oder Sprühen erhalten haben.
Ebenso lässt sich eine reine Wirkstoffschicht zwischen die beiden Polymerschichten oder und als Topcoat im Sprüh- bzw. Tauchverfahren auftragen

### Beispiel 7 : Zweischichtsystem mit einem biostabilen Polymer als Basisschicht und einem bioabbaubaren Polymeren als Topcoat und Rapamycin bzw. einer Wirkstoffkombination mit Rapamycin

PS- Lösung: 176 mg Polyethersulfon werden eingewogen und mit Chloroform auf 20 g aufgefüllt (0,88%ige Lösung).
PLGA-Lösung : 35%ige Lösung aus Rapamycin oder in Kombination mit einem oder mehreren Wirkstoffen (Rapamycinanteil mind. 20%) und PLGA (0,8%ige Lösung)

Auch hier wird der bare Stent oder ein hämokompatibel beschichteter Stent benutzt. Nach dem Trocknen der ersten Schicht, lässt sich die bioabbaubare Polymerschicht auftragen, wobei das Sprühverfahren und das Pipettierverfahren, dass die gezielte Auftragung auf der vasculären Seite erlaubt, bevorzugt werden. Der Wirkstoff kann auch hier zusätzlich zwischen den beiden Polymerschichten und/oder auf der Oberfläche als zusätzliche Schicht durch Sprüh- bzw. Tauchverfahren oder Pipettierverfahren aufgebracht werden.

### Beispiel 8: Beschichtung eines Stents mit biostabilem oder bioabbaubarem Polmer mit hohem Anteil an Hydrogel (PVP, Silikon, Hydrosome, Alginat, Peptid, Glycosaminoglycane) als Porenbildner (bzw. Kanalformierung)

Rapamycin (bzw. eine Wirkstoffkombination, 35Gew. %) wird mit Polysulfon und Hydrogel in Chloroform gelöst, so dass eine Lösung entsteht, die 8% Hydrogel enthält. Diese Lösung wird auf den Stent wie in den Beispielen davor aufgebracht. Die Gesamtkonzentration der polymeren Lösung sollte unter 0,9% sein, um ein optimales Sprühverhalten zu erreichen. Beim Tauchverfahren sollte die Lösung nicht über 30% Polymeranteil liegen.
Die Rapamycinbeladung kann auch durch nachträgliches Eintauchen des bereitsbeschichteten Stent in eine Wirkstofflösung (2%ig) erfolgen.
Beispiel 8a) Sprühlösung Polysulfon / PVP ohne Zugabe von Rapamycin
   24 mg PS und 2,4 mg PVP werden eingewogen und mit Chloroform auf 3 g aufgefüllt. → 0,80 % PS, 0,08 % PVP
Beispiel 8b) Sprühlösung Polysulfon / PVP unter Zugabe von Rapamycin
   18,2 mg PS, 14,1 mg Rapamycin und 3,2 mg PVP werden eingewogen und mit Chloroform auf 4 g aufgefüllt. → 0,45 % PS, 0,35 % Rapamycin, 0,08 % PVP

### Beispiel 9 : Kovalente hämokompatible Beschichtung von Stents

### a.) Herstellung von desulfatiertem reacyliertem Heparin:

100 ml Amberlite IR-122 Kationenaustauscherharz wurden in eine Säule mit 2 cm Durchmesser gefüllt, mit 400 ml 3M HCl in die H⁺-Form überführt und mit destilliertem Wasser gespült, bis das Eluat chloridfrei und pH neutral war. 1 g Natrium-Heparin wurde in 10 ml Wasser gelöst, auf die Kationenaus-tauschersäule gegeben und mit 400 ml Wasser eluiert. Das Eluat wurde in eine Vorlage mit 0,7 g Pyridin getropft und anschließend mit Pyridin auf pH 6 titriert und gefriergetrocknet.

0,9 g Heparin-Pyridinium-Salz wurden in einem Rundkolben mit Rückflußkühler mit 90 ml einer 6/3/1 Mischung aus DMSO/1,4-Dioxan/Methanol (V/V/V) versetzt und 24 Stunden auf 90°C erhitzt. Dann wurden 823 mg Pyridiniumchlorid zugegeben und weitere 70 Stunden auf 90°C erhitzt. Anschließend wurde mit 100 ml Wasser verdünnt und mit verdünnter Natronlauge auf pH 9 titriert. Das desulfatierte Heparin wurde gegen Wasser dialysiert und gefriergetrocknet.

100 mg des desulfatierten Heparins wurden in 10 ml Wasser gelöst, auf 0°C gekühlt und unter Rühren mit 1,5 ml Methanol versetzt. Zu der Lösung wurden 4 ml Dowex 1x4 Anionenaustauscherharz in der OH⁻-Form und anschließend 150 µl Essigsäureanhydrid gegeben und 2 Stunden bei 4°C gerührt. Danach wird das Harz abfiltriert und die Lösung gegen Wasser dialysiert und gefriergetrocknet.

### b) N-carboxymethyliertes, partiell N-acetyliertes Chitosan:

In 150 ml 0,1 N HCl wurde 2 g Chitosan gelöst und unter Stickstoff 24 Stunden unter Rückfluß gekocht. Nach dem Abkühlen auf Raumtemperatur wurde der pH der Lösung mit 2 N NaOH auf 5,8 eingestellt. Die Lösung wurde gegen demineralisiertes Wasser dialysiert und gefriergetrocknet.

1 g des so partiell hydrolysierten Chitosans wurden in 100 ml 1%iger Essigsäure gelöst. Nach hinzufügen von 100 ml Methanol wurden 605 µl Essigsäureanhydrid gelöst in 30 ml Methanol zugegeben und 40 Minuten bei Raumtemperatur gerührt. Das Produkt wurde durch Eingießen in eine Mischung von 140 ml Methanol und 60 ml 25%iger NH₃-Lösung ausgefällt. Es wurde abfiltriert, mit Methanol und Diethylether gewaschen und unter Vakuum über Nacht getrocknet.

1 g des partiell hydrolysierten und partiell N-acetylierten Chitosans wurde in 50 ml Wasser suspendiert. Nach dem Hinzufügen von 0,57 g Glyoxylsäuremonohydrat löste sich das Chitosanderivat innerhalb der nächsten 45 Minuten auf. Der pH Wert der Lösung wurde mit 2 N NaOH auf 12 eingestellt. Eine Lösung von 0,4 g Natriumcyanoborhydrid in möglichst wenig Wasser wurde zugegeben und für 45 Minuten gerührt. Das Produkt wurde in 400 ml Ethanol ausgefällt, abfiltriert, mit Ethanol gewaschen und über Nacht im Vakuum getrocknet.

### c) Herstellung von desulfatiertem N-propionyliertem Heparin:

100 ml Amberlite IR-122 Kationenaustauscherharz wurden in eine Säule mit 2 cm Durchmesser gefüllt, mit 400 ml 3M HCl in die H⁺-Form überführt und mit destilliertem Wasser gespült, bis das Eluat chloridfrei und pH neutral war. 1 g Natrium-Heparin wurde in 10 ml Wasser gelöst, auf die Kationenaus-tauschersäule gegeben und mit 400 ml Wasser eluiert. Das Eluat wurde in eine Vorlage mit 0,7 g Pyridin getropft und anschließend mit Pyridin auf pH 6 titriert und gefriergetrocknet.

0,9 g Heparin-Pyridinium-Salz wurden in einem Rundkolben mit Rückflußkühler mit 90 ml einer 6/3/1 Mischung aus DMSO/1,4-Dioxan/Methanol (V/V/V) versetzt und 24 Stunden auf 90°C erhitzt. Dann wurden 823 mg Pyridiniumchlorid zugegeben und weitere 70 Stunden auf 90°C erhitzt. Anschließend wurde mit 100 ml Wasser verdünnt und mit verdünnter Natronlauge auf pH 9 titriert. Das desulfatierte Heparin wurde gegen Wasser dialysiert und gefriergetrocknet.

100 mg des desulfatierten Heparins wurden in 10 ml Wasser gelöst, auf 0°C gekühlt und unter Rühren mit 1,5 ml Methanol versetzt. Zu der Lösung wurden 4 ml Dowex 1x4 Anionenaustauscherharz in der OH⁻-Form und anschließend 192 µl Propionsäureanhydrid gegeben und 2 Stunden bei 4°C gerührt. Danach wird das Harz abfiltriert und die Lösung gegen Wasser dialysiert und gefriergetrocknet.

### d) N-carboxymethyliertes, partiell N-propionyliertes Chitosan:

In 150 ml 0,1 N HCl wurde 2 g Chitosan gelöst und unter Stickstoff 24 Stunden unter Rückfluß gekocht. Nach dem Abkühlen auf Raumtemperatur wurde der pH der Lösung mit 2 N NaOH auf 5,8 eingestellt. Die Lösung wurde gegen demineralisiertes Wasser dialysiert und gefriergetrocknet.

1 g des so partiell hydrolysierten Chitosans wurden in 100 ml 1%iger Essigsäure gelöst. Nach hinzufügen von 100 ml Methanol wurden 772 µl Propionsäureanhydrid gelöst in 30 ml Methanol zugegeben und 40 Minuten bei Raumtemperatur gerührt. Das Produkt wurde durch Eingießen in eine Mischung von 140 ml Methanol und 60 ml 25%iger NH₃-Lösung ausgefällt. Es wurde abfiltriert, mit Methanol und Diethylether gewaschen und unter Vakuum über Nacht getrocknet.

1 g des partiell hydrolysierten und partiell N-acetylierten Chitosans wurde in 50 ml Wasser suspendiert. Nach dem Hinzufügen von 0,57 g Glyoxylsäuremonohydrat löste sich das Chitosanderivat innerhalb der nächsten 45 Minuten auf. Der pH Wert der Lösung wurde mit 2 N NaOH auf 12 eingestellt. Eine Lösung von 0,4 g Natriumcyanoborhydrid in möglichst wenig Wasser wurde zugegeben und für 45 Minuten gerührt. Das Produkt wurde in 400 ml Ethanol ausgefällt, abfiltriert, mit Ethanol gewaschen und über Nacht im Vakuum getrocknet.

### Beispiel 10 : Kovalente hemokompatible Beschichtung von Stents

Nicht expandierte Stents aus medizinischem Edelstahl LVM 316 wurden im Ultraschallbad 15 Minuten mit Aceton und Ethanol entfettet und bei 100°C im Trockenschrank getrocknet. Danach wurden sie für 5 Minuten in eine 2%ige Lösung von 3-Aminopropyltriethoxysilan in einem Gemisch Ethanol/Wasser (50/50 : (v/v)) getaucht und anschließend für 5 Minuten bei 100°C getrocknet. Anschließend wurden die Stents über Nacht mit demineralisiertem Wasser gewaschen.

3 mg der hämokompatiblen Substanz aus Beispiel 10 (z.B.. desulfatiertes und reacetyliertes Heparin wurden bei 4°C in 30 ml 0,1M MES-Puffer (2-(N-Morpholino)ethansulfonsäure) pH 4,75 gelöst und mit 30 mg N-Cyclohexyl-N'-(2-morpholinoethyl)carbodiimid-methyl-p-toluolsulfonat versetzt. In dieser Lösung wurden 10 Stents für 15 Stunden bei 4°C gerührt. Anschließend wurde mit Wasser, 4M NaCl-Lösung und Wasser für je 2 Stunden gespült.

### Beispiel 11 : Bestimmung des Glucosamingehaltes der beschichteten Stents mittels HPLC

Hydrolyse : Die beschichteten Stents werden in kleine Hydrolyserohre gegeben und mit 3ml 3M HCl genau eine Minute bei Raumtemperatur stehengelassen. Die Metallproben werden entfernt und die Röhrchen nach dem Verschließen 16h im Trockenschrank bei 100°C inkubiert. Danach lässt man abkühlen, es wird dreimal zur Trockne eingedampft und in 1 ml entgastem und filtriertem Wasser aufgenommen und gegen einen ebenfalls hydrolysierten Standard in der HPLC vermessen :

| Stent Nr. | Fläche Probe | Ac-Heparin [g/Probe] | Fläche [cm²] | Ac-Heparin [g/cm²] | Ac-Heparin [pmol/cm²] |
|---|---|---|---|---|---|
| 1 | 129,021 | 2,70647E-07 | 0,74 | 3,65739E-07 | 41,92 |
| 2 | 125,615 | 2,63502E-07 | 0,74 | 3,56084E-07 | 40,82 |
| 3 | 98,244 | 1,93072E-07 | 0,74 | 2,60908E-07 | 29,91 |
| 4 | 105,455 | 2,07243E-07 | 0,74 | 2,80058E-07 | 32,10 |
| 5 | 119,061 | 2,33982E-07 | 0,74 | 3,16192E-07 | 36,24 |
| 6 | 129,202 | 2,53911E-07 | 0,74 | 3,43124E-07 | 39,33 |
| 7 | 125,766 | 2,53957E-07 | 0,74 | 3,43185E-07 | 39,34 |

### Beispiel 12: Biokompatible Beschichtung von Stents mit Leinöl unter Zugabe eines Katalysators und einem synthetischen Polymeren, insbesondere Polyvinylpyrrolidon und anschliessender Wirkstoffzugabe

a) Nicht expandierte Stents aus medizinischem Edelstahl LVM 316 wurden im Ultraschallbad 15 Minuten mit Aceton und Ethanol entfettet und bei 100°C im Trockenschrank getrocknet. Anschließend wurden die Stents über Nacht mit demineralisiertem Wasser gewaschen.
   Ca. 10mg KMnO₄ werden in 500µl Wasser gelöst und soviel PVP wie möglich zugegeben. Die Masse wird flächig auf einer Polypropylenunterlage ausgebreitet und bei Raumtemperatur über Nacht trocknen gelassen.
   Von dieser spröden Masse werden 2,5mg in 1ml Chloroform gelöst und nach Zugabe von 10,5 µl Leinöl die resultierende Lösung mit einer Airbrush-Sprühpistole (EVOLUTION von Harder & Steenbeck) aus 6cm Entfernung auf einen rotierenden 18mm LVM Edelstahlstent gesprüht. Danach wurde der beschichtete Stent 24h bei 80°C gelagert.
b) Wirkstoffzugabe zu einem beschichteten Stent im Tauchverfahren

Der in Beispiel 18a) beschichtete Stent wurde in einer Lösung von 800µg Rapamycin in 1ml Ethanol getaucht und quellen gelassen. Nach Abschluß des Quellvorgangs wurde der Stent herausgenommen und getrocknet.

### Beispiel 13: Biokompatible Beschichtung von Stents mit Leinöl und Rapamycin

Nicht expandierte Stents aus medizinischem Edelstahl LVM 316 wurden im Ultraschallbad 15 Minuten mit Aceton und Ethanol entfettet und bei 100°C im Trockenschrank getrocknet. Anschließend wurden die Stents über Nacht mit demineralisiertem Wasser gewaschen.
Leinöl und Rapamycin (70:30) wird im Mischungsverhältnis 1:1 in Chloroform gelöst und dann auf den gleichmäßig rotierenden Stent gesprüht. Nach Abdampfen des Chloroforms im leichten Luftstrom wird der Stent im Trockenschrank bei 80°C gelagert.
Die durchschnittliche Coatingmasse beträgt 0,15mg ± 0,02mg.

### Beispiel 14: Biokompatible Beschichtung von Rapamycin-eluierenden Stents mit einer ethanolischen Sprühlösung aus Leinöl und dem synthetischen Polymeren Polyvinylpyrrolidon (PVP)

Nach dem Reinigen der Stents wie in den Beispielen zuvor bereits beschrieben wird eine ethanolsche Sprühlösung hergestellt, die 0,25% Leinöl und 0,1% PVP enthält und mit einer Sprühpistole auf den um seine eigene Achse rotierenden Stent gleichmäßig aufgesprüht. Anschließend wird über Nacht bei 70°C getrocknet. Die Coatingmasse beträgt durchschnittlich 0,2mg ± 0,02mg.
Rapamycin bzw. eine Wirkstoffkombination mit Rapamycin wird entweder anschliessend durch Quellung eingebracht oder der Sprühlösung mit mindestens 20Gew.% Rapamycingehaltbeigemischt.

### Beispiel 15: Biokompatible Beschichtung von Stents mit Leinöl und dem synthetischen Polymeren Polyvinylpyrrolidon (PVP) im Zweischichtsystem unter Zugabe eines restenosehemmenden Wirkstoffes

Nach dem Reinigen der Stents wird eine erste Schicht aus 0,35Gew.-% Rapamycin gelöst in Chloroform auf den Stent aufgesprüht. Nach dem Trocknen dieser Schicht bei Raumtemperatur wird die zweite Schicht einer Chloroform-Lösung mit 0,25% Leinöl und 0,1% PVP aufgesprüht.

### Beispiel 16 : Biokompatible Beschichtung von Stents mit Leinöl und α-Linolensäure

Nach dem Reinigen der Stents mit Aceton und Ethanol, wie vorherig beschrieben, wird eine in Ethanol gelöste Mischung mit 0,20% Leinöl und 0,5% α-Linolensäure hergestellt und gleichmäßig auf den Stent aufgesprüht.

### Beispiel 17 Vollflächige Beschichtung eines Esophagus-Stents durch Tauchbeschichtung

### a) Vorbeschichtung der Stentstreben

An den Stab eines Rotators wird ein Stent befestigt und bei geringer Drehzahl mit sehr langsamen Auf- und Abbewegungen der Pistole wird der Stent mit einer 1%igen Polyurethanlösung eingesprüht.. Durch das Aufsprühen wird der Stent gräulich-matt, so dass man eine optische Sprühkontrolle durchführen kann. Besonders wichtig ist es, dass der Rand einwandfrei besprüht ist, was durch ein zusätzliches Sprühen rundherum gewährleistet wird. Danach lässt man trocknen.

### b) Vollflächige Beschichtung eines nach a) gesprühten Stents

Polyurethan und 35Gew.% Rapaymcin/Tergurid (4:1) werden in THF gelöst, so dass man eine 14%ige Lösung erhält. Ein nach Beispiel 18a) vorbeschichteter Stent wird vorsichtig auf das passende Formwerkzeug aufgeschoben.
Das Werkzeug mit dem aufgezogenen Stent wird kopfüber in reines THF getaucht bis Luftblasen erscheinen, die aufsteigen. Danach taucht man langsam in die 14%ige Polyurethan-Lösung. Nach 15 Sekunden zieht man den Kern wieder langsam heraus und bringt ihn sofort in die Waagerechte und dreht den Kern, damit sich das PU gleichmäßig auf dem Stent verteilt und trocknet.
Wenn es nicht mehr verläuft, wird der Kern unter dem Abzug trocknen gelassen und anschliessend für 45 min bei 95°C im Trockenschrank getempert. Nach dem Abkühlen wird in eine warme 0,3%ige SDS-Lösung eingetaucht, um den Stent vom Werkzeug abzulösen. Nach Reinigung unter fließendem Wasser und Spülen mit 0,5 m NaOH wird sehr gründlich unter fließendem lauwarmem Wasser und in VE-Wasser gespült.

### Beispiel 18 Teilflächige Beschichtung eines neuronalen Stentes zur Behandlung von Aneurysmen

Lösung: 3,2 mg PU gelöst in 20ml N-Methyl-2-Pyrrolidon und 33Gew.% Rapamycin Ein sprühbeschichteter Stent wird auf ein passendes frei drehbares Formwerkzeug geschoben, so das er vollständig auf dem glatten Untergrund aufliegt.
Das Auftragen der Beschichtung erfolgt in mindestens zwei Schichten, wobei mit einem Pinselhaar Lösung aufgenommen und diese in das zu beschichtende Feld aufgetragen wird, bis das Feld komplett mit Lösung bedeckt ist.
Wenn jedes ausgewählte zu beschichtende Feld in der gewünschten Beschichtungsdicke gefüllt ist, wird der Stent bei 90°C getrocknet. Nach dem Abkühlen wird der Stent vom Formwerkzeug gelöst.

### Beispiel 19 : Beschichtung eines Faltenballons mit Rapamycin mit Hilfe mittels Sprühverfahren

Nach vorsichtiger Vorbenetzung des Ballons mit Aceton wird der Ballon unter Drehen um die Längsachse gleichmässig mit einer Lösung von Rapamycin in Ethylacetat besprüht und getrocknet. Damit die Falten nicht beim Drehen auseinandergehen, wird im Ballon Vakuum erzeugt. Damit die Falten nicht beim Drehen auseinandergehen, wird im Ballon Vakuum erzeugt.

### Beispiel 20 : Vollflächige Beschichtung eines Faltenballons mit Rapamycin mit Hilfe des Pipettierverfahrens

Der Faltenballon wird in horizontaler Lage auf einer drehbaren Achse fixiert. Damit die Falten nicht beim Drehen auseinandergehen, wird im Ballon Vakuum erzeugt. So wird Schritt für Schritt der in Ethanol gelöste Wirkstoff entlang der Längsachse ausserhalb und in den Falten mit einer Teflonkanüle als Erweiterung einer Nadelspritze solange beschichtet, bis eine durchgängige Rapamycinschicht zu erkennen ist. Danach wird der Ballon getrocknet.

Der Wirkstofflösung wird vorzugweise ein Hilfsstoff zugesetzt, der die Durchgängigkeit des Wirkstoffes in die Zellen erleichtert. Dazu werden beispielsweise 150 mg Rapamycin 4,5 ml Aceton, 100µl lodopromid und 450µl Ethanol gemischt.

### Beispiel 21: Bestimmung der Wirkstoffverluste durch Expansion in einem in vitro-Modell

Dazu wird der mit Rapamycin und einem Hilfsstoff beschichtete Faltenballon in einem mit PBS-Puffer gefüllten Silikonschlauch expandiert. Anschliessend wird die auf dem Ballon verbleibende Beschichtung in einer definierten Menge Acetonitril gelöst und der Rapamycingehalt mittels HPLC quantifiziert. Desgleichen wird die an der Schlauchwand haftende Menge Rapamycin mit Acetonitril gespült und quantifiziert wie auch der Gehalt im Puffer bestimmt.

### Beispiel 22: Partielle Beschichtung eines Faltenballons mit Rapamycin mit Hilfe des Pipettierverfahrens

Der Faltenballon wird in horizontaler Lage auf einer drehbaren Achse fixiert, so dass die zu befüllende Falte immer auf der oberen Seite zu liegen kommt und Vakkum angelegt, so dass die Falten sich nicht öffnen können.
Eine 1%ige dünnflüssige alkoholische Lösung von Rapamycin wird angesetzt, welche derart dünnviskos ist, dass sich diese Lösung aufgrund von Kapillarkräften selber in die Falten eines Faltenballons hineinziehen kann. Mittels einer Kapillare, welche an einem Ende der Falte ansetzt, lässt man die alkoholische Lösung in die Falte fließen, bis sich der Falteninnenraum aufgrund von Kapillarkräften vollständig gefüllt hat. Man lässt den Falteninhalt trocknen, dreht den Ballon und befüllt die nächste Falte. Jede Falte wird nur einmal befüllt

### Beispiel 23 :

Der in Beispiel 7 nur in den Falten wirkstoffbeladene lässt sich in einem zweiten Schritt mittels Sprühverfahren mit einer polymeren äusseren Schicht als Barriere überziehen. Dazu muss die Konzentration der polymeren Sprühlösung so gering gehalten werden, dass die nach dem Trocknen erhaltene Polymerschicht die gleichmässige Entfaltung nicht behindert. Beispielsweise ist hier bereits eine 0,5%ige PVP-Lösung geeignet.

### Beispiel 24 : Beschichtung eines inflatierten Katheterballons exlusiv in den Falten in Anwesenheit eines auf dem Ballon gecrimpten Stent

a) Eine 35%ige Lösung von Rapamycin bzw. einer Wirkstoffkombination (z.B. Rapamycin und Thalidomid oder Thalidomid/Paclitaxel-Mischung) in Chloroform wird mit Hilfe einer Pipettieranlage in die Falten eines drehend gelagerten Faltenballons gebracht, bis diese sichtbar gleichmäßig befüllt sind. Anschliessend wird der Faltenballon langsam drehend bei Raumtemperatur getrocknet. Die Anwesenheit eines auf dem Ballon gecrimpten Stents bzw. Drug eluting stents stört dabei nicht.
b) Biostabiles bzw. bioabbaubares Polymer bzw. eine Kombination der beiden (siehe auch vorangegangene Beispiele) und eine Wirkstoffombination mit mindestens 30Gew.% Rapamycin werden mit Chloroform gelöst, so dass der Gesamt-Wirkstoffanteil der Lösung 30Gew. % beträgt. Die Gesamtlösung ist 0,9%ig. Diese Lösung lässt sich nach dem Tauchverfahren oder Sprühverfahren gleichermassen abwenden. Auch hier kann der Stent bereits anwesend sein.

## Patentansprüche

1. Stent beschichtet mit mindestens einer polymeren Schicht aus Poly(D,L-lactidco-Glycolid) und Rapamycin, wobei der Stent eine unterste Beschichtung aus einem hämokompatiblen Polymer aufweist, und wobei Rapamycin eine Konzentration von 0,001 - 10 mg per cm² der Stentoberfläche hat.

2. Stent gemäß Anspruch 1, wobei die hämokompatible Beschichtung kovalent an die Stentoberfläche gebunden ist.

3. Stent gemäß einem der vorherigen Ansprüche, wobei Rapamycin in Kombination mit einem zweiten Wirkstoff vorliegt.

4. Stent gemäß Anspruch 3, wobei der zweite Wirkstoff aus der Gruppe umfassend Tacrolimus, Paclitaxel und seine Derivate, Fasudil®, Vitronektinrezeptorantagonisten, Thalidomid, Cyclosporin A, Tergurid, Lisurid und Celecoxip ausgewählt wird.

5. Stent gemäß einem der vorherigen Ansprüche, wobei nicht nur die Stentstreben, sondern auch die Zwischenräume zwischen den Stentstreben mit einem Polymer oder einer Trägermatrix, die Rapamycin enthalten, beschichtet sind.

6. Stent gemäß einem der vorherigen Ansprüche, wobei der Stent kontinuierlich oder sequenziell durch Sprüh-, Tauch-, Streich-, Pipettier-, Seifenblasen-, Plasmaverfahren, Elektrospinning und/oder Lasertechnik beschichtet wird.

## Claims

1. Stent coated with at least a polymer layer of poly(D,L-lactide-co-glycolide acid), and rapamycin, wherein the stent has a lowermost coating of a hemocompatible polymer, wherein rapamycin has a concentration from 0.001 - 10 mg per cm² of stent surface.

2. Stent according to claim 1, wherein the hemocompatible coating is bound covalently to the stent surface.

3. Stent according to any one of the previous claims, wherein rapamycin is present in combination with a second active agent.

4. Stent according to any one of the previous claims, wherein the second active agent is selected from the group comprising:
tacrolimus, paclitaxel and its derivatives, fasudil®, vitronektin receptor antagonists, thalidomid, cyclosporin A, tergurid, lisurid, celecoxip.

5. Stent according to any one of the previous claims, wherein not only the stent struts but also the interstices between the stent struts are coated with the polymer in which rapamycin is present.

6. Stent according to any one of the previous claims, wherein the stent is coated continuously or sequentially by spraying method, dipping method, brushing method, pipetting method, soap bubble method, plasma method, electrospinning and/or laser technique.

## Revendications

1. Un stent recouvert d'au moins une couche polymère de poly(D,L-lactide-co-glycolide) et de rapamycine, où le stent présente une couche inférieure en polymère hémocompatible et où la concentration de rapamycine se situe entre 0,001 et 10 mg par cm² de surface du stent.

2. Un stent conforme à la revendication 1, où le revêtement hémocompatible présente une liaison covalente avec la surface du stent.

3. Un stent conforme à l'une des revendications précédentes, où la rapamycine est combinée à un deuxième principe actif.

4. Un stent conforme à la revendication 3, où le deuxième principe actif est issu du groupe comprenant le tacrolimus, le paclitaxel et ses dérivés, le fasudil®, les antagonistes aux récepteurs de vitronectine, le thalidomide, la cyclosporine A, le terguride, le lisuride et le célécoxib.

5. Un stent conforme à l'une des revendications précédentes, où les tiges du stent ainsi que les espaces intermédiaires entre les tiges sont recouverts d'un polymère ou d'une matrice porteuse comprenant de la rapamycine.

6. Un stent conforme à l'une des revendications précédentes, où le stent présente un revêtement continu ou séquentiel appliqué par pulvérisation, immersion, enduit, pipetage, bulle, plasma, électrospinning et/ou laser.
